# EUROPEAN PATENT APPLICATION

(11) **EP 4 834 888 A1**
(43) Date of publication of application: **04.11.2026**
(21) Application number: 24911506.4
(22) Date of filing: 27.12.2024
(51) Int. Cl.: C07D 295/088, C07D 295/13, C07D 211/22, A61K 45/00, A61K 47/18, A61K 9/51

(54) **CATIONIC LIPID COMPRISING NITROGEN-CONTAINING HETEROCYCLE AND GLYCINE**

(30) Priority: 29.12.2023 CN 202311855265; 15.03.2024 CN 202410300878
(71) Applicant: Xiamen Sinopeg Biotech Co., Ltd, Xiamen, Fujian 361100 (CN)
(72) Inventor: LIN, Sheng, Xiamen, Fujian 361100 (CN); LIN, Minggui, Xiamen, Fujian 361100 (CN); WANG, Linlin, Xiamen, Fujian 361100 (CN); WANG, Ailan, Xiamen, Fujian 361100 (CN); WEI, Guohua, Xiamen, Fujian 361100 (CN); WENG, Wengui, Xiamen, Fujian 361100 (CN); LIU, Chao, Xiamen, Fujian 361100 (CN); ZHU, Qi, Xiamen, Fujian 361100 (CN)
(74) Representative: Weickmann & Weickmann PartmbB
(86) International application number: PCT/CN2024/143275
(87) International publication number: WO 2025/140590

(57) **Abstract**

The present invention relates to a glycine-containing cationic lipid having a structure as shown in Formula (1), wherein the definitions of the symbols are consistent with those described herein. The glycine-branched cationic lipid contain a azacycle as the central branching core, glycine as a secondary branching core, and saturated or unsaturated hydrocarbon groups as the tail chains. exhibiting advantages of low toxicity, low immunogenicity, and high biocompatibility. The amino acid moieties contained in the novel cationic lipids of the present application not only serve as secondary branching cores but also provide tertiary amine structures, which, together with the azacyclic central core, form a multi-level tertiary amine architecture, increasing the number of tertiary amines that can be ionized to bear positive charges. Consequently, the preparation of LNPs requires less cationic lipid, which enhances safety while improving the translation efficiency of nucleic acids.

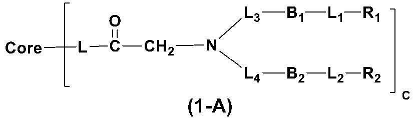

## Description

### TECHNICAL FIELD

The present application relates to the field of drug delivery, specifically relates to an azacycle-containing, glycine-based cationic lipid as a pharmaceutical carrier, as well as a lipid composition comprising the cationic lipid, a lipid pharmaceutical composition, and formulations and applications thereof.

### BACKGROUND

Bioactive substances such as small molecule drugs, peptides, proteins, and nucleic acids, particularly nucleic acid drugs, are prone to degradation by nucleases and have a short half-life, hindering their effective cellular delivery. Although chemical modifications can significantly improve the stability of nucleic acids and avoid immune recognition, the scope for chemical modifications is limited, so delivery systems are typically required to achieve effective delivery of nucleic acid drugs. Delivery systems can enhance the efficacy and safety of nucleic acid drug therapy, and currently, lipid nanoparticles (LNPs) are the most commonly used delivery systems for nucleic acid drugs.

LNPs formed by cationic lipids and other lipid components (such as cholesterol, phospholipids, PEGylated lipids) with nucleic acid drugs can prevent the degradation of nucleic acids in plasma and promote cellular uptake of nucleic acids. In the LNP delivery systems, the structure of nucleic acid, the structure of cationic lipid, and various lipid components are all crucial to the effectiveness of delivery systems, especially the cationic lipid with the highest molar ratio, which significantly affects the effectiveness of delivery systems. Although there are well-established cationic lipids such as MC3, SM102, and ALC-0315 in existing technologies, there is still a need in this field to develop other cationic lipids that can be used for the delivery of nucleic acids to achieve better delivery effects, for example, protecting nucleic acids from degradation and clearance in serum, high tolerability, and providing a sufficient therapeutic index.

Although recent advances have disclosed azacycle-branched cationic lipids, such as the compounds disclosed in CN115010681A and US20220040308A1, but they do not contain amino acid (e.g., glycine) linker arms, or do not contain degradable groups, or contain a limited number of degradable groups, which cannot meet the requirements of all delivery systems. Therefore, there is still a need in this field to develop novel cationic lipids containing both an azacycle and amino acid (e.g., glycine) linker arms.

### SUMMARY

The present application provides a novel azacycle-containing, glycine-based cationic lipid and preparation methods thereof, a lipid composition containing the cationic lipid, a lipid pharmaceutical composition containing the lipid composition and formulation thereof, and a liposome or LNP containing the lipid composition. Especially, the LNP-nucleic acid pharmaceutical composition containing the lipid composition and its formulations have the advantages of high delivery efficiency, safety, low toxicity, and high biocompatibility, which can improve the therapeutic and/or preventive effects of drugs.

The above-described purposes of this application can be realized via the embodiments below:
In one embodiment of the present application, provided herein is a cationic lipid:
A cationic lipid, wherein the structure is represented by the general formula (1-A): 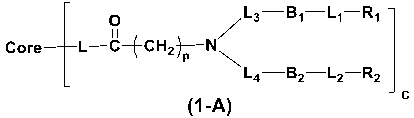
or a salt, tautomer, stereoisomer, deuterated derivative, or solvate thereof;
wherein, Core is an azacyclic core;
L is a divalent linking group L_{d}; the L_{d} is selected from the group consisting of -(CH₂)ₜₘ-, -Z-, -(CH₂)ₜₘZ-, -Z(CH₂)ₜₘ-, -Z(CH₂)ₜₘZ-, -(CH₂)ₜₘZ(CH₂)ₜₘ-, -Z(CH₂)ₜₘZ(CH₂)ₜₘ-, -(CH₂)ₜₘZ(CH₂)ₜₘZ-, and -(CH₂)ₜₘZ(CH₂)ₜₘZ(CH₂)ₜₘ-; wherein, tm is independently an integer from 1 to 12 at each occurrence; Z is independently selected from the group consisting of -C(=O)-, -OC(=O)-, -C(=O)O-, -OC(=O)O-, -O-, -S-, -NH-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S- at each occurrence;wherein, R_{c} is independently H or a C₁₋₁₂ alkyl group at each occurrence;
p is an integer from 1 to 4;
B₁ and B₂ are each independently a linking bond or a C₁₋₂₀ alkylene group at each occurrence;
L₁, L₂, L₃, and L₄ are each independently a linking bond or a divalent linking group -(CH₂)ₜₙLₐ(CH₂)ₜₙ- at each occurrence; the Lₐ is selected from the group consisting of -OC(=O)-, -C(=O)O-, -OC(=O)O-, -CH(OH)-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-; wherein, s is 1, 2, 3, or 4; tn is independently an integer from 0 to 12 at each occurrence; L₁ and L₃ are not linking bonds simultaneously; L₂ and L₄ are not linking bonds simultaneously;
R₁ and R₂ are each independently a C₅₋₃₀ hydrocarbon group or a C₅₋₃₀ hydrocarbon derivative group at each occurrence;
c is 2, 3, or 4.

The present application also provides a lipid composition, embodied as follows:
A lipid composition containing a cationic lipid with the structure represented by the formula (1-A).

The present application also provides a lipid pharmaceutical composition, embodied as follows:
A lipid pharmaceutical composition comprising a lipid composition and a drug; wherein, the lipid composition comprises a cationic lipid with the structure represented by the formula (1-A); the drug is selected from the group consisting of a nucleic acid drug, a gene vaccine, an anti-tumor drug, a small molecule drug, a peptide drug, and a protein drug.

The present application also provides a formulation of lipid pharmaceutical composition, embodied as follows:
A formulation of lipid pharmaceutical composition containing the aforementioned lipid pharmaceutical composition and a pharmaceutically acceptable diluent or excipient.

The present application also provides a liposome or LNP, embodied as follows:
A liposome or LNP containing a lipid composition, wherein the lipid composition comprises a cationic lipid with the structure represented by formula (1-A).

**Compared with the prior art, the present application brings the following beneficial effects:**
The novel cationic lipids of the present application contain an azacycle as the branching core, amino acid (e.g., glycine or glycine derivative) moieties as the linking arms, and saturated or unsaturated hydrocarbon groups as the tail chains. The cationic lipids of the present application can complex with negatively charged drugs or nucleic acids through electrostatic interactions, thereby enabling effective delivery of nucleic acids into cells. Furthermore, the use of biocompatible amino acid moieties or amino acid derivative moieties as the linking arms in the present application further improves the biocompatibility of the cationic lipids. The starting materials of amino acids or amino acid derivatives are readily available, which can be naturally obtained or synthesized simply, offering the advantages of convenience, safety, and cost-effectiveness in production.

The amino acid moieties contained in the novel cationic lipids of the present application not only serve as secondary branching cores but also provide tertiary amine structures, which, together with the azacyclic central core, form a multi-level tertiary amine architecture, increasing the number of tertiary amines that can be ionized to bear positive charges. Consequently, the preparation of LNPs requires less cationic lipid, which enhances safety while improving the translation efficiency of nucleic acids.

The novel cationic lipids of the present application contain one or more biodegradable groups between the azacycle and the hydrophobic tails. The presence of degradable groups enables the LNP pharmaceutical compositions prepared from the cationic lipids to degrade timely within endosomes and exhibit low cytotoxicity, which addresses the issue in the prior art where LNP pharmaceutical compositions prepared from non-degradable or poorly degradable cationic lipids accumulate in endosomes, acidify the endosomal environment, and hinder the endosomal escape of drugs (e.g., mRNA), thereby preventing the delivered drugs from exerting their full effects. Moreover, these biodegradable groups are stable at physiological pH and can be enzymatically hydrolyzed within living cells in tissues, forming multiple low-toxicity small-molecule acids, alcohols, amines, amino acids, etc., thereby further reducing the cytotoxicity of the lipids and improving the delivery efficiency.

The positive charges of nitrogen atoms of the azacycle in the novel cationic lipid of the present application may be delocalized over the entire ring, so that the cationic lipid exhibits higher transfection efficiency and lower cytotoxicity.

### BRIEF DESCRIPTION OF THE DRAWINGS

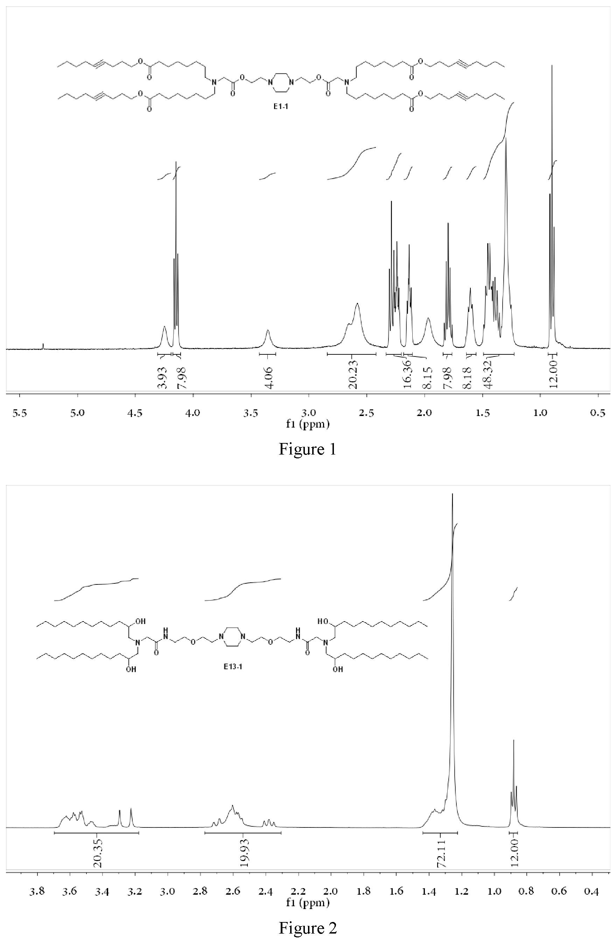

Figure 1 is the ¹H NMR spectrum of cationic lipid E1-1 prepared in Example 1.
Figure 2 is the ¹H NMR spectrum of cationic lipid E13-1 prepared in Example 13.

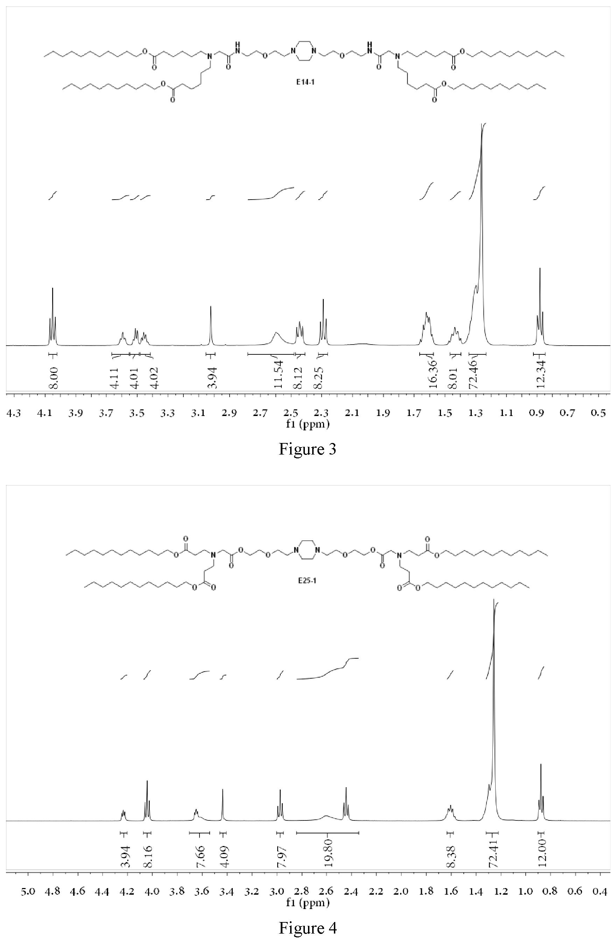

Figure 3 is the ¹H NMR spectrum of cationic lipid E14-1 prepared in Example 14.
Figure 4 is the ¹H NMR spectrum of cationic lipid E25-1 prepared in Example 25.

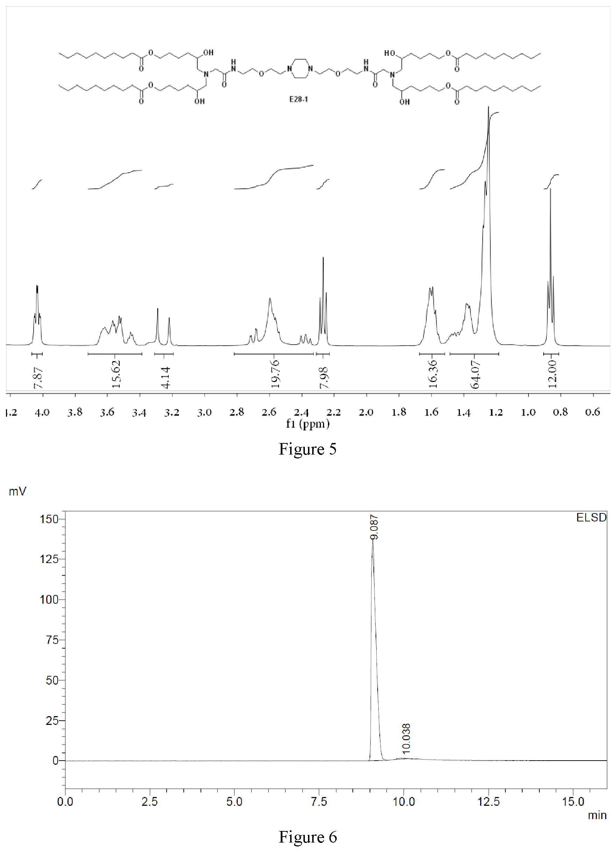

Figure 5 is the ¹H NMR spectrum of cationic lipid E28-1 prepared in Example 28.
Figure 6 is the high-performance liquid chromatography (HPLC) test results of cationic lipid E13-1 prepared in Example 13.
Figure 7 is the mass spectrometry (MS) of cationic lipid E13-1 prepared in Example 13.
Figure 8 is the cytotoxicity test results of LNP-mRNA pharmaceutical composition L-15 prepared in Example 47.
Figure 9 is the imaging results after mouse injection of LNP-mRNA pharmaceutical composition L-15 prepared in Example 47.

### DETAILED DESCRIPTION

### Description of Terms

In the present application, all technologies and scientific terms used herein have the same meaning as those commonly understood by those of ordinary skill in the art, unless otherwise described. The disclosures of all patents and other publications cited herein are incorporated by reference in their entirety as if fully set forth herein. In the event of any conflict between the description and interpretation of the terms used in this application and any document incorporated by reference, the description and interpretation of the following terms shall prevail.

In the present application, unless otherwise specified, when the structure involved has isomers, it can refer to any one of the isomers. For example, when cis- and trans- isomers are present, it can refer to either a cis isomer or a trans isomer; when E/Z isomers exist, the structure can be either an (E)-structure or a (Z)-structure; and when optical activity is present, it can be either a levorotatory form or a dextrorotatory form.

In the present application, a numerical interval may be indicated by a hyphen (e.g., 0-12), a tilde (e.g., 0~12), or "to" (e.g., 0 to 12, 1 to 12). In the present application, an integer interval represents a group consisting of all integers within the range, including both endpoints, unless otherwise specified. For example, the integer interval 0-12 represents the group consisting of 0, 1, 2, 3, 4, 5, 6, 7, 8, 9, 10, 11, and 12. The numerical interval in the present application includes, but is not limited to, ranges represented by integers, non-integers, percentages, and fractions, and includes both endpoints, unless otherwise specified.

In the present application, "about" and "approximately" generally indicate a range of ±10% for numerical values, and in some cases may be expanded to ±15%, but not more than ±20%, based on the preset values. For example, when the molar percentage of steroid lipid in the total lipids is about 40%, it is generally assumed to include cases where the molar percentage of steroid lipids ranges from 30% to 50%.

In the present application, unless otherwise specified, "any" includes any one, any two, and any two or more.

In the present application, unless otherwise specified, the term "include", "contain", "comprise", and similar expressions shall be interpreted as "including but not limited to" in the specification and claims, in an open and inclusive manner.

In the present application, when two or more objects are "each independently preferably" selected from multiple levels of preferable options, the objects are not necessarily selected from preferable options of the same level. It is allowed that one is selected from a wider range of options while another one is selected from a narrower range of options. It is also allowed that one is selected from the maximum range of options while another is selected from any allowable options. It is also allowed that the objects are selected from preferable options of the same level.

In the present application, "each independently at each occurrence" not only means that different groups can be each independently selected from the definitions, but also means that the same group at different positions can be independently selected from the definitions. For example, "independently selected form the group consisting of a linking bond, -OC(=O)-, -C(=O)O-, -OC(=O)O-, -C(=O)-, -O-, -NH-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-, wherein the R_{c} is each independently a hydrogen atom or a C₁₋₁₂ alkyl group at each occurrence" indicates that, in the group "-NRcC(=O)NRc-", both R_{c} are each independently a hydrogen atom or a C₁₋₁₂ alkyl group, i.e., both R_{c} can be the same or different.

In the present application and unless otherwise specified, a divalent linking group, e.g., a hydrocarbylene group, an alkylene group, an arylene group, an amide bond, and the like, can use either one of the two linking ends to connect to another group. For example, when an amide bond serves as a divalent linking group between GroupA and GroupB, both GroupA-C(=O)NH-GroupB and GroupB-NHC(=O)-GroupA are allowable.

In the present application, when distinguishing the terminus from the substituents of a linking group becomes questionable, " 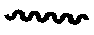 " is used to indicate the connection location between the linking group and the other groups. For example, in structural formulas 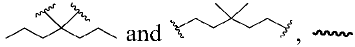 is used to indicate the connection locations between the divalent linking groups and the other groups; the two structural formulas mentioned above represent -CH(CH₂CH₂CH₃)₂- and -CH₂CH₂CH(CH₃)₂-CH₂CH₂-, respectively.

In the present application, a numerical interval written in the subscript of "C" indicates the number of carbon atoms of a group. For example, C₁₋₁₂ indicates "having 1 to 12 carbon atoms"; C₁₋₃₀ indicates "having 1 to 30 carbon atoms". The "substituted C₁₋₁₂ alkyl group" refers to a C₁₋₁₂ alkyl group with one or more hydrogen atoms being replaced. The "C₁₋₁₂ substituted alkyl group" refers to an alkyl group with one or more hydrogen atoms being replaced, with 1 to 12 carbon atoms remaining. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁, and C₁₂ alkylene groups. In the present application and unless otherwise specified, a subscript being a numerical interval indicates that the subscript can be any integer within the interval which includes two endpoints.

In the present application, the term "group" may be referred to as "bond" without changing the meaning. For example, an ether group (-O-) can also be termed an ether bond, and an ester group (-OC(=O)- or -C(=O)O-) can also be termed an ester bond.

In the present application, the "carbon chain length" between two groups refers to the minimum number of carbon atoms involved in the pathway from one group to another, excluding the groups themselves. For example, in the structures -OC(=O)CH₂CH₂OC(=O)and -OC(=O)CH(CH₃)CH₂OC(=O)-, the carbon chain lengths between the two ester bonds are both 2 (also referred to as C2); the carbon chain length between the two ester bonds of -OC(=O)CH₂CH₂OCH₂CH₂OC(=O)- is C4 (also referred to as C4).

In the present application, heteroatoms are not particularly limited, including but not limited to O, S, N, P, Si, F, Cl, Br, I, B, etc.

In the present application, the heteroatom used for substitution is referred to as a "substituent atom", and the group used for substitution is referred to as a "substituent group".

In the present application, the term "substituted" indicates that at least one hydrogen atom of the described group (e.g., aliphatic hydrocarbon, hydrocarbon, alkyl, or alkylene group) is replaced by a bond connected to a non-hydrogen atom. The non-hydrogen atom include but are not limited to halogen atoms such as F, Cl, Br, and I, oxo group (=O), hydroxyl group (-OH), hydrocarbonoxy groups (-OR_{d}; wherein, R_{d} is a C₁₋₁₂ alkyl group), carboxyl group (-COOH), amine groups (-NR_{c}R_{c}; wherein, both R_{c} are each independently H or a C₁₋₁₂ alkyl group), C₁₋₁₂ alkyl groups, and cycloalkyl groups. In some embodiments, the substituent is a C₁₋₁₂ alkyl group. In other embodiments, the substituent is a cycloalkyl group. In other embodiments, the substituent is a halogen atom, such as fluorine atom. In other embodiments, the substituent is an oxo group. In other embodiments, the substituent is a hydroxyl group. In other embodiments, the substituent is an alkoxy group. In other embodiments, the substituent is a carboxyl group. In other embodiments, the substituent is an amine group.

In the present application, "optional" or "optionally" (e.g., optionally substituted) means that the event of the situation described thereafter may or may not occur, and the description includes instances in which the event or situation occurs as well as instances in which the event or situation does not occur. For example, "optionally substituted hydrocarbon groups" means that the hydrocarbon groups may or may not be substituted, and the description includes both substituted and unsubstituted hydrocarbon groups.

In the present application, a compound or a group can be substituted and heteroatom-incorporated simultaneously, e.g., by replacing a hydrogen atom with a nitrophenyl group, or by replacing -CH₂-CH₂-CH₂- with -CH₂-S-CH(CH₃)-.

In the present application, a "linking bond" is only for connecting and does not contain any atoms. When a group is defined as including a linking bond, the group is optionally absent.

In the present application, a "group" contains at least one atom, referring to the radical formed by a compound losing one or more atoms. Relative to a compound, the remaining group formed by the removal of other groups is also referred to as the "residue". The valence of groups is not particularly limited, and examples include a monovalent group, a divalent group, a trivalent group, a tetravalent group, ..., a hectovalent group, etc. Wherein, groups with valence being equal to or greater than two are collectively defined as linking groups. A linking group may also contain only one atom, such as ether group or thioether group.

In the present application, "hydrocarbon" refers to a class of compounds that contain only carbon atoms and hydrogen atoms.

In the present application, hydrocarbons are classified into aliphatic hydrocarbons and aromatic hydrocarbons in terms of the type of hydrocarbon groups. Hydrocarbons that do not contain phenyl rings or hydrocarbyl-substituted phenyl rings are defined as aliphatic hydrocarbons. Hydrocarbons that contain at least one phenyl ring or hydrocarbyl-substituted phenyl ring are defined as aromatic hydrocarbons. An aromatic hydrocarbon may contain aliphatic hydrocarbon structures, such as toluene, diphenylmethane, 2,3-dihydroindene, etc.

In the present application, hydrocarbons are classified into saturated hydrocarbons and unsaturated hydrocarbons in terms of the degree of saturation. All aromatic hydrocarbons are unsaturated hydrocarbons. Saturated aliphatic hydrocarbons are also referred to as alkanes. There is no specific limit to the degree of unsaturation of unsaturated aliphatic hydrocarbons. For example, unsaturated aliphatic hydrocarbons may include, but are not limited to, alkenes (containing carbon-carbon double bonds), alkynes (containing carbon-carbon triple bonds), and dienes (containing conjugated carbon-carbon double bonds), etc. When the aliphatic moieties of aromatic hydrocarbons are saturated, the aromatic hydrocarbons are also termed aralkanes, such as toluene.

In the present application, the structures of hydrocarbons are not particularly limited and may include, for example, linear structures without pendant groups, branched structures with pendant groups, ring-containing structures, dendritic structures, comb-like structures, and hyperbranched structures, etc. Unless otherwise specified, preferable structures include linear structures without pendant groups, branched structures with pendant groups, and ring-containing structures, corresponding to linear hydrocarbons, branched hydrocarbons and cyclic hydrocarbons, respectively. Wherein, hydrocarbons that contain no rings are termed open-chain hydrocarbons, including but not limited to linear structures without pendant groups, and branched structures with pendant groups. Open-chain hydrocarbons belong to aliphatic hydrocarbons. Therefore, linear hydrocarbons are also referred to as linear aliphatic hydrocarbons. Branched hydrocarbons are also referred to as branched aliphatic hydrocarbons.

In the present application, "hydrocarbon group" refers to a residue formed by removing at least one hydrogen atom from a hydrocarbon molecule. Depending on the number of hydrogen atoms removed, hydrocarbon groups can be classified into monovalent hydrocarbon groups (with one hydrogen atom removed), divalent hydrocarbon groups (with two hydrogen atoms removed, also referred to as hydrocarbylene groups), trivalent hydrocarbon groups (with three hydrogen atoms removed), and the like. Accordingly, when n hydrogen atoms are lost, the valence of the formed hydrocarbon group is n. Unless otherwise specified, hydrocarbon groups in the present application specifically refer to monovalent hydrocarbon groups. Unless otherwise expressly stated in this specification, a hydrocarbon group is optionally substituted.

In the present application, the source of hydrocarbon group is not particularly limited and may include, for example, from aliphatic hydrocarbons or aromatic hydrocarbons, from saturated hydrocarbons or unsaturated hydrocarbons, from linear hydrocarbons, branched hydrocarbons or cyclic hydrocarbons, and from hydrocarbons or heterohydrocarbons, etc. According to the degree of saturation, hydrocarbon groups can be derived from alkanes, alkenes, alkynes, dienes, etc. With respect to cyclic hydrocarbons, hydrocarbon groups can be derived from alicyclic hydrocarbons or aromatic hydrocarbons, or from monocyclic hydrocarbons or polycyclic hydrocarbons. With respect to heterocyclic hydrocarbons, hydrocarbon groups can be derived from aliphatic heterocyclic hydrocarbons or aromatic heterocyclic hydrocarbons.

In the present application, "aliphatic hydrocarbon group" refers to the residue formed by removing at least one hydrogen atom from an aliphatic hydrocarbon molecule. Unless otherwise specified, aliphatic hydrocarbon groups refer to monovalent hydrocarbon groups in the present application. Aliphatic hydrocarbon groups include both saturated and unsaturated aliphatic hydrocarbon groups. Unless otherwise expressly stated in this specification, aliphatic hydrocarbon groups are optionally substituted.

In the present application, "alkyl group" refers to a hydrocarbon group obtained from an alkane by removing a hydrogen atom at any position. Unless otherwise specified, the alkyl group can be linear or branched, and substituted or unsubstituted. Specifically, a propyl group refers to either a 1-propyl group or an isopropyl group, and a propylene group refers to a 1,3-propylene group, a 1,2-propylene group, or an isopropylidene group. Unless otherwise specified in this specification, alkyl groups are optionally substituted.

In the present application, "unsaturated hydrocarbon group" refers to the hydrocarbon group obtained from an unsaturated hydrocarbon by removing hydrogen atoms. The unsaturated hydrocarbon obtained by removing hydrogen atoms from unsaturated carbon atoms can be classified into alkenyl groups, alkynyl groups, dienyl groups, and the like.

In the present application, "alkenyl group" refers to a linear or branched-chain hydrocarbon group containing at least one carbon-carbon double bond, formed by removing hydrogen atoms from any position of an alkene. For example, "C₂₋₁₅ alkenyl group" refers to a linear or branched alkenyl group containing 2 to 15 carbon atoms and at least one carbon-carbon double bond, i.e., one, two, three, four, or more carbon-carbon double bonds. Unless otherwise specified, the alkenyl groups include substituted and unsubstituted alkenyl groups in the present application. Unless otherwise expressly stated in this specification, alkenyl groups are optionally substituted.

In the present application, "alkynyl group" refers to a linear or branched-chain hydrocarbon group containing at least one carbon-carbon triple bond, formed by losing hydrogen atoms from any position of an alkyne. For example, "C₂₋₁₅ alkynyl group" refers to a linear or branched alkynyl group containing 2 to 15 carbon atoms and at least one carbon-carbon triple bond. An alkynyl group can contain one, two, three, four, or more carbon-carbon triple bonds. Unless otherwise specified, alkynyl groups include substituted and unsubstituted alkynyl groups in the present application. Unless otherwise expressly stated in this specification, alkynyl groups are optionally substituted.

In the present application, "hydrocarbylene group" or "hydrocarbylene chain" refers to a linear or branched divalent hydrocarbon chain that connects the remainder of a molecule to free radicals, consisting only of carbon and hydrogen, which is saturated or unsaturated. Examples include, a hydrocarbylene group with one to twenty-four carbon atoms (a C₁₋₂₄ hydrocarbylene group), a hydrocarbylene group with one to twelve carbon atoms (a C₁₋₁₂ hydrocarbylene group), and specifically, a methylene group, an ethylene group, a propylene group, an n-butylene group, a vinylidene group, a propenylene group, an n-butenylene group, a propynylene group, an *n*-butynylene group, etc. Unless otherwise explicitly stated in this specification, hydrocarbylene groups are optionally substituted.

In the present application, an "alkylene group" is also a divalent alkyl group, including open-chain alkylene groups and divalent cycloalkyl groups. The open-chain alkylene group refers to a divalent alkyl group without a cyclic structure, and the divalent cycloalkyl group refers to a divalent alkyl group containing a cyclic structure. Unless otherwise expressly stated in this specification, alkylene groups are optionally substituted.

In the present application, "molecular weight" represents the mass of a compound, and "average molecular weight" represents the mass of a compound component of a general formula in macroscopic matter. Unless otherwise specified, the molecular weight of polymers refers to the "average molecular weight", which generally refers to the "number average molecular weight" (Mₙ). The number average molecular weight can be used to describe the molecular weight of either polydisperse blocks or substances, or monodisperse blocks or substances. Unless otherwise specified, the measuring unit of "molecular weight" and "average molecular weight" is Dalton (Da). Polymers can be characterized by the "degree of polymerization", which indicates the number of repeating units (e.g., oxyethylene units of PEG, EO-units) in a molecule. Accordingly, "average degree of polymerization", "number average degree of polymerization" or "number of EO units" represents the average value or the number average value of the number of repeating units, and unless otherwise specified, each refers to the number average degree of polymerization.

In the present application, with respect to percentages, "about" generally refers to a range of ±0.5%.

In the present application, the terms "stable" and "degradable" are a pair of opposing concepts. Detailed examples of stable groups and degradable groups are given in paragraphs [0134] to [0145] of CN113402405A.

In the present application, "hydroxyl protecting group" includes all the groups that can be used as common hydroxyl protecting groups. A hydroxyl protecting group is preferably selected from the group consisting of an alkanoyl group (e.g., an acetyl group, a butyryl group), an aromatic alkanoyl group (e.g., a benzoyl group), a benzyl group, a triphenylmethyl group, a trimethylsilyl group, a t-butyldimethylsilyl group, an allyl group, an acetal group, and a ketal group. Removal of the acetyl group is generally carried out under basic conditions, most commonly by the ammonolysis with NH₃/MeOH or by the methanol anion-catalyzed methanolysis. The benzyl group can be easily removed via palladium-catalyzed hydrogenolysis in a neutral solution at room temperature, or via a reduction reaction by metallic sodium in ethanol or liquid ammonia. The triphenylmethyl group is generally removed via catalytic hydrogenolysis. The trimethylsilyl group is generally removed using reagents containing fluoride ions (e.g., tetrabutylammonium fluoride/anhydrous THF, etc.). The t-butyldimethylsilyl ether is relatively stable and can withstand ester hydrolysis conditions with alcoholic potassium hydroxide and mild reduction conditions (e.g., Zn/CH₃OH and the like), which can be removed by fluoride ions (e.g., Bu₄N⁺F⁻) in THF solution or by aqueous acetic acid at room temperature.

In the present application, "carboxyl protecting group" refers to the protecting group which can be transformed into a carboxyl group via the hydrolysis or the deprotection reaction of a carboxyl protecting group itself. A carboxyl protecting group is preferably selected from the group consisting of an alkyl group (e.g., a methyl group, an ethyl group, and a butyl group) and an aralkyl group (e.g., a benzyl group), and more preferably selected from the group consisting of a butyl group (tBu), a methyl group (Me) and an ethyl group (Et). In the present application, "protected carboxyl group" refers to the group protected by an appropriate carboxyl protecting group, preferably selected from the group consisting of a methoxycarbonyl group, an ethoxycarbonyl group, *t*-butoxycarbonyl group, and a benzyloxycarbonyl group. The carboxyl protecting groups can be removed through hydrolysis catalyzed by acids or bases, or through pyrolysis reactions occasionally. For example, *t*-butyl groups can be removed under mild acidic conditions, and benzyl groups can be removed by hydrogenolysis. The reagent used for the removal of carboxyl protecting groups is selected from the group consisting of TFA, H₂O, LiOH, NaOH, KOH, MeOH, EtOH and combinations thereof, preferably selected from the group consisting of the combination of TFA and H₂O, the combination of LiOH and MeOH, and the combination of LiOH and EtOH. A protected carboxyl group can undergo deprotection to produce the corresponding free acid, wherein the deprotection is conducted in the presence of a base, and the base forms a pharmaceutically acceptable salt with the free acid produced via the deprotection.

In the present application, "amino protecting group" includes all the groups which are used as amino protecting groups generally, such as an aryl C₁₋₆ alkyl group, a C₁₋₆ alkoxy group, a C₁₋₆ alkyl group, a C₁₋₆ alkoxycarbonyl group, an aryloxycarbonyl group, a C₁₋₆ alkylsulfonyl group, an arylsulfonyl group, a methylsilyl group, etc. An amino protecting group is preferably selected from the group consisting of a *t*-butoxycarbonyl group (Boc), a p-methoxybenzyloxycarbonyl group (Moz), and a 9-fluorenylmethyloxycarbonyl (Fmoc). The reagent used for the removal of amino protecting groups is selected from the group consisting of TFA, H₂O, LiOH, MeOH, EtOH, and combinations thereof, preferably selected from the group consisting of the combination of TFA and H₂O, the combination of LiOH and MeOH, and the combination of LiOH and EtOH. The Boc protecting group can be removed using TFA or HCl/EA, preferably TFA. The Fmoc protecting group can be removed using the N,N-dimethylformamide (DMF) solution containing 20% piperidine.

In the present application, "cationic lipid" refers to a lipid having a positive charge or being ionizable. Wherein, "cation" refers to the corresponding structure bearing a positive charge, either permanently, or non-permanently but in response to certain conditions (such as pH). Therefore, the cations include permanent cations and cationizable substances. Permanent cations refer to the corresponding compounds, groups, or atoms that bear positive charges under conditions of any pH value or hydrogen ion activity of their environment. Typically, a positive charge is generated by the presence of quaternary nitrogen atom. When a compound carries multiple such positive charges, it can be termed a permanent cation. A cationizable substance refers to a compound, group, or atom that is positively charged at a lower pH and uncharged at a higher pH of its environment. Moreover, in non-aqueous environments where pH cannot be determined, a cationizable compound, group, or atom is positively charged at high hydrogen ion concentration and uncharged at low concentration or activity of hydrogen ions. It depends on the individual properties of the cationizable or polycationizable compound, in particular the pKa of the corresponding cationizable group or atom, at which pH or hydrogen ion concentration said compound is charged or uncharged. In the diluted aqueous environment, the Henderson-Hasselbalch equation can be used to estimate the fraction of positively charged cationizable compounds, groups, or atoms, which is well-known to those skilled in the art. For example, in some embodiments, if a compound or moiety is cationizable, then, preferably, it is positively charged at a pH of about 1 to 9, preferably 4 to 9, 5 to 8, or even 6 to 8, more preferably equal to or below 9, equal to or below 8, equal to or below 7, and most preferably at physiological pH (e.g., about 7.3 to 7.4), i.e., under physiological conditions, especially under physiological conditions of cells in vivo. In other embodiments, it is preferable that the cationizable compound or moiety is predominantly neutral at physiological pH (e.g., about 7.0 to 7.4), but becomes positively charged at lower pH values. In some embodiments, pKa of the cationizable compound or moiety is preferably about 5 to about 7.

In the present application, LNPs, cationic peptides, proteins, polysaccharides, lipids and polymers are uncharged, having a neutral charge or being respectively electrically neutral under physiological conditions, especially under the physiological conditions of cells in vivo. Cationic peptides or proteins preferably contain a larger amount of cationic amino acids, such as a greater amount of Arg, His, Lys, or Orn compared to other amino acid residues (especially more cationic amino acids than anionic amino acid residues such as Asp or Glu), or contain components formed primarily by cationic amino acid residues. The term "cationic" can also refer to "polycationic" components/compounds, and can also refer to cationic lipids that are capable of being positively charged. Examples include, cationic lipids contain one or more amine groups with positive charges, and preferably cationic lipids that are ionizable to exist in either a positively charged or neutral form depending on pH. The ionization of cationic lipids affects the surface charge of LNPs at different pH conditions. This charge state can affect plasma protein absorption, blood clearance, and tissue distribution, as well as the ability to form non-bilayer structures that are essential for the intracellular delivery of nucleic acids.

In the present application, "PEGylated lipid" refers to the molecules containing both lipid and PEG moieties.

In the present application, "neutral lipid" refers to any lipid substance that is uncharged or exists in the form of neutral zwitterion at the chosen pH, preferably phospholipid. The neutral lipid can be synthetic or natural.

In the present application, "steroid lipid" refers to a steroid or steroid analog.

In the present application, "amino acid residues" include amino acids with hydrogen atoms removed from amino groups and/or hydroxyl groups removed from carboxyl groups and/or hydrogen atoms removed from sulfhydryl groups and/or amino groups protected and/or carboxyl groups protected and/or sulfhydryl groups protected. Imprecisely, amino acid residues can be referred to as amino acids. The source of amino acids in the present application is not particularly restricted unless otherwise specified, which could be either natural or non-natural, or a mix of both. The amino acid structure type in the present application is not particularly restricted unless otherwise specified, which could be L-type or D-type, or a mix of both. In one embodiment of the present application, the amino acid is a hydrophilic amino acid, e.g., glycine (Gly).

In the present application, a variant form refers to a structure that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present application, "variant form of reactive group" refers to a reactive form that retains its reactivity (remains a reactive group) after undergoing at least one process of chemical change selected from oxidation, reduction, hydration, dehydration, electronic rearrangement, structural rearrangement, salt complexation and decomplexation, ionization, protonation, deprotonation, substitution, deprotection, leaving group transformation, etc.; alternatively, it may refer to a non-reactive form of protected reactive group.

In the present application, the term "any suitable" in "any suitable linking group" or "any suitable reactive group", etc., indicates that the described structure conforms to the basic principles of chemical structure and can enable the preparation method of the present application to be successfully implemented. The chemical structure described in this way can be considered to have a clear, definite range.

In the present application, when at least two structural types are enumerated, "any combination" of the enumerated structural types refers to a combination of any two or more of the relevant structural types listed. The number of structural units is not limited, and the number of any structural unit can be zero, one or greater than one. When the number of structural units of the same type is greater than 1, the structural units may be the same or different chemical structures, and the total number of the structural units is at least 2. For example, any arbitrary combination of an alkylene group, a divalent cycloalkyl group, a divalent cycloalkenyl group, a divalent cycloalkynyl group, a divalent cyclodienyl group, an arylene group, a carbon-carbon double bond, a carbon-carbon triple bond, a conjugated carbon-carbon double bond, a divalent aliphatic heterocyclic linking group, a divalent aromatic heterocyclic linking group, and a carbon chain linking group with heteroatom-containing pendant groups, can be, e.g., -Ph-CH₂-Ph- (arylene-alkylene-arylene), -CH₂-Ph-CH₂CH₂-(alkylene-arylene-alkylene; wherein, the alkylene group has a quantity of 2, with different chemical structures), or a structure obtained from the phenyl ring of any aforementioned example being replaced with the ring of a hexane, diazepine, or 1-(2-pyridinyl)hexahydro-1H-1,4-diazepine. Another examples include, cycloalkenyl hydrocarbon group = cycloalkenyl group + hydrocarbylene group = hydrocarbon group with a cycloalkenyl group as the substituent, and cyclodienyl hydrocarbon group = hydrocarbon group with a cyclodienyl group as the substituent.

In the present application, "N/P ratio" refers to molar ratio of nitrogen atoms in cationic lipids to phosphates in nucleic acids.

In the present application, "nucleic acid" refers to DNA, RNA, or their modified form, containing purine or pyrimidine bases present in DNA (adenine "A", cytosine "C", guanine "G", thymine "T") or purine or pyrimidine bases present in RNA (adenine "A", cytosine "C", guanine "G", uracil "U").

In the present application, "RNA" refers to ribonucleic acid that may be naturally or non-naturally occurring. For example, RNA may include modified and/or non-naturally occurring components such as one or more nucleobases, nucleosides, nucleotides, or linkers. An RNA may include a cap structure, a chain-terminating nucleoside, a stem-loop, a polyA sequence, and/or a polyadenylation signal. An RNA can be a nucleotide sequence encoding a specific polypeptide, or can be a messenger RNA (mRNA). Translation of mRNA encoding a particular polypeptide, for example, in vivo translation of an mRNA inside a mammalian cell, may produce the encoded polypeptide. RNAs may be selected from the group consisting of small interfering RNA (siRNA), asymmetrical interfering RNA (aiRNA), microRNA (miRNA), dicer-substrate RNA (dsRNA), small hairpin RNA (shRNA), messenger RNA (mRNA), single guide RNA (sgRNA), self-amplifying RNA (saRNA), circular RNA (circRNA), Cas9 mRNA, and mixtures thereof.

In the present application, antisense oligonucleotides or small interfering RNA (siRNA) can inhibit the expression of target genes and target proteins in vitro or in vivo.

In the present application, FLuc mRNA can express luciferase protein, which emits bioluminescence in the presence of luciferin substrate, so FLuc is commonly used in mammalian cell culture to measure gene expression and cell viability.

In the present application, "inhibition of target gene expression" refers to the ability of nucleic acid to silence, reduce or inhibit the expression of the target gene. To examine the extent of gene silencing, a test sample (e.g., a cell sample in culture expressing the target gene) is contacted with nucleic acids that inhibit the expression of the target gene. The expression of the target gene in the test sample or test animal is compared to expression of the target gene in a control sample (e.g., a sample of cells in culture expressing the target gene) that is not contacted with or administered the nucleic acids. The expression of the target gene in the control sample can be assigned a value of 100%. In particular embodiments, inhibition of target gene expression is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%.

In the present application, methods for determining the expression level of target genes include but are not limited to dot blots, northern blots, in situ hybridization, ELISA, immunoprecipitation, enzymatic function, and phenotypic assays.

In the present application, "transfection" refers to the introduction of a species (e.g., an RNA) into cells. Transfection may occur, such as, in vitro, ex vivo, or in vivo.

In the present application, "antigen" typically refers to a substance that can be recognized by the immune system, preferably recognized by the adaptive immune system, and trigger an antigen-specific immune response, for example, forming antibodies and/or antigen-specific T cells as a part of the adaptive immune response. Typically, the antigen may be or may contain a peptide or a protein that can be presented to T cells by MHC. In the present application, the antigen may be a translation product of the provided nucleic acid molecule (preferably mRNA as defined herein). In this context, fragments, variants, and derivatives of peptides and proteins containing at least one epitope are also understood as antigens.

In the present application, "delivery" refers to delivering an entity to the target, for example, delivering drugs and/or therapeutic agents and/or prophylactic agents to subjects, wherein the subjects are tissues and/or cells of humans and/or other animals.

In the present application, "pharmaceutically acceptable carrier" refers to a diluent, adjuvant, excipient, or vehicle administered together with the therapeutic agent, which is, within the scope of reasonable medical judgment, suitable for contact with tissues of human and/or other animals without causing excessive toxicity, irritation, allergic reaction, or other problems or complications corresponding to reasonable benefit/risk ratio. Pharmaceutically acceptable carriers that can be used in the pharmaceutical composition in the present application include but are not limited to sterile liquids, such as water and oil, including oils from petroleum, animals, vegetables, or synthesis, e.g., peanut oil, soybean oil, mineral oil, sesame oil, etc. When the pharmaceutical composition is administered intravenously, water is an exemplary carrier. Physiological saline, glucose, and aqueous glycerol solution can also be used as liquid carriers, especially as an injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. The composition can also contain a small amount of humectant, emulsifier, or pH buffer as needed. Oral preparations can contain standard carriers, such as pharmaceutical-grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and water for hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present application, "vaccines" are preventive or therapeutic materials that provide at least one antigen or antigenic function. Antigen or antigenic function can stimulate the body's adaptive immune system to provide an adaptive immune response.

In the present application, "treatment" refers to the management and care of patients to combat diseases, obstacles, or symptoms, intended to delay the progression of diseases, obstacles, or conditions, reduce or alleviate symptoms and complications, and/or cure or eliminate diseases, disorders, or conditions. The patients to be treated are preferably mammals, particularly humans.

### 1. Cationic Lipids

An embodiment of the present application:
A cationic lipid, wherein the structure is represented by the general formula (1-A): 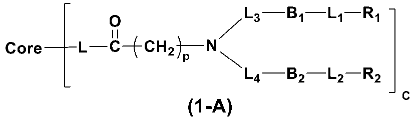
or a salt, tautomer, stereoisomer, deuterated derivative, or solvate thereof;
wherein, Core is an azacyclic core;
L is a divalent linking group L_{d}; the L_{d} is selected from the group consisting of -(CH₂)ₜₘ-, -Z-, -(CH₂)ₜₘZ-, -Z(CH₂)ₜₘ-, -Z(CH₂)ₜₘZ-, -(CH₂)ₜₘZ(CH₂)ₜₘ-, -Z(CH₂)ₜₘZ(CH₂)ₜₘ-, -(CH₂)ₜₘZ(CH₂)ₜₘZ-, and -(CH₂)ₜₘZ(CH₂)ₜₘZ(CH₂)ₜₘ-; wherein, tm is independently an integer from 1 to 12 at each occurrence; Z is independently selected from the group consisting of -C(=O)-, -OC(=O)-, -C(=O)O-, -OC(=O)O-, -O-, -S-, -NH-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S- at each occurrence; wherein, R_{c} is independently H or a C₁₋₁₂ alkyl group at each occurrence;
p is an integer from 1 to 4;
B₁ and B₂ are each independently a linking bond or a C₁₋₂₀ alkylene group at each occurrence;
L₁, L₂, L₃, and L₄ are each independently a linking bond or a divalent linking group -(CH₂)ₜₙLₐ(CH₂)ₜₙ- at each occurrence; the Lₐ is selected from the group consisting of -OC(=O)-, -C(=O)O-, -OC(=O)O-, -CH(OH)-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-; wherein, s is 1, 2, 3, or 4; tn is independently an integer from 0 to 12 at each occurrence; L₁ and L₃ are not linking bonds simultaneously; L₂ and L₄ are not linking bonds simultaneously;
R₁ and R₂ are each independently a C₅₋₃₀ hydrocarbon group or a C₅₋₃₀ hydrocarbon derivative group at each occurrence;
c is 2, 3, or 4.

1.1. In a specific embodiment of the present application, provided herein is a cationic lipid, wherein the structure is represented by the general formula (1-A-1): 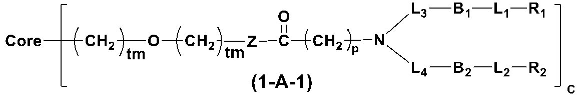 wherein, tm is independently an integer from 1 to 5 at each occurrence; Z is -O- or -NH-; the definitions of other symbols are the same as those in formula (1-A).

### 1.2. Core

In the present application, Core is an azacyclic core.

In a specific embodiment of the present application, the aforementioned Core 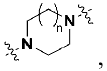 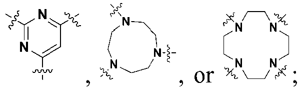 wherein, n is 1 or 2; Core is preferably 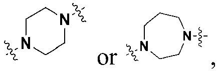 most preferably

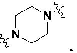

**1.3.** L
In the present application, L is a divalent linking group L_{d}.

In a specific embodiment of the present application, the L is selected from the group consisting of -(CH₂)ₜₘ-, -(CH₂)ₜₘO-, -(CH₂)ₜₘC(=O)NH-, -(CH₂)ₜₘOC(=O)NH-, -(CH₂)ₜₘNHC(=O)NH-, -(CH₂)ₜₘO(CH₂)ₜₘ-, -(CH₂)ₜₘC(=O)(CH₂)ₜₘ-, -(CH₂)ₜₘC(=O)O(CH₂)ₜₘ-, -C(=O)(CH₂)ₜₘC(=O)(CH₂)ₜₘ-, -(CH₂)ₜₘC(=O)O(CH₂)ₜₘO-, -(CH₂)ₜₘOC(=O)(CH₂)ₜₘ-, -(CH₂)ₜₘC(=O)NH(CH₂)ₜₘ-, -(CH₂)ₜₘNHC(=O)(CH₂)ₜₘ-, -(CH₂)ₜₘOC(=O)O(CH₂)ₜₘ-, -(CH₂)ₜₘNHC(=O)O(CH₂)ₜₘ-, -(CH₂)ₜₘOC(=O)NH(CH₂)ₜₘ-, -(CH₂)ₜₘC(=O)O(CH₂)ₜₘO(CH₂)ₜₘ-, -(CH₂)ₜₘNH-, -(CH₂)ₜₘO(CH₂)ₜₘNH-, -(CH₂)ₜₘOC(=O)NH(CH₂)ₜₘ-, -(CH₂)ₜₘNHC(=O)NH(CH₂)ₜₘ-, and -(CH₂)ₜₘO(CH₂)ₜₘO-, wherein the left end is connected to the azacycle; preferably, L is selected from the group consisting of -(CH₂)ₜₘO(CH₂)ₜₘ-, -(CH₂)ₜₘC(=O)O(CH₂)ₜₘ-, -(CH₂)ₜₘO-, -(CH₂)ₜₘC(=O)O(CH₂)ₜₘO-, -(CH₂)ₜₘNH-, -(CH₂)ₜₘC(=O)O(CH₂)ₜₘO(CH₂)ₜₘ-, -(CH₂)ₜₘO(CH₂)ₜₘNH-, -(CH₂)ₜₘOC(=O)(CH₂)ₜₘ-, and -(CH₂)ₜₘO(CH₂)ₜₘO-; preferably, L is selected from the group consisting of -CH₂C(=O)O(CH₂)₄-, -CH₂C(=O)O(CH₂)₃-, -CH₂C(=O)O(CH₂)₂O(CH₂)₂-, -(CH₂)₂O-, -(CH₂)₂NH-, -(CH₂)₂O(CH₂)₂NH-, -CH₂C(=O)O(CH₂)₂O-, and -(CH₂)₂O(CH₂)₂O-, wherein the left end is connected to the azacycle.

**1.4. B₁**, **B₂**
In the present application, B₁ and B₂ are each independently a linking bond or a C₁₋₂₀ alkylene group.

In a specific embodiment of the present application, the B₁ and B₂ are selected from any of the following cases:
Case (1): B₁ and B₂ are each independently a C₁₋₂₀ alkylene group, more preferably a C₂₋₁₀ alkylene group;
Case (2): one of B₁ and B₂ is a linking bond, and the other is a C₁₋₂₀ alkylene group;
Case (3): both B₁ and B₂ are linking bonds;
B₁ and B₂ are more preferably selected from the aforementioned Case (1) or Case (3);
wherein, the aforementioned C₂₋₁₀ alkylene group is preferably selected from the group consisting of an ethylene group, a propylene group, a butylene group, a pentylene group, a hexylene group, a heptylene group, and an octylene group.

**1.5. L₁, L₂**
In the present application, L₁ and L₂ are each independently a linking bond or Lₐ, wherein the Lₐ is selected from the group consisting of -OC(=O)-, -C(=O)O-, -OC(=O)O-, -CH(OH)-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-.

In a specific embodiment of the present application, L₁ and L₂ are preferably selected from any of the following cases:
Case (1): L₁ and L₂ are each independently Lₐ;
Case (2): one of L₁ and L₂ is a linking bond, and the other is Lₐ;
Case (3): both L₁ and L₂ are linking bonds;
more preferably, L₁ and L₂ are each independently selected from the group consisting of a linking bond, -C(=O)-, -O-, -NH-, -OC(=O)-, -C(=O)O-, -OC(=O)O-, -CH(OH)-, -OC(=O)NH-, -NHC(=O)O-, -NHC(=O)-, and -C(=O)NH-.

**1.6. L₃**, **L₄**
In the present application, L₃ and L₄ are each independently selected from the group consisting of a linking bond, -Lₐ-, -(CH₂)ₜₙLₐ-, -Lₐ(CH₂)ₜₙ-, and -(CH₂)ₜₙLₐ(CH₂)ₜₙ-; wherein, tn is an integer from 1 to 12.

In a specific embodiment of the present application, preferably, L₃ and L₄ are each independently selected from the group consisting of -(CH₂)ₜₙC(=O)-, -(CH₂)ₜₙC(=O)O-, -(CH₂)ₜₙOC(=O)-, -(CH₂)ₜₙOC(=O)O-, -(CH₂)ₜₙOC(=O)NH-, and -(CH₂)ₜₙNHC(=O)O-, wherein the right end is connected to B₁ or B₂; preferably, L₃ and L₄ are each independently selected from the group consisting of a linking bond, -CH₂CH(OH)-, -(CH₂)₂C(=O)NH-, -C(=O)-, and -C(=O)O-; more preferably, L₃ and L₄ arethe same, and each a linking bond, -CH₂CH(OH)-, or -(CH₂)₂C(=O)NH-.

**1.7. R₁**, **R₂**
In the present application, R₁ and R₂ are each independently a C₅₋₃₀ linear hydrocarbon group, a C₅₋₃₀ branched hydrocarbon group, or a C₅₋₃₀ hydrocarbon derivative group; the C₅₋₃₀ hydrocarbon derivative group is 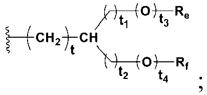 the linear hydrocarbon group and branched hydrocarbon group are each independently substituted or unsubstituted, wherein the substitution is preferably a C₁₋₆ alkyl group, halogen, or a hydroxyl group; the hydrocarbon group is preferably an alkyl, alkenyl, or alkynyl group.

In a specific embodiment of the present application, the linear hydrocarbon group is selected from the group consisting of a linear alkyl group, a linear alkenyl group, and a linear alkynyl group, more preferably a C₅₋₂₅ linear hydrocarbon group.

In a specific embodiment of the present application, the branched hydrocarbon group is selected from the group consisting of a branched alkyl group, a branched alkenyl group, and a branched alkynyl group, each independently represented by

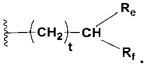

In the aforementioned R₁ and R₂, t is an integer from 0 to 12, t₁ and t₂ are each independently an integer from 0 to 5, t₃ and t₄ are each independently 0 or 1, and t₁, t₂, t₃, and t₄ are not simultaneously zero; Rₑ and R_{f} are each independently selected from the group consisting of a C₁₋₁₅ alkyl group, a C₂₋₁₅ alkenyl group, and a C₂₋₁₅ alkynyl group; more preferably, Rₑ and R_{f} are each independently selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a vinyl group, a propenyl group, a butenyl group, a pentenyl group, a hexenyl group, a heptenyl group, an octenyl group, a nonenyl group, a decenyl group, an ethynyl group, a propynyl group, a butynyl group, a pentynyl group, a hexynyl group, a heptynyl group, an octynyl group, a nonynyl group, and a decynyl group; more preferably, Rₑ and R_{f} are each independently selected from the group consisting of a methyl group, an ethyl group, a propyl group, a butyl group, a pentyl group, a hexyl group, a heptyl group, and an octyl group.

In a specific embodiment of the present application, R₁ and R₂ are preferably selected from any of the following cases:
Case (1): R₁ and R₂ are each independently a C₅₋₃₀ linear hydrocarbon group;
Case (2): one of R₁ and R₂ is a C₅₋₃₀ linear hydrocarbon group, and the other is a C₅₋₃₀ branched hydrocarbon group represented by 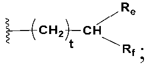
Case (3): R₁ and R₂ are each independently a C₅₋₃₀ branched hydrocarbon group represented by 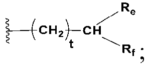
Case (4): R₁ and R₂ are each independently represented by 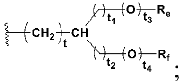
Case (5): one of R₁ and R₂ is a C₅₋₃₀ linear hydrocarbon group or a C₅₋₃₀ branched hydrocarbon group represented by 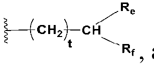 and the other is

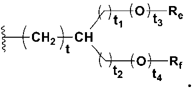

In a specific embodiment of the present application, the R₁ and R₂ selected from the above cases are more preferably, each independently selected from the group consisting of the following structures:

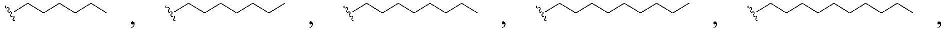

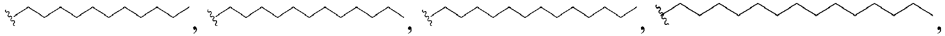

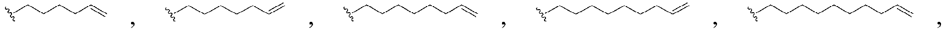

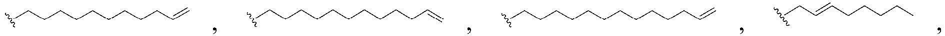

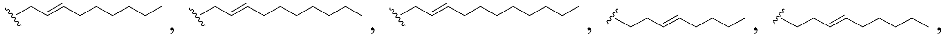

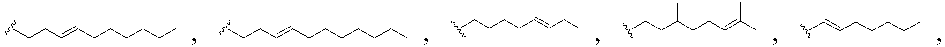

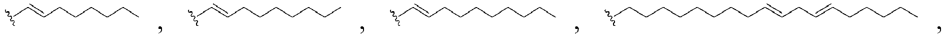

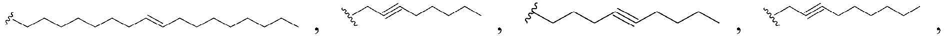

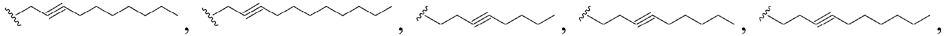

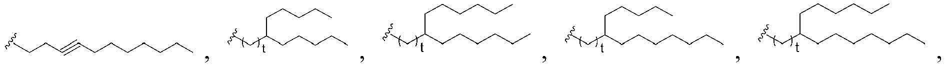

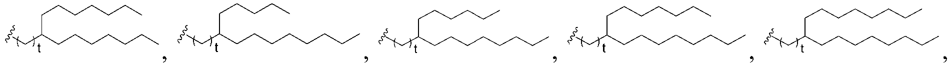

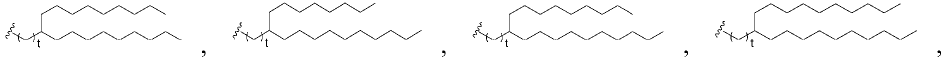

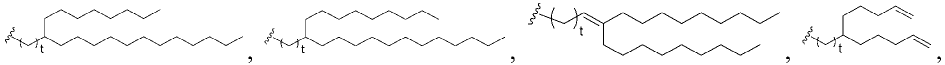

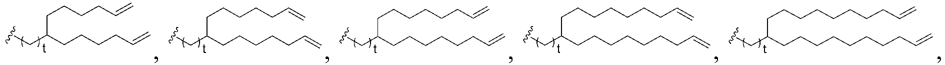

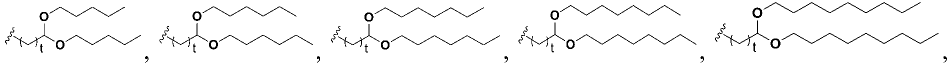

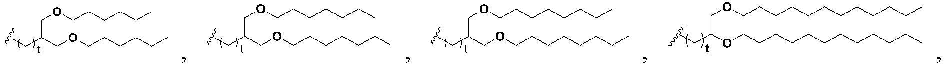

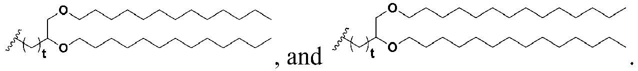

### 1.8. Fragments -B₁-L₁-R₁ and -B₂-L₂-R₂

In a specific embodiment of the present application, at each occurrence, the fragments -L₃-B₁-L₁-R₁ and -L₄-B₂-L₂-R₂ are each independently selected from the group consisting of the following structures:

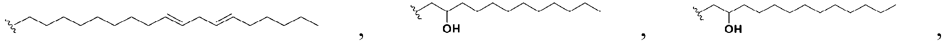

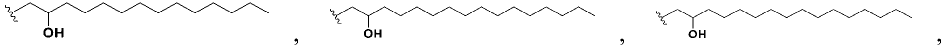

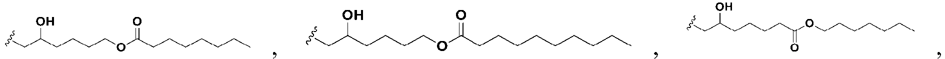

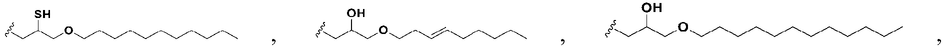

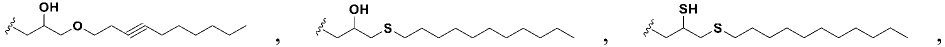

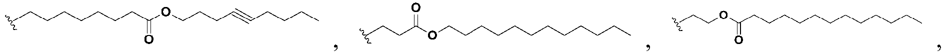

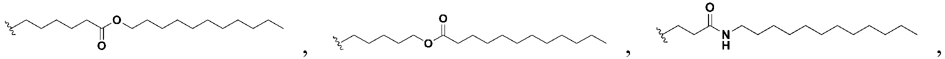

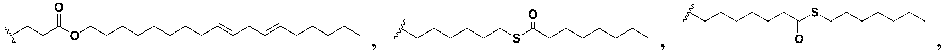

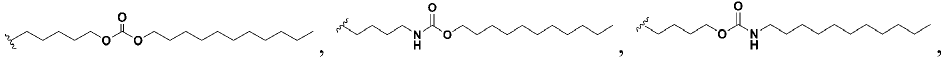

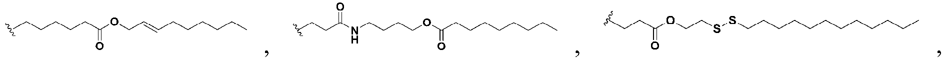

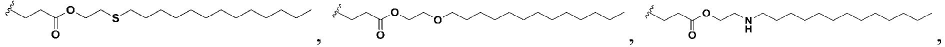

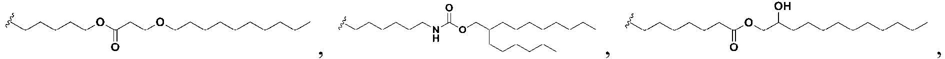

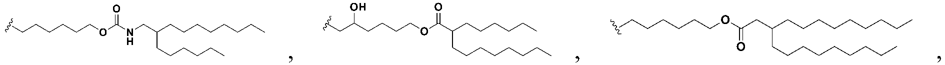

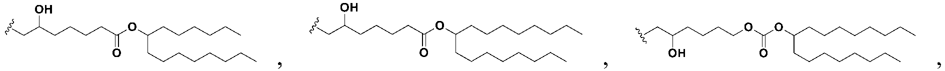

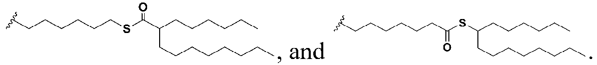

### 1.9. Examples of general formulas of structures

In a specific embodiment of the present application, the cationic lipid satisfies any one of the following general formulas: 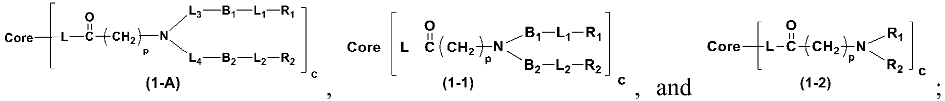 wherein, L₁, L₂, L₃, L₄, B₁, and B₂ are not linking bonds; the definitions of other symbols are the same as those in formula (1-A).

In a specific embodiment of the present application, the aforementioned cationic lipid is preferably satisfies any one of the following general formulas: 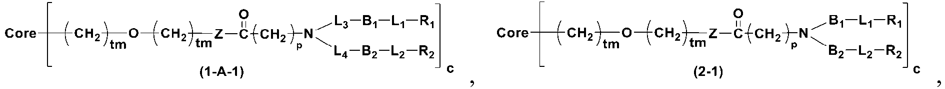 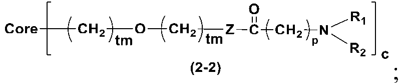 more preferably, the cationic lipid is satisfies any one of the following general formulas: 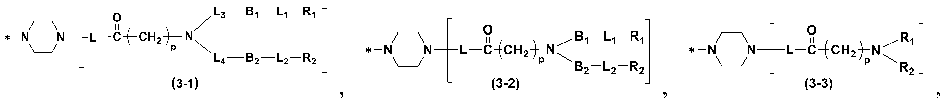 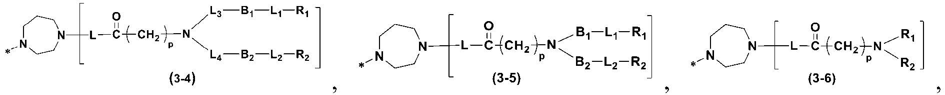 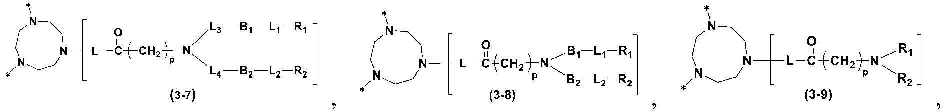 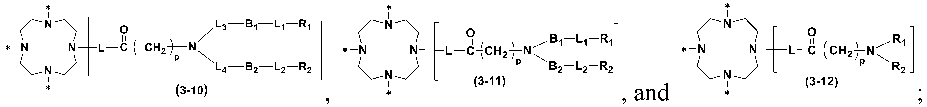 most preferably, the cationic lipid is preferably selected from the group consisting of the following general formulas: 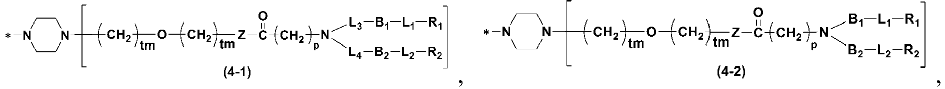 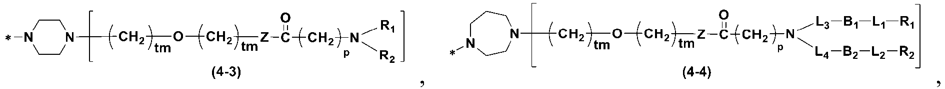 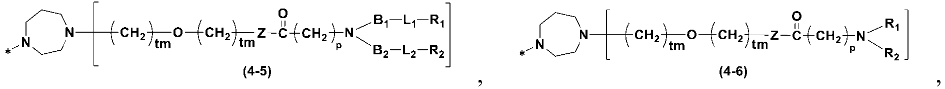 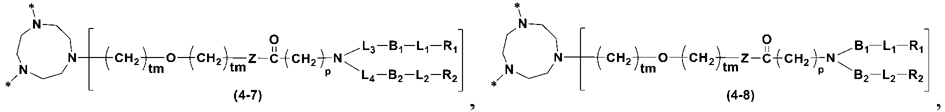 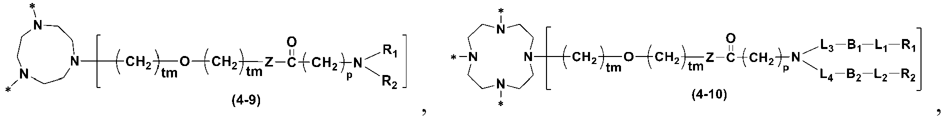 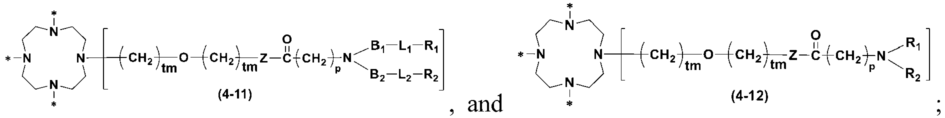 wherein, * refers to the same structure as in [ ]; at each occurrence, L₁ and L₂ in all of the aforementioned general formulas are preferably each independently selected from the group consisting of -C(=O)-, -O-, -NH-, -C(=O)O-, -OC(=O)-, -OC(=O)O-, -C(=O)NH-, -NHC(=O)-, -OC(=O)NH-, and -NHC(=O)O-; more preferably, L₁ and L₂ are each independently selected from the group consisting of -C(=O)-, -C(=O)O-, -OC(=O)-, and -OC(=O)O-;
more preferably, the structure of a cationic lipid conforms to any one of formulas (4-1) to (4-3); preferably, in formula (4-1), L₃ and L₄ are each independently -CH₂CH(OH)-, L₁ and L₂ are selected from the group consisting of -C(=O)O-, -OC(=O)-, and -OC(=O)O-; R₁ and R₂ of formula (4-3) are each independently a C₅₋₂₅ alkenyl group or a C₅₋₂₅ alkynyl group.

### 1.10. Examples of specific structures

In a specific embodiment of the present application, the cationic lipid is preferably selected from the group consisting of the following structures: 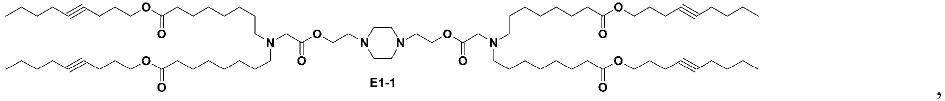 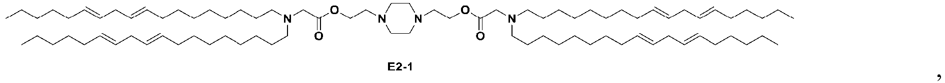 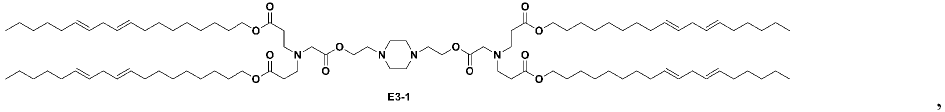 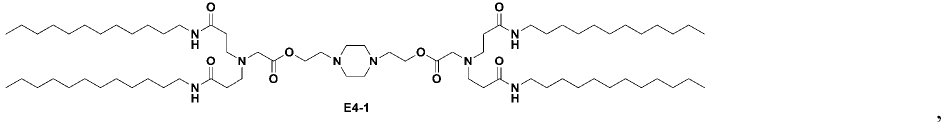 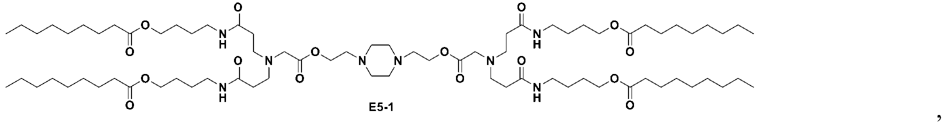 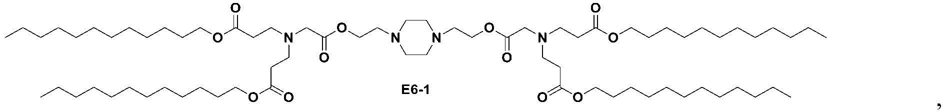 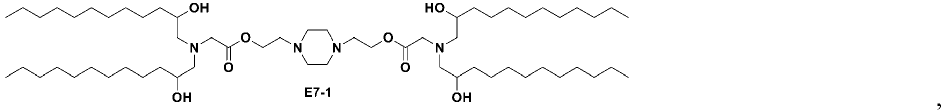 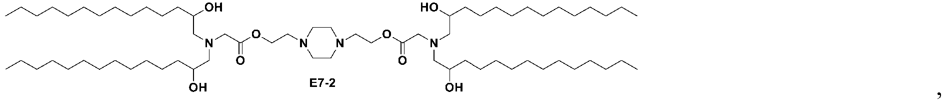 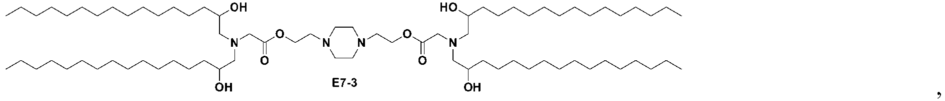 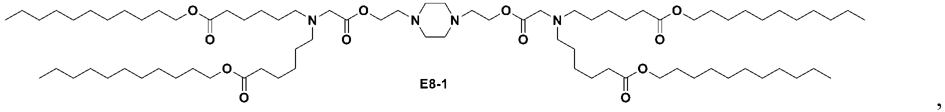 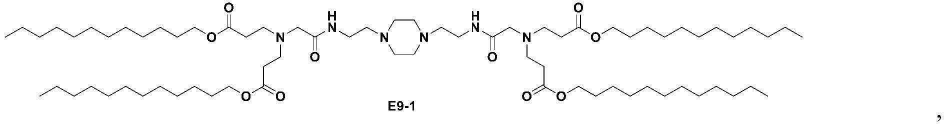 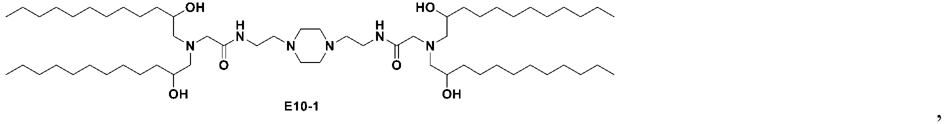 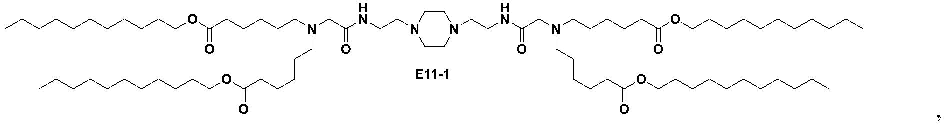 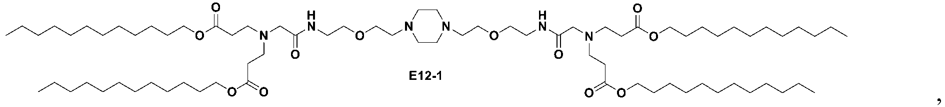 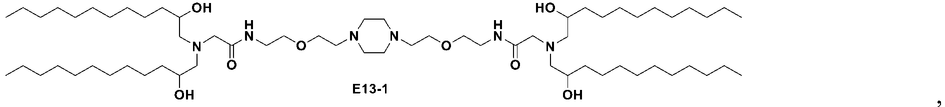 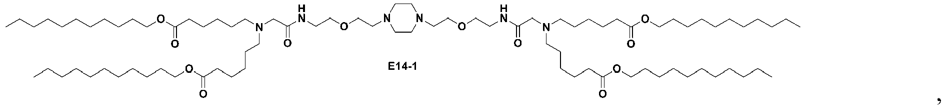 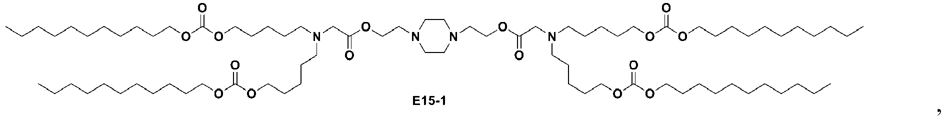 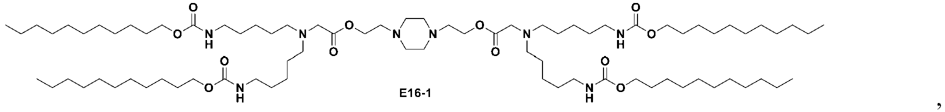 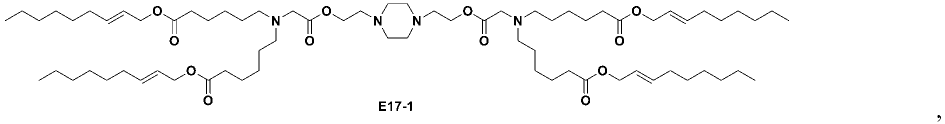 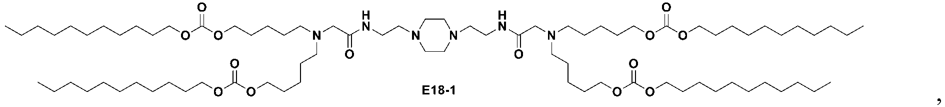 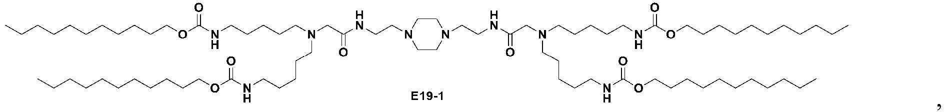 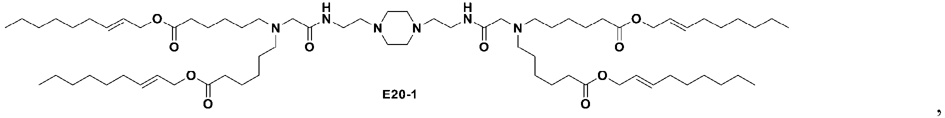 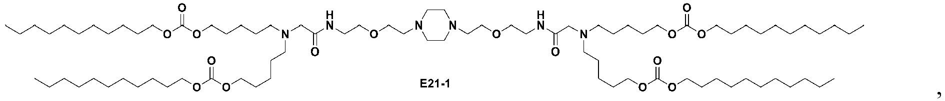 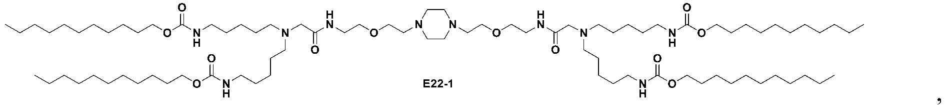 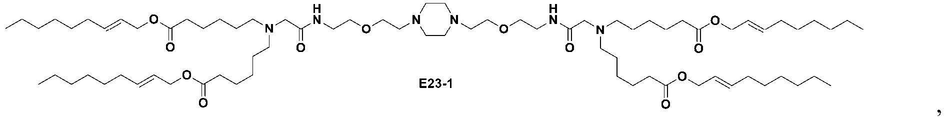 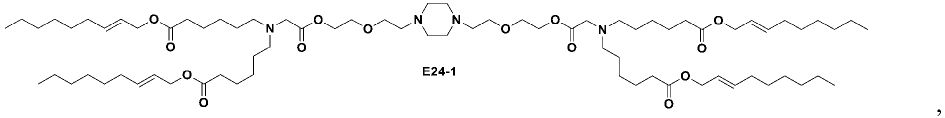 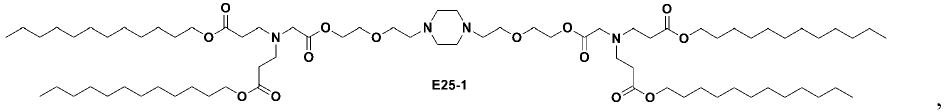 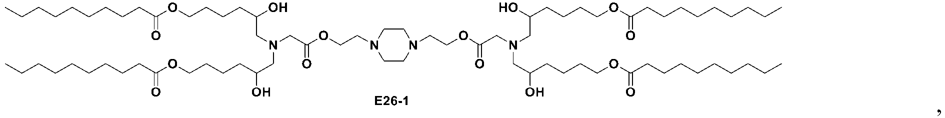 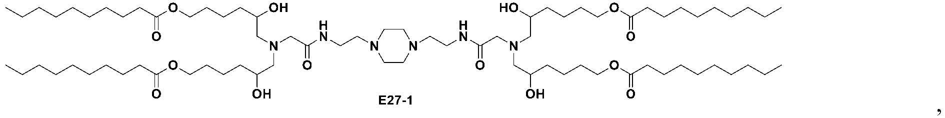 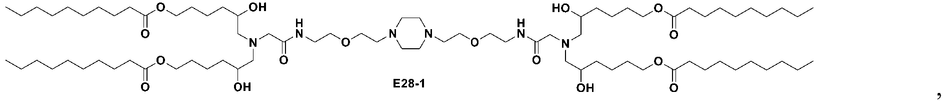 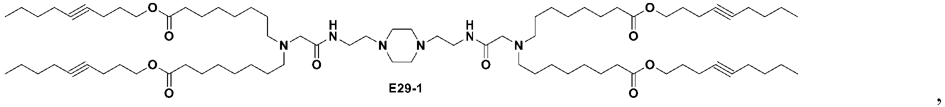 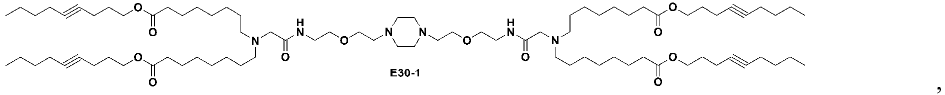 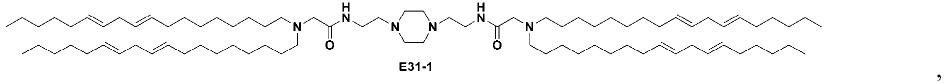 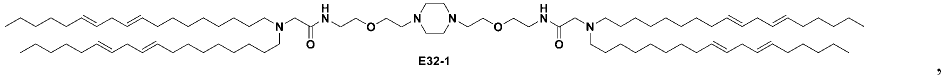 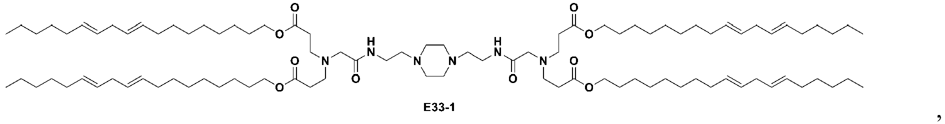 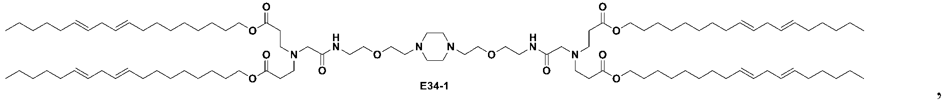 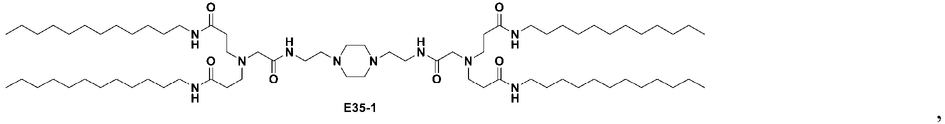 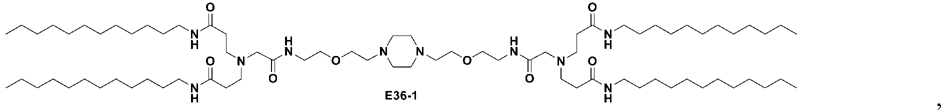 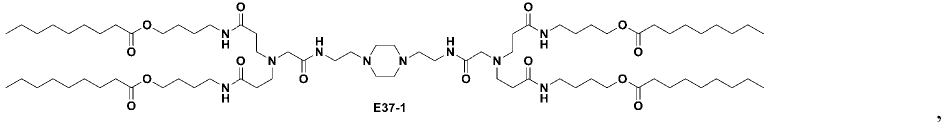 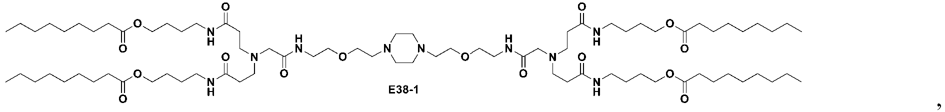 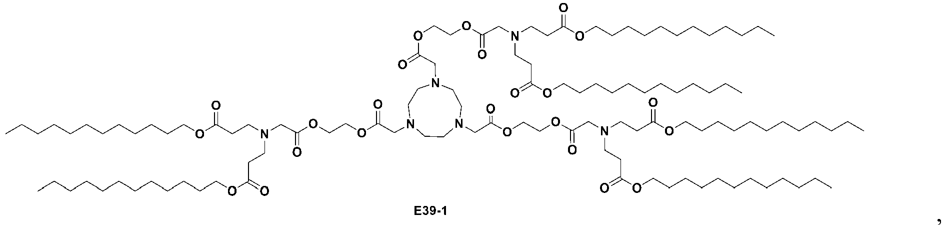 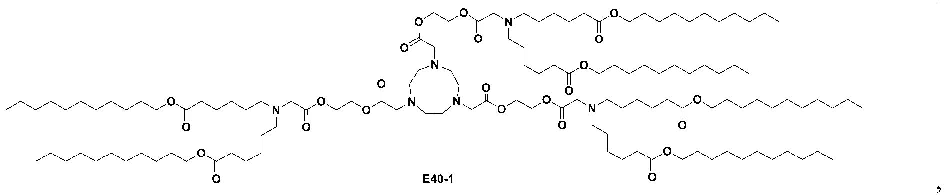 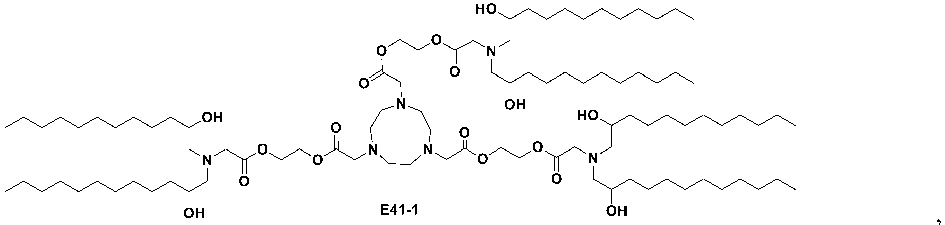 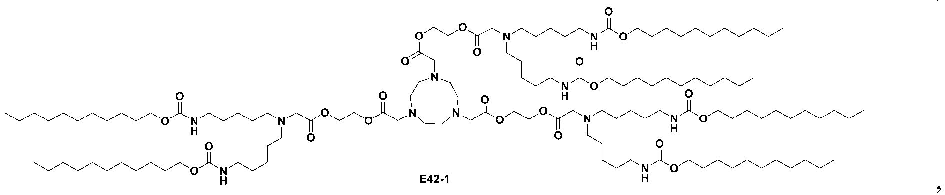 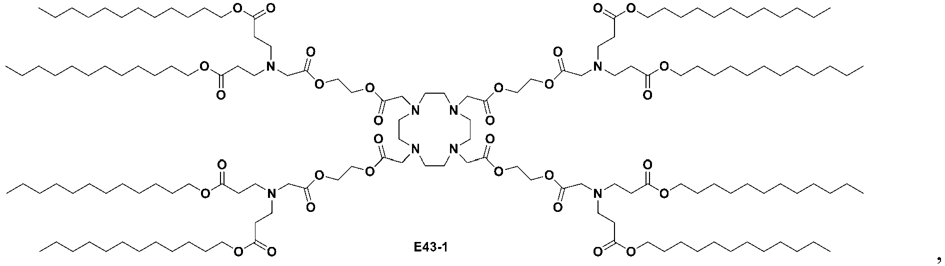 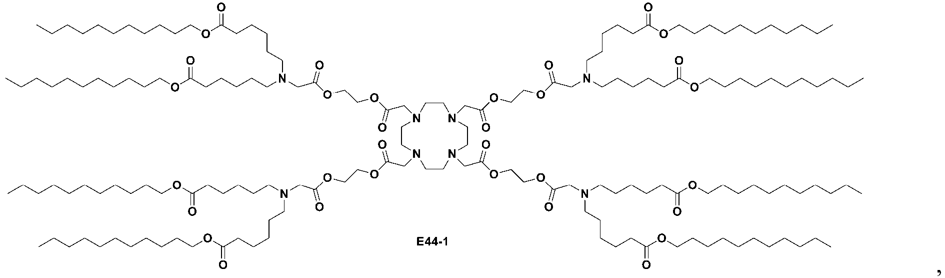 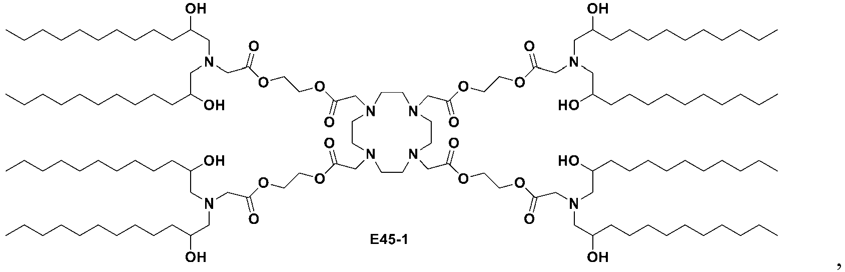 and 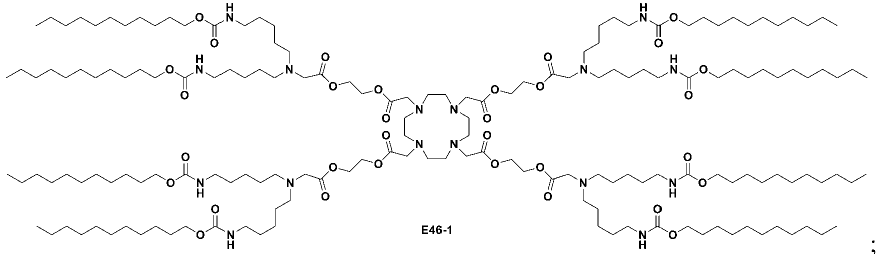 or the cationic lipid is selected from the group consisting of the following structures: 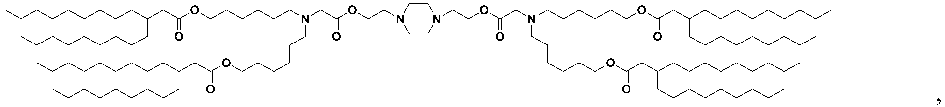 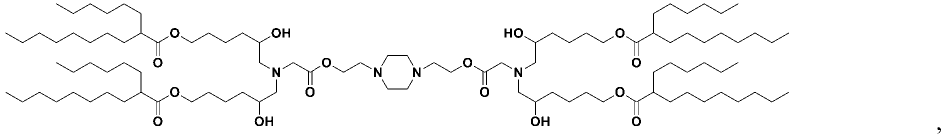 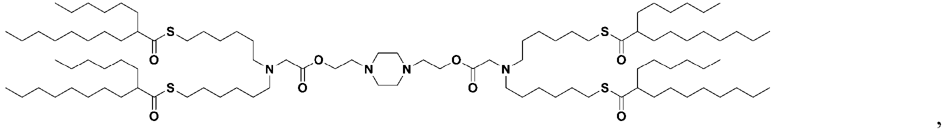 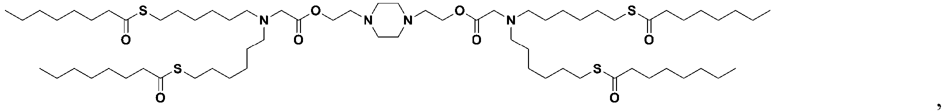 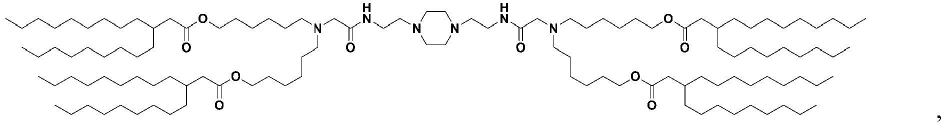 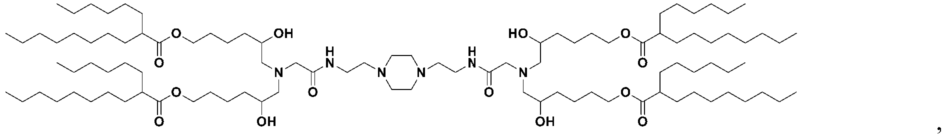 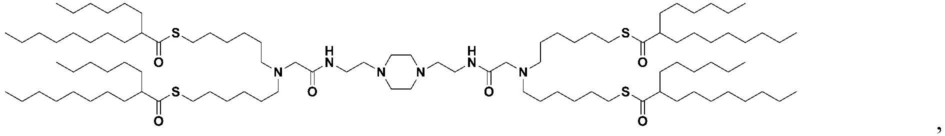 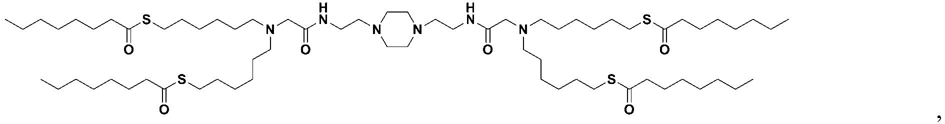 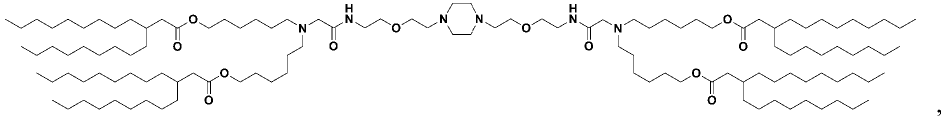 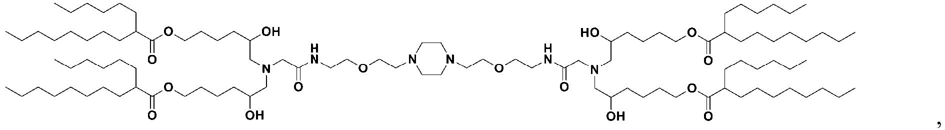 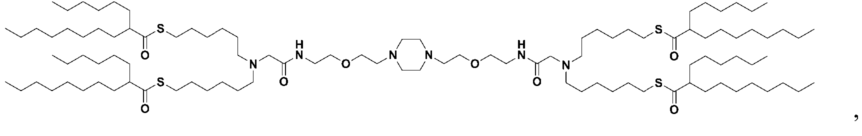 and 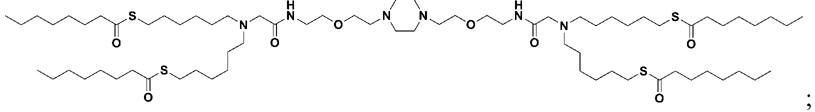 or the cationic lipid is selected from the group consisting of the following structures: 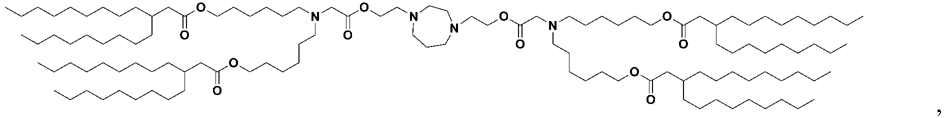 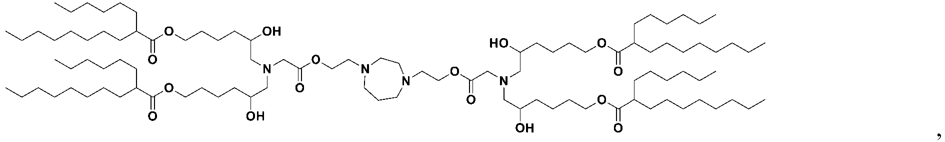 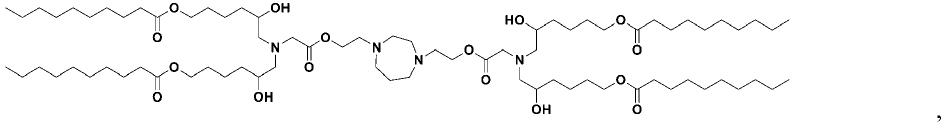 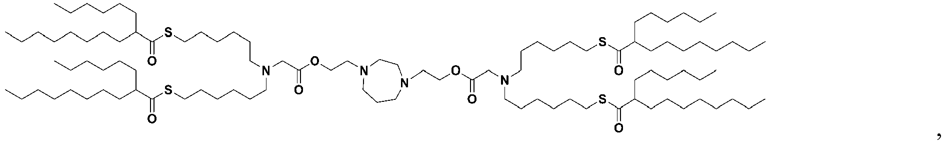 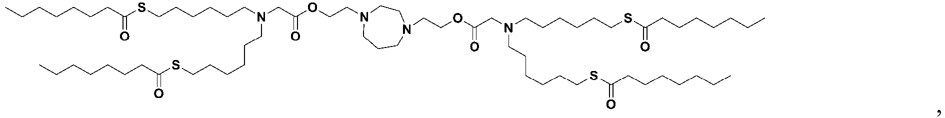 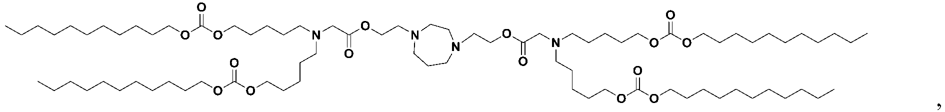 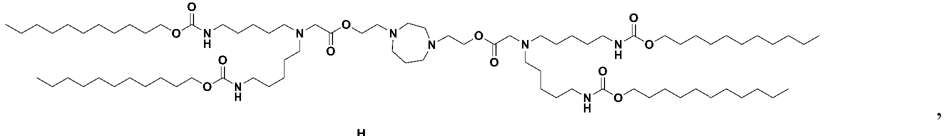 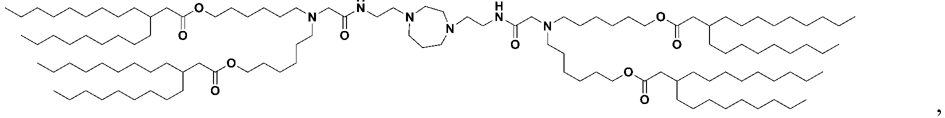 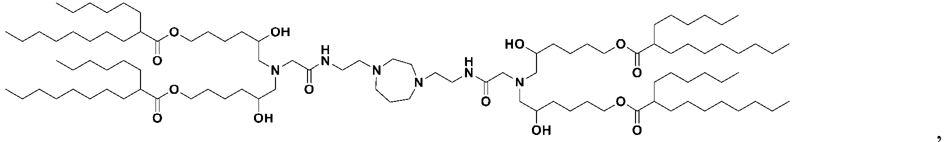 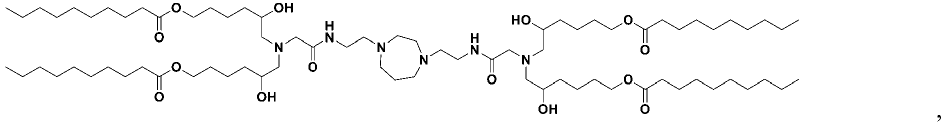 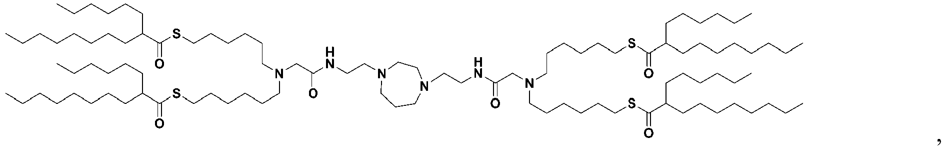 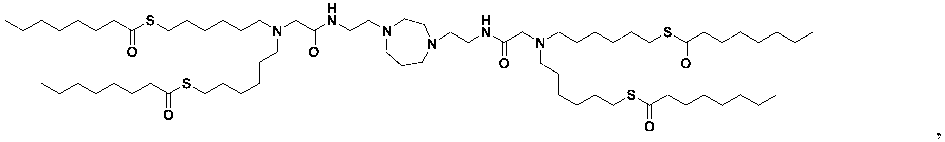 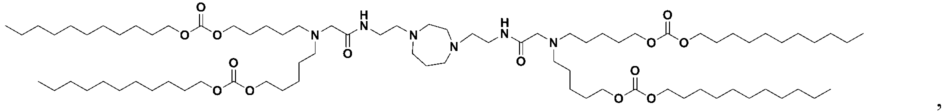 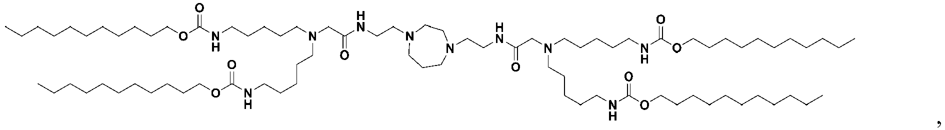 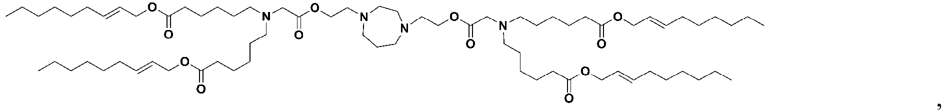 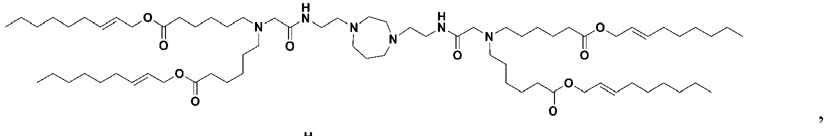 and 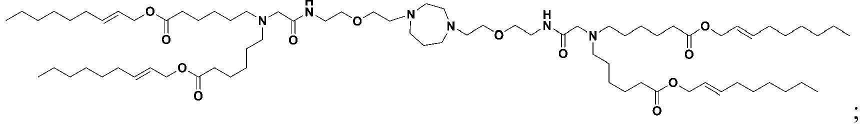 or the cationic lipid is selected from the group consisting of the following structures: 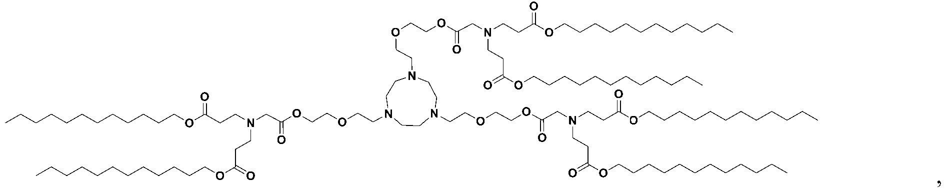 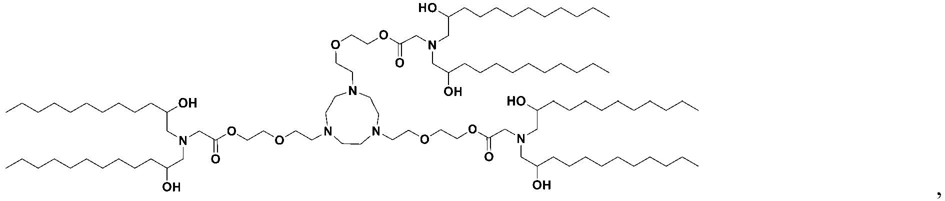 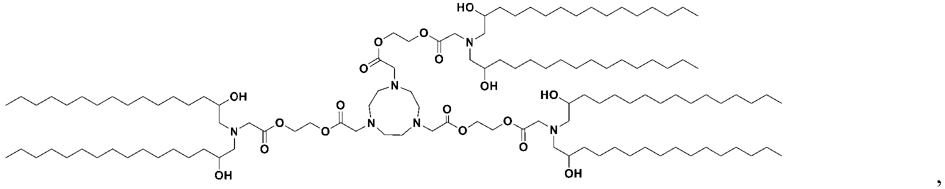 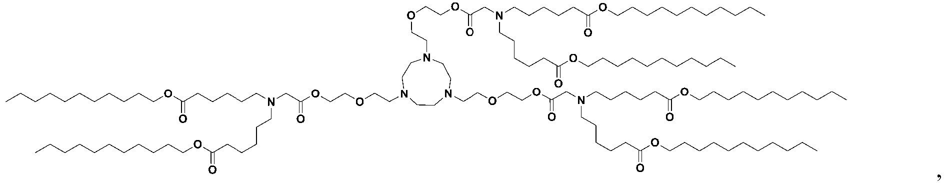 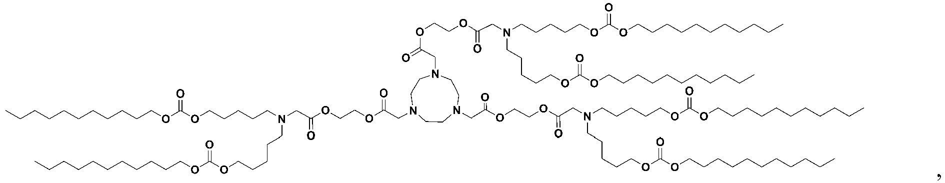 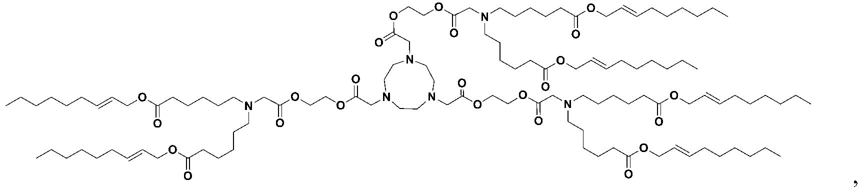 and 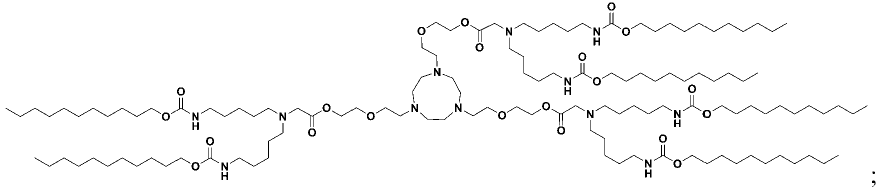 or the cationic lipid is selected from the group consisting of the following structures: 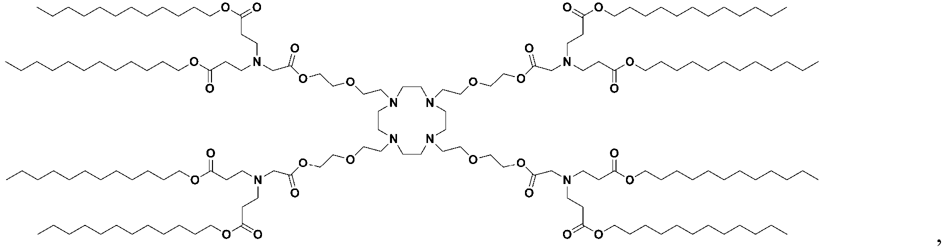 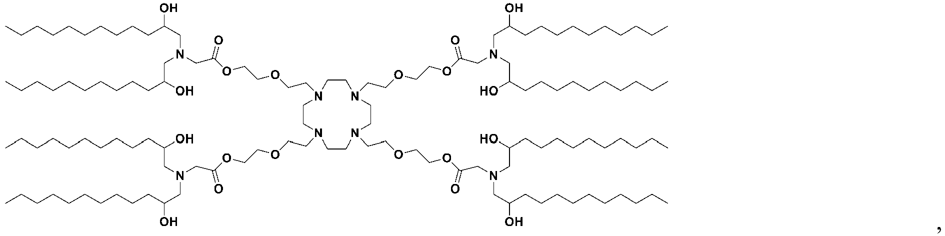 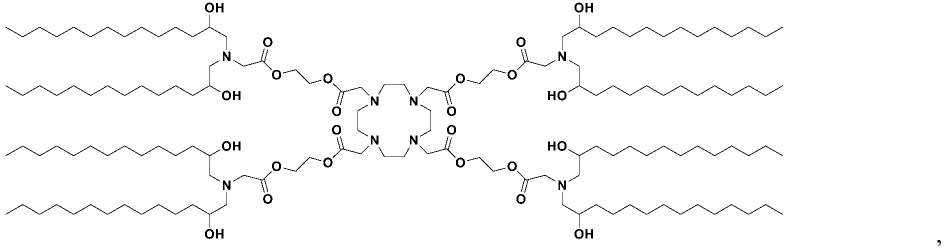 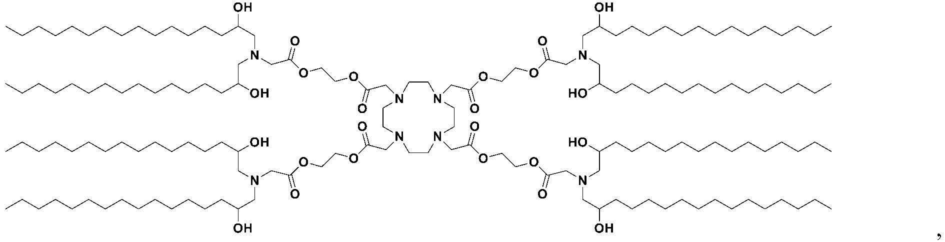 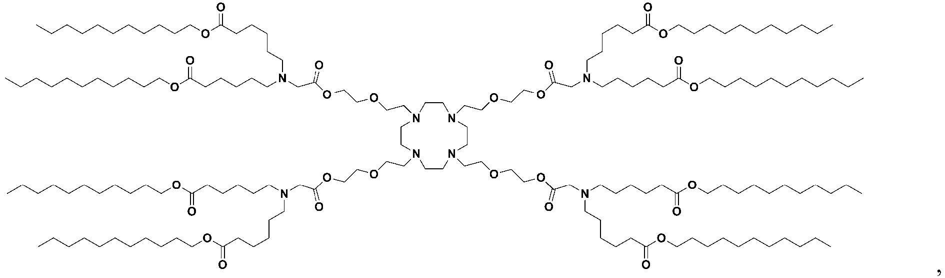 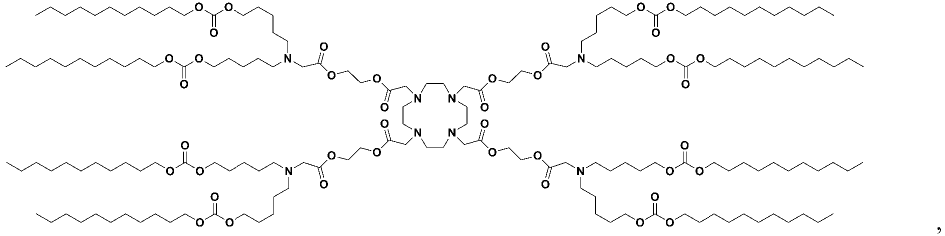 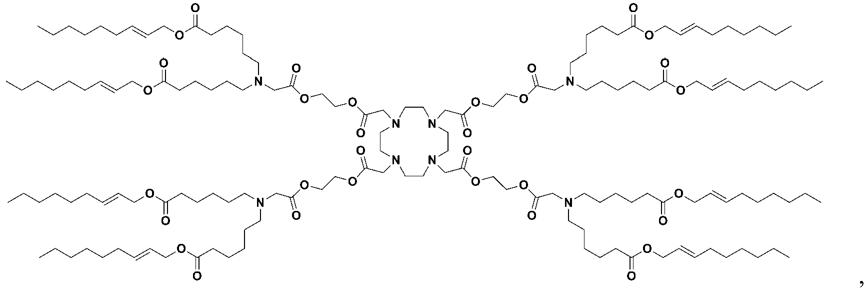 and

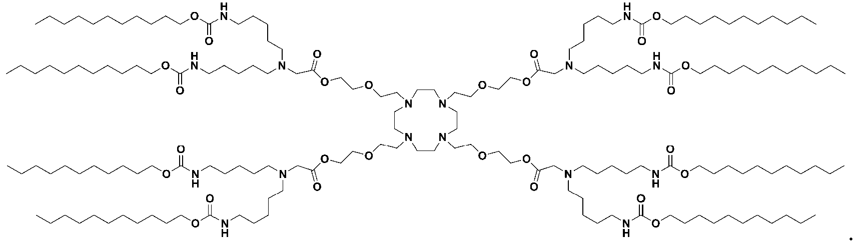

### 2. Preparation of Cationic Lipids

**2.1.** The intermediates/starting materials involved in the preparation process of cationic lipids of the present application include but are not limited to CORE₀, IM-N, IM-AA', and CORE-AA'.

The preparation process of the present application may involve a starting material CORE₀ containing an azacyclic core. The CORE₀ contains c identical instances of reactive group F₀; wherein, F₀ is a reactive group that can react with the terminal group of amino acids or amino acid derivatives, and is preferably a protected or unprotected form of -OH, -COOH, -NH₂, or -NH-; c is 2, 3, or 4. CORE₀ can be obtained by purchase, or though further modification of a compound containing an azacycle. The modification refers to a preparation process with any suitable single-step or multi-step chemical reactions. For example, in Example 12, CORE₀ 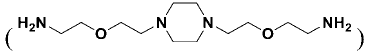 can be obtained by performing a substitution reaction between S12-1 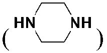 and S12-2 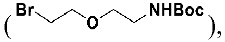 followed by the removal of Boc protecting group.

In the present application, CORE₀ is selected from the group consisting of the following structures: 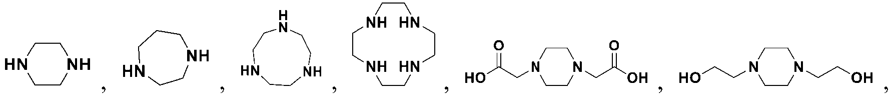 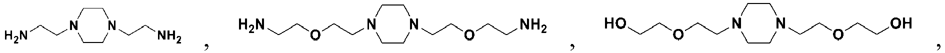 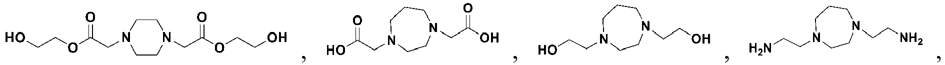 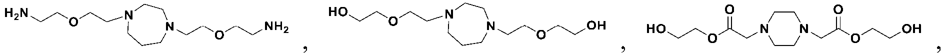 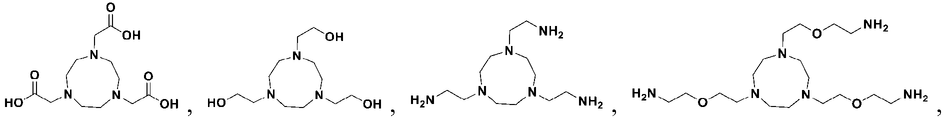 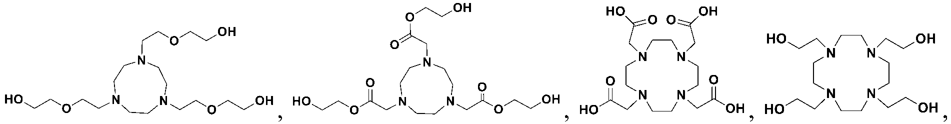 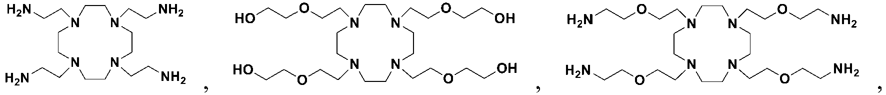 and

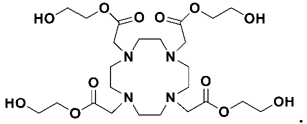

The preparation process of the present application may involve a small-molecule intermediate IM-N with a reactive group and a hydrophobic hydrocarbon tail chain, and its structure is represented by F_{c}-B₁-L₁-R₁ or F_{N}-B₂-L₂-R₂; wherein, F_{C} and F_{N} are reactive groups that can react with the terminal group of amino acids or amino acid derivatives, preferably selected from -OH, -COOH, -Br, -CHO, -CH=CH-, and 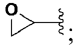 the definitions of other symbols are the same as those in formula (1-A). IM-N can be prepared by any suitable chemical reaction, which includes simple esterification, amidation, alkylation, addition, or substitution reactions, etc., either as single-step or multi-step processes. For example, in Example 1, the small-molecule intermediate IM-N 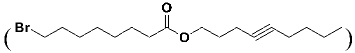 can be obtained by performing a condensation reaction between S1-4 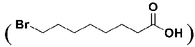 and S1-5

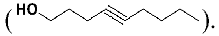

In the present application, the structure of the aforementioned IM-N is preferably selected from the group consisting of F_{c}-R₁, F_{N}-R₂, F_{C}-L₁-R₃, F_{N}-L₂-R₂, F_{C}-B₁-L₁-R₁, and F_{N}-B₂-L₂-R₂; specifically, IM-N is more preferably selected from the group consisting of the following structures:

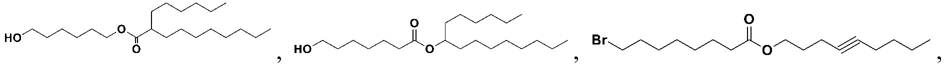

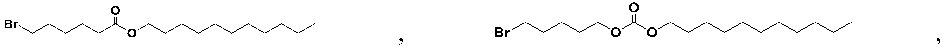

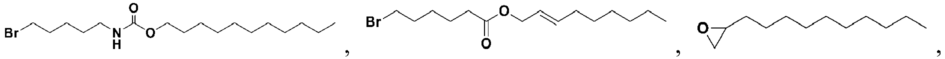

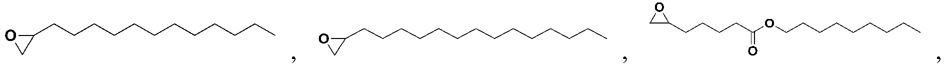

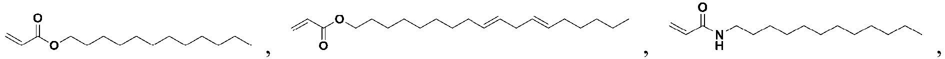

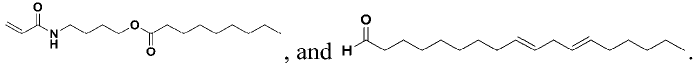

The preparation process of the present application may involve an intermediate IM-AA' with one reactive amino acid terminal group and at least two hydrophobic hydrocarbon tail chains, and its structure is represented by 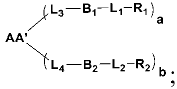 wherein, AA' contains one reactive amino acid terminal group, and the definitions of other symbols are the same as those in formula (1-A). IM-AA' can be obtained by modifying amino acids or their derivatives with IM-N through a single or multiple steps, which may involve identical or different reactions.

In the present application, the structure of the aforementioned IM-AA' is preferably selected from the group consisting of 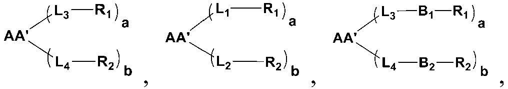 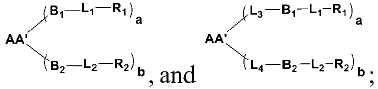 specifically, IM-AA' is more preferably selected from the group consisting of the following structures:

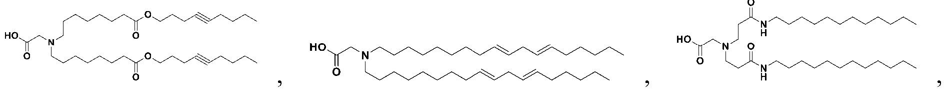

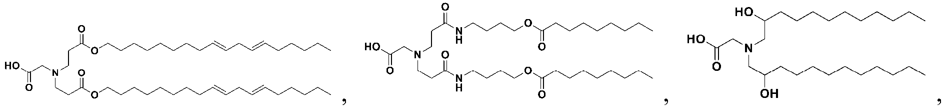

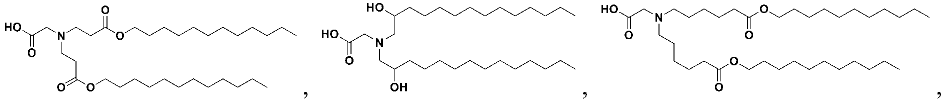

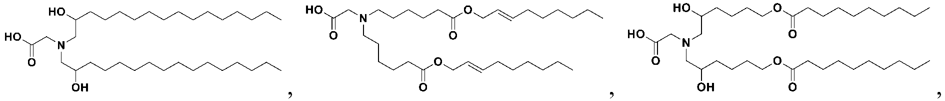

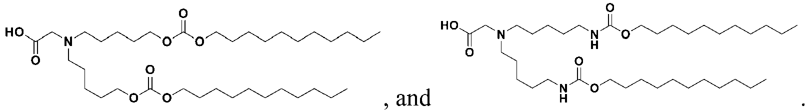

The preparation process of the present application may involve an intermediate IM-AA with one amino acid residue and at least two hydrophobic hydrocarbon chains, and its structure is represented by 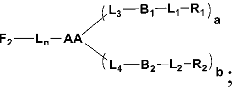 wherein, Lₙ is any suitable divalent linking group, F₂ is a reactive group that can react with the F₀ of CORE₀, and the definitions of other symbols are the same as those in formula (1-A). IM-AA can be obtained by functionalizing the reactive amino acid terminal group of IM-AA'.

In the present application, the structure of the aforementioned IM-AA' is preferably selected from the group consisting of 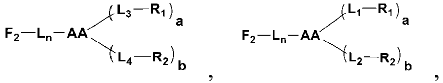 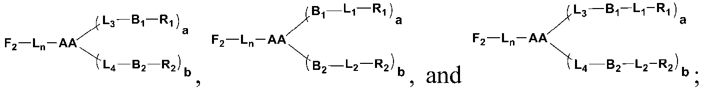 specifically, IM-AA is more preferably selected from the group consisting of the following structures: 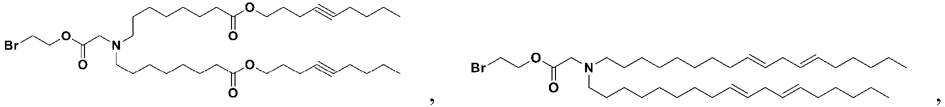 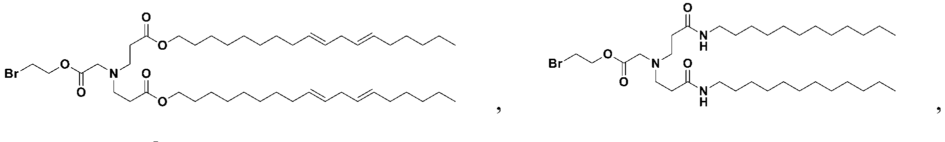 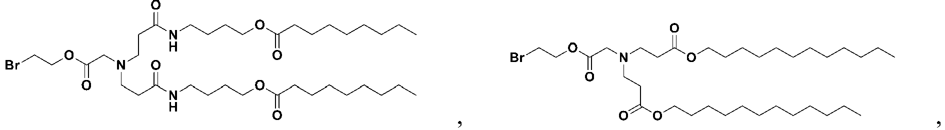 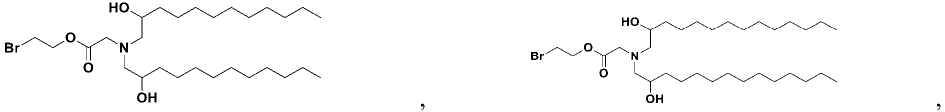 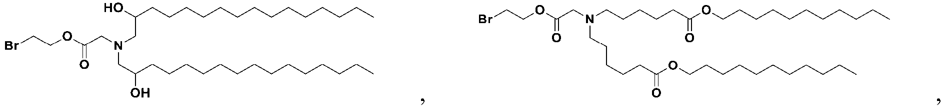 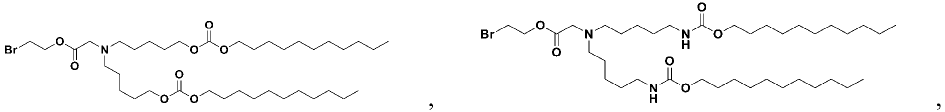 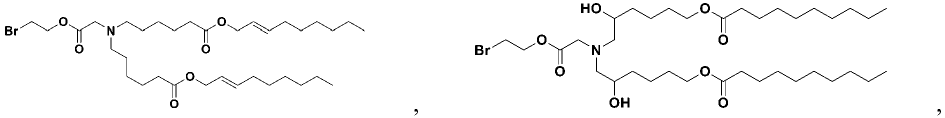 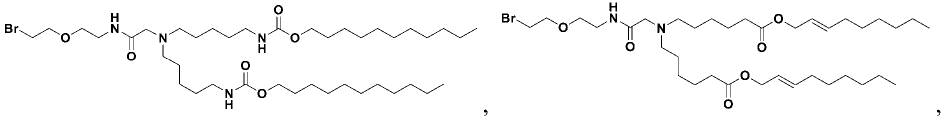 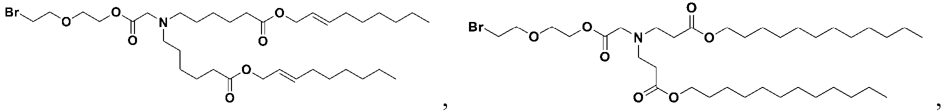 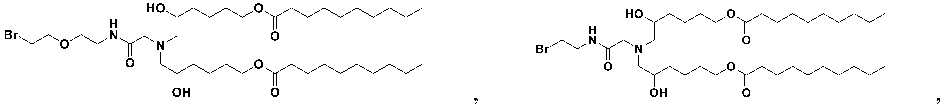 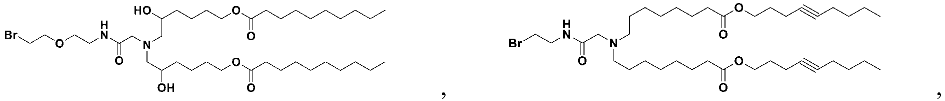 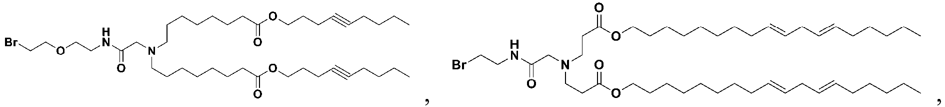 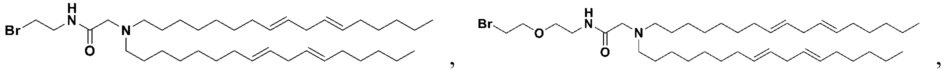 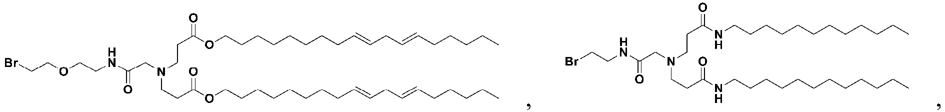 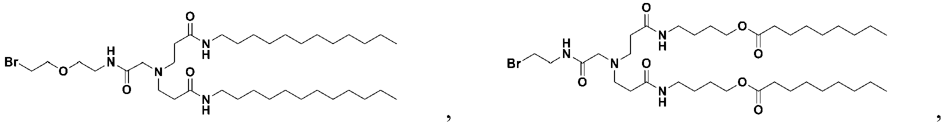 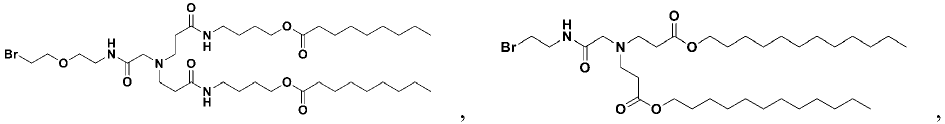 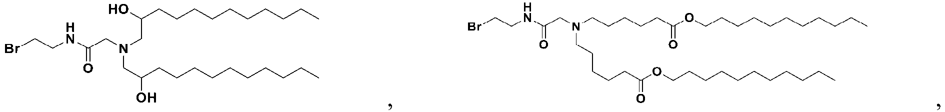 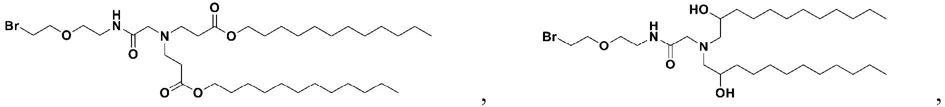 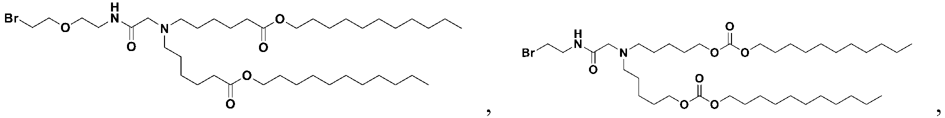 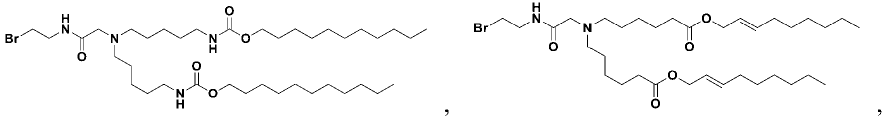 and

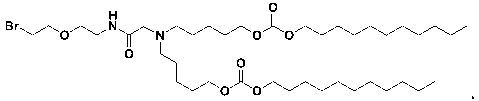

The preparation process of the present application may involve an intermediate CORE-AA' containing an azacycle, and its structure is represented by 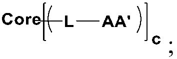 wherein, AA' contains one reactive amino acid terminal group, and the definitions of other symbols are the same as those in formula (1-A). CORE-AA' can be obtained by reacting CORE₀ with amino acids or their derivatives through either a single reaction or multiple reactions that are identical or different to each other. For example, in Example 1, the small-molecule intermediate CORE-AA' 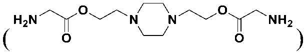 can be obtained by a condensation reaction between S1-1 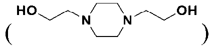 and S1-2 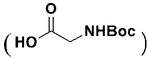 ) followed by the removal of Boc protecting group.

In the present application, CORE-AA' is selected from the group consisting of the following structures:

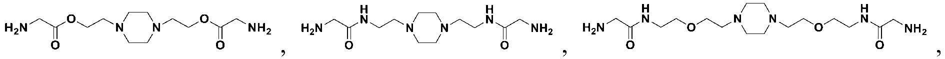

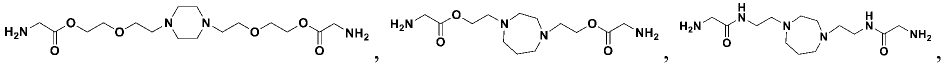

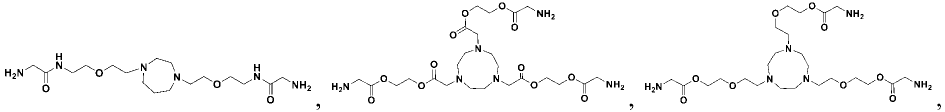

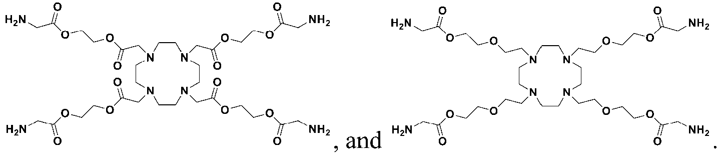

In the present application, the cationic lipid can be obtained by reacting any of the aforementioned CORE₀ with any of IM-AA' or IM-AA, and it can also be obtained by reacting any of the aforementioned CORE-AA' with any of IM-N.

### 2.2. Description of Relevant Starting Materials and/or Steps in the Preparation Process

### 2.2.1. Condensation Agents, Oxidizing Agents, and Reducing Agents

In the present application, the condensation agent used in reactions is not particularly limited, but is preferably selected from the group consisting of *N,N'*-diisopropylcarbodiimide (DIC), *N,N'*-dicyclohexylcarbodiimide (DCC), 1-ethyl-(3-dimethylaminopropyl)carbodiimide hydrochloride (EDC·HCl, EDCI), 2-(7-azabenzotriazole)-*N,N,N',N'*-tetramethyluronium hexafluorophosphate (HATU), and benzotriazole-*N,N,N',N'*-tetramethyluronium hexafluorophosphate (HBTU), most preferably DCC. Relevant reactions can be facilitated by the addition of an appropriate catalyst (e.g., 4-dimethylaminopyridine).

In the present application, the oxidizing agent used in reactions is not particularly limited, as long as it is a compound or a combination of compounds that can increase the valency of the substrate, and is preferably selected from the group consisting of phenyliodine(III) bis(trifluoroacetate), 1,4-benzoquinone, benzyltrimethylamonium tribromide, pyridine dichromate, potassium dichromate, ozone, oxygen, hypofluorous acid, sodium hypochlorite, cobalt(III) acetate, cobalt acetate, manganese acetate, palladium acetate, copper acetate, monoperoxyphthalic acid, iodine, *N*-iodosuccinimide, iodoylbenzene, 2-iodoylbenzoic acid, dimethyldioxirane, dimethyl sulfoxide-chloroformyl, dimethyl sulfoxide-acetic anhydride, DDQ, dichlorotris(triphenylphosphine) ruthenium, manganese dioxide, diacetoxyiodobenzene, periodic acid, sodium periodate, sodium periodate-osmium tetroxide, potassium permanganate, sodium perborate, benzoic acid peroxide, dibenzoyl peroxide, nickel peroxide, hydrogen peroxide, cumene hydroperoxide, *t*-butyl hydroperoxide, peracetic acid, *m*-chloroperbenzoic acid, *N*-chlorosuccinimide, pyridinium chlorochromate, palladium chloride-copper chloride, urea-hydrogen peroxide complex, trityl tetrafluoroborate, tributyltin oxide, cobalt trifluoride, vanadium oxytrifluoride, chromium trioxide, manganese triacetate, TEMPO, ammonium ceric nitrate, bromine, *N*-oxidopyridine, silver oxide, *O*-ethylcarbonate peroxide, manganese acetylacetonate, vanadium oxyacetylacetonate, aluminum isoproxide, potassium peroxymonosulfate, dichloroiodobenzene, etc., and combinations thereof; more preferably, the oxidizing agent is selected from the group consisting of oxygen, sodium hypochlorite, hydrogen peroxide, dichloroiodobenzene, potassium peroxymonosulfate, etc., and combinations thereof.

In the present application, there is no particular limitation on the reducing agent used in reactions, as long as it can reduce the Schiff base formed from the reaction between ammonia and aldehydes or ketones to an amino group, and is preferably selected from the group consisting of sodium borohydride, sodium cyanoborohydride, lithium aluminum hydride, borane, diborane, diisobutyl aluminum hydride, diisopine campyl borane, lithium borohydride, zinc borohydride, borane-pyridine, borane-methyl sulfide, borane-tetrahydrofuran, etc., and combinations thereof; the reducing agent is more preferably sodium cyanoborohydride.

In the present application, the solvent used in reactions may be absent or selected from non-protonic solvents. The non-protonic solvents include toluene, benzene, xylene, acetonitrile, ethyl acetate, ether, methyl *t*-butyl ether, tetrahydrofuran, chloroform, dichloromethane, dimethyl sulfoxide, dimethylformamide or dimethylacetamide, preferably tetrahydrofuran, dichloromethane, dimethyl sulfoxide, and dimethylformamide.

In the present application, the base used in reactions is an inorganic base or an organic base, preferably an organic base (e.g., triethylamine, pyridine, 4-dimethylaminopyridine, imidazole, or diisopropylethylamine); preferably, the base is triethylamine or pyridine.

### 2.2.2. "Protection" and "Deprotection" of Relevant Groups Involved in the Reaction Process

In the present application, reaction processes may involve "protection" and "deprotection" processes of relevant groups. To prevent a certain functional group from affecting the reaction, it is usually protected. In addition, when two or more functional groups are present, only the target functional group is selectively required to react, and the other functional groups should therefore be protected. The protecting group not only protects the functional group stably but also needs to be easily removed as needed. Therefore, in organic synthesis, it is important to remove only the protecting group bonded to the specified functional group under appropriate conditions.

In the present application, the definitions of "carboxyl protecting group" and "amino protecting group" are consistent with those in the "Description of Terms" section, and will not be repeated herein.

In the present application, the hydroxyl groups protected by hydroxyl protecting groups are not particularly limited, e.g., the hydroxyl groups of alcoholic hydroxyl groups, phenolic hydroxyl groups, etc. The amino groups protected by amino protecting groups are not particularly limited, such as those from primary amines, secondary amines, hydrazines, amides, etc. The amino groups in the present application are not particularly limited, including but not limited to primary amino groups, secondary amino groups, tertiary amino groups, and quaternary ammonium ions.

In the present application, the deprotection of protected hydroxyl groups is related to the types of hydroxyl protecting groups. The types of hydroxyl protecting groups are not particularly limited; for example, benzyl groups, silyl ethers, and t-butyl groups can be used to protect terminal hydroxyl groups, and the corresponding deprotection methods include the following:

### A: Deprotection of benzyl protecting groups

The deprotection of benzyl groups can be achieved via hydrogenation using hydrogenation reducing agents and hydrogen donors. The water content in the reaction system should be less than 1% to facilitate the reaction.

The catalyst for hydrogenation reduction is not particularly limited, preferably palladium or nickel. The catalyst support is not particularly limited, preferably aluminum oxide or carbon, and more preferably carbon. The amount of palladium used is 1 to 100 wt% relative to the compounds containing protected hydroxyl groups, preferably 1 to 20 wt%.

The reaction solvent is not particularly limited, as long as both starting materials and products can be dissolved, but is preferably methanol, ethanol, ethyl acetate, tetrahydrofuran, or acetic acid, more preferably methanol. The hydrogen donor is not particularly limited but is preferably gaseous hydrogen, cyclohexene, 2-propanol, or ammonium formate, etc. The reaction temperature is preferably in the range of 25 to 40°C. The reaction time is not particularly limited and is negatively correlated with the amount of catalyst used and preferably in the range of 1 to 5 hours.

### B: Deprotection of silyl ether protecting groups

The protected hydroxyl groups in the forms of silyl ethers include trimethyl silyl ether, triethyl silyl ether, *t*-butyl dimethyl silyl ether, *t*-butyl diphenyl silyl ether, etc. The deprotection of such silyl ethers is carried out using compounds containing fluoride ions; wherein, said compounds are preferably selected from the group consisting of tetrabutylammonium fluoride, tetraethylammonium fluoride, hydrofluoric acid, and potassium fluoride, and more preferably tetrabutylammonium fluoride or potassium fluoride. The molar equivalent of the fluoride-containing compound used is 5 to 20 times that of the protected hydroxyl group, preferably 8 to 15 times that of the initiator. When the molar equivalent of the fluoride-containing compound used is less than 5 times that of the protected hydroxyl group, the deprotection might be incomplete. When the molar equivalent of the deprotection reagent used exceeds 20 times that of the protected hydroxyl group, the excess reagents or compounds will bring difficulty in the purification and possibly cause side reactions in subsequent steps. The reaction solvent is not particularly limited, as long as the reactants and the products can be dissolved, but is preferably an aprotic solvent and more preferably tetrahydrofuran or dichloromethane. The reaction temperature is preferably 0 to 30°C; when the temperature is below 0°C, the reaction rate is relatively slow, and the protecting group cannot be completely removed.

### C: Deprotection of t-butyl protecting groups

The deprotection of *t*-butyl groups is carried out under acidic conditions wherein the pH of the solution is preferably 0 to 4. The acid used is not particularly limited, but is preferably acetic acid, phosphoric acid, sulfuric acid, hydrochloric acid, or nitric acid, and more preferably hydrochloric acid. The reaction solvent is not particularly limited, as long as the reactants and the products can be dissolved, but is preferably water. The reaction temperature is preferably 0 to 30°C.

### 2.2.3. Alkylation reactions

The alkylation reactions of the present application are preferably based on the alkylation of hydroxyl groups, sulfhydryl groups or amino groups, corresponding to the formation of ether bonds, thioether bonds, secondary amino groups or tertiary amino groups, respectively. Examples include the following:

### 2.2.3.1. Alkylation reaction of substrate alcohols with sulfonates or halides

Under basic conditions, an ether intermediate can be obtained via nucleophilic substitution of the substrate alcohol with a sulfonate derivative or halide. Wherein, the molar equivalent of the sulfonate or halide is 1 to 50 times that of the substrate alcohol, preferably 1 to 5 times. When the molar equivalent of the sulfonate or halide is less than 1 times that of the substrate alcohol, the substitution might be incomplete and cause difficulty in the purification process. When the molar equivalent of the sulfonate or halide exceeds 50 times that of the substrate alcohol, the excess sulfonate or halide tends to bring difficulty in the purification process, and might be brought into the subsequent step and therefore cause more side reactions which further increase difficulty in the purification.

The resulting product is a mixture of ether intermediate and excess sulfonate or halide, and can be purified by means such as anion exchange resin, osmosis, ultrafiltration, etc. Wherein, the anion exchange resin is not particularly limited, as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or organic solvents; wherein, the organic solvent is not particularly limited, as long as it can dissolve the product, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50 times that of sulfonates or halides, and preferably 1 to 10 times.

### 2.2.3.2. Alkylation reaction of substrate amines with sulfonates or halides

Under basic condition, an amine intermediate can be obtained via nucleophilic substitution of the substrate amine with a sulfonate derivative or halide. Wherein, the molar equivalent of the sulfonate or halide is 1 to 50 times that of the substrate amine, and preferably 1 to 5 times. When the molar equivalent of sulfonate or halide is less than 1 times that of the substrate amine, the substitution may not be complete, causing difficulty in the purification process. When the molar equivalent of sulfonate or halide exceeds 50 times that of the substrate amine, the excess sulfonate or halide tends to cause difficulty in the purification process, and may be mixed into the subsequent step, resulting in an increase in side reactions in the subsequent step and increasing the difficulty of purification.

The resulting product is a mixture of amine intermediate and excess sulfonate or halide, and can be purified by a purification means such as column chromatography, anion exchange resin, osmosis treatment, ultrafiltration treatment, and the like. Wherein, the anion exchange resin is not particularly limited as long as the target product can undergo ion-exchange and adsorb on the resin, preferably the ion exchange resin of a tertiary amine or quaternary ammonia salt based on dextran, agarose, polyacrylate, polystyrene, poly(diphenylethylene), or the like. The solvents used for osmosis treatment and ultrafiltration treatment are not limited, generally water or an organic solvent. The organic solvent is not particularly limited as long as the product can be dissolved therein, preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base (e.g., triethylamine, pyridine, 4-dimethylaminopyridine, imidazole, and diisopropylethylamine), or an inorganic base (e.g., sodium carbonate, sodium hydroxide, sodium bicarbonate, sodium acetate, potassium carbonate, and potassium hydroxide), preferably an organic base, and more preferably triethylamine or pyridine. The molar amount of base is 1 to 50 times that of the sulfonate or halide, preferably 1 to 10 times, and more preferably 3 to 5 times.

### 2.2.3.3. Alkylation reaction of substrate amines with aldehyde derivatives

The substrate amine reacts with an aldehyde derivative to obtain an imine intermediate, which forms an amine intermediate under the action of reducing agents. Wherein, the molar equivalent of the aldehyde derivative is 1 to 20 times that of the substrate amine, preferably 1 to 2 times, and more preferably 1 to 1.5 times. When the molar equivalent of aldehyde exceeds 20 times that of the substrate amine, the excess reagent tends to cause difficulty in the purification process and may be mixed into the subsequent step to increase the purification difficulty. When the molar equivalent of aldehyde is less than 1 times that of the substrate amine, the substitution may not be complete, causing the purification process to be difficult. Wherein, the resulting product can be obtained after purification by means such as cation exchange resin, osmosis treatment, ultrafiltration treatment, and the like. The cation exchange resin is not particularly limited as long as it can undergo ion-exchange with quaternary ammonium cations and realize the isolation. The solvents used for osmosis treatment and ultrafiltration treatment are not limited, generally water or an organic solvent. The organic solvent is not particularly limited as long as the product can be dissolved therein, preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, etc., and more preferably water or methanol.

The reducing agent is not particularly limited as long as the imine can be reduced to an amine, preferably sodium borohydride, lithium aluminum hydride, sodium cyanoborohydride, Zn/AcOH, etc., and more preferably sodium cyanoborohydride. The molar amount of reducing agent is generally 0.5 to 50 times that of the aldehyde derivatives, and preferably 1 to 10 times.

### 3.1. Lipid Compositions

In the present application, provided herein is a lipid composition comprising any cationic lipid whose structure is represented by the general formula (1-A).

In a specific embodiment of the present application, the lipid composition comprises a cationic lipid whose structure is represented by general formula (1-A), and preferably, further comprises one or more types of lipids selected from the group consisting of phospholipid, steroid lipid and PEGylated lipid; the lipid composition is selected from any one of the following cases:
Case (1): the lipid composition further comprises a phospholipid;
Case (2): the lipid composition further comprises a steroid lipid;
Case (3): the lipid composition further comprises a PEGylated lipid;
Case (4): the lipid composition further comprises a phospholipid and a steroid lipid;
Case (5): the lipid composition further comprises a phospholipid and a PEGylated lipid;
Case (6): the lipid composition further comprises a steroid lipid and a PEGylated lipid;
Case (7): the lipid composition further comprises a phospholipid, a steroid lipid, and a PEGylated lipid;
Case (8): the lipid composition further comprises a phospholipid, a steroid lipid, a PEGylated lipid, and another cationic lipid;
Case (9): the lipid composition further comprises a phospholipid, a steroid lipid, a PEGylated lipid, and an anionic lipid;
more preferably, the lipid composition further comprises three types of lipids, simultaneously, which are a neutral lipid, a steroid lipid, and a PEGylated lipid.

In a specific embodiment of the present application, the phospholipid in the lipid composition is preferably selected from the group consisting of 1,2-dilinoleoyl-sn-glycero-3-phosphocholine (DLPC), 1,2-dimyristoleoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphatidylcholine (DSPC), 1,2-diundecanoyl-sn-glycero-3-phosphatidylcholine (DUPC), 1-plamitoyl-2-oleoyl-sn-glycero-3-phosphocholine (POPC), 1,2-di-O-octadecenyl-sn-glycero-3-phosphatidylcholine (18:0 Diether PC), 1-oleoyl-2-cholesterylhemisuccinyl-sn-glycero-3-phosphocholine (OChemsPC), 1-O-hexadecyl-sn-glycero-3-phosphatidylcholine (C16 Lyso PC), 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoleoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine (LPE), and combinations thereof.

In a specific embodiment of the present application, the steroid lipid in the lipid composition is preferably selected from the group consisting of cholesterol, fecal sterol, sitosterol, ergosterol, campesterol, stigmasterol, rapeseed sterol, lycopene, ursolic acid, α-tocopherol, and combinations thereof.

In a specific embodiment of the present application, the PEGylated lipid in the lipid composition is preferably selected from the group consisting of 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), and PEG-dialkyloxypropyl (PEG-DAA), specifically including PEG500-dipalmitoylphosphatidylcholine, PEG2000-dipalmitoylphosphatidylcholine, PEG500-stearylphosphatidylethanolamine, PEG2000-distearylphosphatidylethanolamine, PEG500-1,2-oleoylphosphatidylethanolamine, PEG2000-1,2-oleoylphosphatidylethanolamine, PEG2000-2,3-dimyristoylglycerol (PEG-DMG), and combinations thereof.

In a specific embodiment of the present application, the PEGylated lipid in the lipid composition is preferably selected from the group consisting of the following structures and combinations thereof: 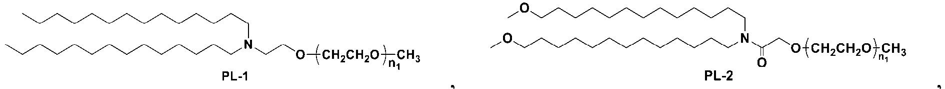 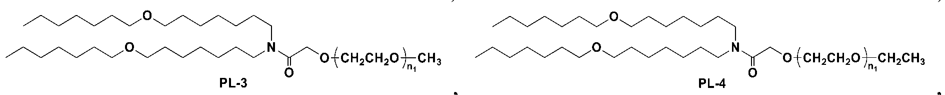 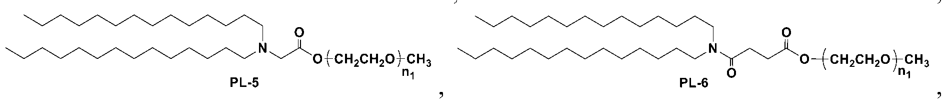 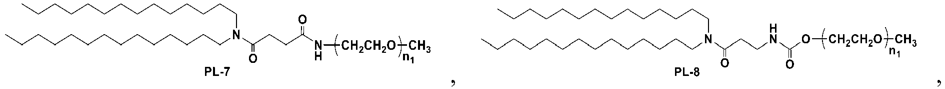 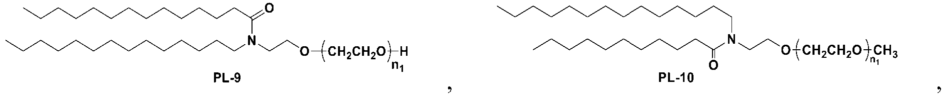 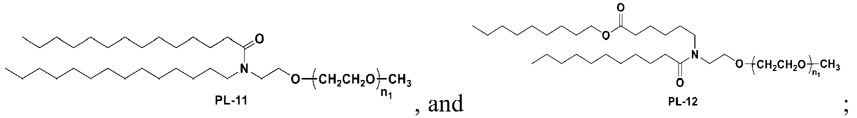 wherein, n₁ is an integer of 25 to 300, and is more preferably selected from the group consisting of 44, 45, 46, 47, and 48.

In a specific embodiment of the present application, the other cationic lipid in the lipid composition is preferably selected from the group consisting of 1,2-dioleoyl-3-trimethylammonium-propane (DOTAP), 1,2-bisoctadecoxy-3-methylammonium propane chloride (DOTMA), 1-[2-(oleoyloxy)ethyl]-2-oleo-3-(2-hydroxyethyl)imidazoline chloride (DOTIM), 1,2-diolecanol-3-dimethylamino-propane (DODMA), 2,3-bis(tetradecanoyloxy)propyltrimonium chloride (DMTAP), didecyldimonium chloride (DDAC), bisdecyldimonium bromide (DDAB), *N,N*-diole-*N,N-*dimethylammonium chloride (DODAC), *N*-(4-carboxybenzyl)-*N,N*-dimethyl-2,3-bis(oleoyloxy)propyl-1-ammonium (DOBAQ), 3,6-bis {4-[bis(2-hydroxydodecyl)amino]butyl}piperazine-2,5-dione (cKK-E12), 1'-((2-(4-(2-(2-(2-(bis(2-hydroxydodecyl)amino)ethyl)(2-hydroxydodecyl)amino)ethyl)pipera zine-1-yl)ethyl)azanediyl)bis(dodecane-2-ol) (C12-200), 4-(*N,N*-dimethylamino)butyric acid(dilinole)methyl ester (DLin-MC3-DMA), 1,2-dimyristoyl-sn-glycero-3-ethylphosphocholine (EPC), ((4-hydroxybutyl) azadiyl)bis(hexane-6,1-diyl)bis(2-hexyldecanoate) (ALC-0315), 8-[(2-hydroxyethyl)(6-oxo-6-(undecoxy)hexyl)amino]caprylic acid (hepta-9-yl) ester (SM-102), and ((2-(2-hydroxyethoxy)ethyl)azadialkyl)bis(hexane-6,1-diyl)bis(2-hexyldecanoate) (DHA-1).

In a specific embodiment of the present application, the lipid composition comprises 20-80% cationic lipid represented by formula (1-A), 5-16% phospholipid, 25-55% steroid lipid, and 0.5-10% PEGylated lipid; the percentages are molar percentages of each lipid with respect to the total lipids in the solvent-comprising solution.

In a specific embodiment of the present application, the anionic lipid in the lipid composition is preferably selected from the group consisting of 1,2-dioleoyl-sn-glycerol-3-phosphate sodium salt (18:1 PA), 1,2-dimyristoyl-sn-glycerol-3-phosphate sodium salt (14:0 PA), bis(monoolein) phosphatidylmonomethylethanolamine (18:1 BMP), and cardiolipin (CL).

In a specific embodiment of the present application, in any of the aforementioned lipid compositions, the molar percentage of cationic lipids in the total lipids in the solvent-comprising solution is preferably 30-65%, more preferably about 35%, 40%, 45%, 46%, 47%, 48%, 49%, 50%, or 55%.

In a specific embodiment of the present application, in any of the aforementioned lipid compositions, the molar percentage of phospholipids in the total lipids in the solvent-comprising solution is preferably 7.5-16%, more preferably about 8%, 9%, 10%, 11%, 12%, or 16%.

In a specific embodiment of the present application, in any of the aforementioned lipid compositions, the molar percentage of steroid lipids in the total lipids in the solvent-comprising solution is preferably 35%-50%, more preferably about 40%, 41%, 42%, 43%, 44%, 45%, 46%, 47%, 48%, 49%, or 50%.

In a specific embodiment of the present application, in any of the aforementioned lipid compositions, the molar percentage of PEGylated lipids in the total lipids in the solvent-comprising solution is preferably 0.5-5%, more preferably 1-3%, more preferably about 1.5%, 1.6%, 1.7%, 1.8%, 1.9%, 2.0%, or 2.5%.

### 3.2. Preparation of Lipid Compositions

In the present application, the lipid composition can be prepared by the following methods, including but not limited to ethanol injection, microfluidic method, T-tube mixing method, and periplasmic extrusion method, preferably ethanol injection and microfluidic method.

### 4. Lipid Pharmaceutical Compositions and Formulations Thereof

### 4.1. Lipid Pharmaceutical Compositions

In one embodiment of the present application, provided herein is a lipid pharmaceutical composition, which comprises any aforementioned lipid composition and a drug; wherein, the lipid composition comprises any aforementioned cationic lipid represented by formula (1-A), and the drug is selected from the group consisting of a nucleic acid drug, a gene vaccine, an antitumor drug, a small molecule drug, a peptide drug, and a protein drug.

In a specific embodiment of the present application, in the lipid pharmaceutical composition, the nucleic acid drug is selected from the group consisting of RNA, DNA, antisense nucleic acid, plasmid, interfering nucleic acid, aptamer, antagomir, and ribozyme; the RNA is selected from the group consisting of mRNA, saRNA, circRNA, miRNA, and siRNA; the nucleic acid drug is preferably selected from the group consisting of DNA, mRNA, miRNA, and siRNA.

In a specific embodiment of the present application, the lipid pharmaceutical composition is preferably used as a drug, selected from any of the following drugs: an antineoplastic agent, an antiviral agent, an antifungal agent, and a vaccine.

In a specific embodiment of the present application, the drug in the lipid pharmaceutical composition includes, but is not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, tioteppa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytosine arabinoside, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitic agents, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, quinizarin, mithramycin, 1-dihydrotestosterone, glucocorticoid, procaine, tetracaine, lidocaine, puromycin, and maytansinoid.

In a specific embodiment of the present application, the N/P ratio of the lipid composition to the nucleic acid is preferably (0.1~100): 1, more preferably (0.2~30): 1, and most preferably (0.5~20): 1.

### 4.2. Formulations of Lipid Pharmaceutical Compositions

In a specific embodiment of the present application, the drug in the lipid pharmaceutical composition is a nucleic acid drug, and the working solution in the formulation of lipid pharmaceutical composition is preferably deionized water, ultrapure water, phosphate buffer or normal saline, more preferably phosphate buffer or normal saline, and most preferably normal saline; the ratio of the lipid composition to the working solution is preferably (0.05~20) g: 100 mL, more preferably (0.1~10) g: 100 mL, and most preferably (0.2~5) g: 100 mL.

In a specific embodiment of the application, provided herein is a formulation of lipid pharmaceutical composition, which comprises the aforementioned lipid pharmaceutical composition and a pharmaceutically acceptable diluent or excipient; the diluent or excipient is preferably deionized water, ultrapure water, phosphate buffer or normal saline, more preferably phosphate buffer or normal saline, and most preferably normal saline.

In the present application, the preparation of the formulation of lipid pharmaceutical composition includes the following steps:
(1) equilibrate the lipid composition in the diluent or excipient;
(2) add the nucleic acid drug to the equilibrated mixture conprising the lipid composition and the diluent or excipient, for complexation;
wherein, the equilibration time is preferably 0.1~12 hours, preferably 0.2~6 hours, and more preferably 0.5~3 hours; the complexation time is preferably 0.1~12 hours, preferably 0.2~5 hours, and more preferably 0.5~2 hours.

### 5. Liposomes, Lipid Nanoparticles (LNPs) and Preparation Thereof

### 5.1. Liposomes or LNPs

In a specific embodiment of the present application, provided herein is a liposome or LNP, which comprises any aforementioned lipid pharmaceutical composition.

In a specific embodiment of the present application, the aforementioned LNP is preferably an LNP-pharmaceutical composition, an LPP-pharmaceutical composition, or a PNP-pharmaceutical composition, preferably an LNP-pharmaceutical composition, more preferably an LNP-nucleic acid pharmaceutical composition, more preferably an LNP-mRNA pharmaceutical composition.

### 5.2. Preparation of Liposomes or LNPs

In a specific embodiment of the present application, liposomes can be prepared by the following methods, including but not limited to thin-film dispersion method, ultrasonic dispersion method, reverse-phase evaporation method, freeze-drying method, freeze-thaw method, double emulsion method, and injection method, preferably thin-film dispersion method, ultrasonic dispersion method and/or reverse-phase evaporation method.

In a specific embodiment of the present application, LNPs can be prepared by the following methods, including but not limited to microemulsion method, double emulsion method, high shear homogenization and ultrasonication, thin-film hydration extrusion method, and microfluidic method.

In a specific embodiment of the present application, liposomes are prepared by the thin-film dispersion method which includes the following steps:
Step (1): weigh cationic lipids, steroid lipids, neutral lipids, and PEGylated lipids, fully dissolve them in an organic solvent, shake well, remove the organic solvent by vacuum rotary evaporation to obtain an oil film, and furtherly dry with a vacuum pump to remove the organic solvent;
Step (2): add phosphate buffer with dissolved cryoprotectant, and use water-bath ultrasonication to obtain a translucent emulsion;
Step (3): add the emulsion to a high-pressure homogenizer for overpressure, and then add the over pressurized homogenized emulsion to the liposome extruder for film-passing to obtain the cationic liposome;
Step (4): optionally, dry the liposome in a freeze dryer to obtain a liposome powder.

In a specific embodiment of the present application, in the preparation methods of liposomes, the ratio of the liposome to the phosphate buffer with dissolved cryoprotectant could be 1 mg : (0.1~100) mL, preferably 1 mg : (0.3~50) mL, and more preferably 1 mg : (0.5~5) mL.

In a specific embodiment of the present application, the LNPs are preferably prepared by the method of microfluidics, vertexing, or simple mixing, and the steps are as follows:
Step (1): dissolve each lipid component in the organic solvent to obtain the lipid composition dissolved in organic phase, wherein the organic phase is preferably ethanol;
Step (2): add the nucleic acid drug to the buffer to obtain the aqueous solution, wherein the aqueous phase is preferably a citrate buffer or sodium acetate buffer;
Step (3): use a microfluidic device, a vortex or a pipette to mix the organic phase solution with the aqueous phase solution to obtain the LNP composition, and perform purification such as ultrafiltration and the like to remove the organic solvent and the free nucleic acid molecules.

The preparation method of the present application includes any protection and deprotection processes of specific groups required for the reaction. The intermediates and final products prepared in this application can be purified by methods including but not limited to extraction, recrystallization, adsorption treatment, precipitation, reverse precipitation, thin-film dialysis, supercritical extraction, and column chromatography (including gel columns, ion columns, silica columns, etc.). The characterization of the structure, molecular weight, and purity of the intermediates and final products can be performed using methods including but not limited to ¹H NMR, electrophoresis, UV-visible spectrophotometry, FTIR, AFM, GPC, HPLC, MALDI-TOF MS, and circular dichroism spectroscopy. In the present application, the structure of cationic lipid final product is characterized by NMR, and its molecular weight is confirmed by mass spectrometry; the molecular weight (defaulting to number-average molecular weight, Mn) and polydispersity index (PDI) of the polymer are determined by gel permeation chromatography (GPC), and the degree of polymerization (defaulting to number-average degree of polymerization) of the polymer is calculated based on its molecular weight. The following specific examples are further descriptions of the preparation methods of cationic lipids, lipid compositions, and formulations of lipid pharmaceutical compositions, and the biological activity assays for lipid pharmaceutical compositions. The specific examples are disclosed to further illustrate the application, but should not be regarded as a limitation of the scope of present application.

### Example 1: Cationic Lipid (E1-1)

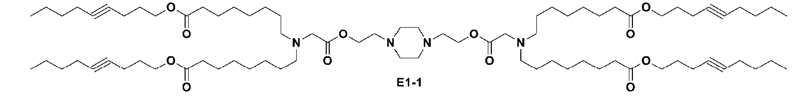

The preparation process is as follows:
Step a: Boc-glycine (**S1-2,** 1.47 g, 8.4 mmol), *p*-toluenesulfonic acid (**TsOH,** 0.76 g, 4.4 mmol), and anhydrous toluene (20 mL) were added sequentially to a round-bottom flask, and then the reaction solution was heated to 135°C and refluxed for 3 hours. After the reaction was completed, the reaction solution was cooled to room temperature, and 1,4-bis(2-hydroxyethyl)piperazine (**S1-1,** 0.70 g, 4.0 mmol) was added in batches, and then the temperature was raised to 135°C to reflux the reaction overnight. After the reaction was completed, the reaction solution was concentrated, and the residue was dissolved in dichloromethane, sequentially washed twice with water, twice with saturated sodium carbonate solution, once with saturated saline, and finally dried with anhydrous magnesium sulfate, filtered, and the filtrate was concentrated. The crude product was purified by column chromatography to obtain an ester with a Boc-protected amino group (1.00 g). To remove the Boc protecting group, a solution of trifluoroacetic acid/dichloromethane (1:2, v/v) was prepared in a dry and clean round-bottom flask. The dichloromethane solution of the above ester (0.97 g, 2.5 mmol) was slowly added under ice bath conditions, and the reaction was conducted at room temperature for 2 hours. After the reaction was completed, water was added to the reaction solution and stirred thoroughly, followed by extraction. The organic phase was dried with anhydrous magnesium sulfate, filtered, and the filtrate was concentrated, and then recrystallized to obtain **S1-3** containing two free amino groups (0.67 g).
Step b: under nitrogen atmosphere, dicyclohexylcarbodiimide (**DCC,** 3.63 g, 17.6 mmol) was added to a round-bottom flask containing 8-bromocaprylic acid (**S1-4,** 1.78 g, 8.0 mmol), 4-nonyn-1-ol (**S1-5,** 1.34 g, 9.6 mmol) and 4-(dimethylamino)pyridine (**DMAP,** 0.24 g, 2.0 mmol) dissolved in dichloromethane (50 mL), and the reaction was conducted for 16 hours at room temperature. After the reaction was completed, the precipitate was removed by filtration, the filtrate was concentrated, and the crude product was purified by column chromatography to obtain 8-bromooctanoic acid 4-nonyn-1-yl ester (**S1-6,** 2.25 g).
Step c: under nitrogen atmosphere, compound S1-3 (0.29 g, 1.0 mmol) was dissolved in acetonitrile (30 mL), and then **S1-6** (2.07 g, 6.0 mmol) and *N,N*-diisopropylethylamine (**DIPEA,** 0.65 g, 5.0 mmol) were added sequentially under slow stirring, and the reaction was stirred for about 20 hours at room temperature. After the reaction was completed, the reaction solution was concentrated, then dissolved with dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain cationic lipid **E1-1** (1.09 g). ¹H NMR (400 MHz, CDCl₃) δ: 4.25 (s, 4H, pip-CH₂C*H*₂-), 4.18-4.11 (m, 8H, -C(=O)OC*H*₂-), 3.35 (s, 4H, -OC(=O)C*H*₂N<), 2.84-2.42 (m, 20H; 8H, pip-*H*; 4H, pip-C*H*₂CH₂-; 8H, >NC*H*₂CH₂-), 2.33-2.20 (m, 16H, -C*H*₂C≡CC*H*₂(CH₂)₂CH₃), 2.18-2.11 (m, 8H, -CH₂C*H*₂C(=O)O-), 1.84-1.76 (m, 8H, -C(=O)OCH₂C*H*₂CH₂-), 1.64-1.23 (m, 56H, -CH₂C*H*₂CH₂-, -C*H*₂CH₃), 0.89 (t, 12H, -CH₂C*H*₃). MS (ESI): m/z=1345.6 ([M+H]⁺).

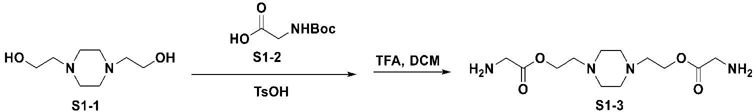

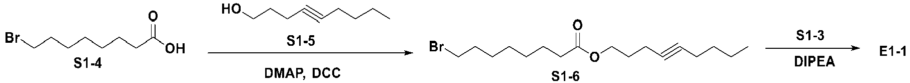

### Example 2: Cationic Lipid (E2-1)

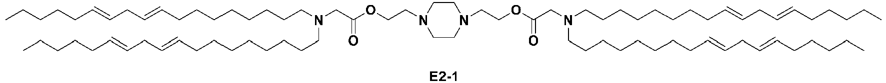

The preparation process is as follows:
**S1-3** (0.58 g, 2.0 mmol) was dissolved in dichloromethane solution (30 mL) under ice bath conditions. Under vigorous stirring, (9Z,12Z)-octadecane-9,12-dienal (**S2-1,** 1.58 g, 6.0 mmol) was added, and then sodium triacetoxyborohydride **(NaBH(OAc)₃**, 1.27 g, 6.0 mmol) was added in three batches within 10 minutes. The reaction system was warmed to room temperature and the reaction was continued for 2 hours. After the reaction was completed, NaOH aqueous solution (1 M, 10 mL) was added. After stirring for 15 minutes, water was added to dilute the reaction solution. The reaction solution was extracted twice with dichloromethane (15 mL*2). The organic phases were combined, washed with saturated saline (15 mL), dried with anhydrous magnesium sulfate, filtered, the filtrate was concentrated, and the crude product was purified by column chromatography to obtain cationic lipid **E2-1** (2.38 g, 92.9%).¹H NMR (400 MHz, CDCl₃) δ: 5.45-5.24 (m, 16H, -C*H*=C*H*-), 4.24 (t, 4H, pip-CH₂C*H*₂-), 3.32 (s, 4H, -OC(=O)C*H*₂N<), 2.82-2.43 (m, 28H; 8H, pip-*H*; 4H, pip-C*H*₂CH₂-; 8H, >NC*H*₂CH₂-; 8H, -CH=CHC*H*₂CH=CH-), 2.03-1.97 (m, 16H, -CH=CHC*H*₂-), 1.64-1.22 (m, 72H, -CH₂C*H*₂CH₂-, -C*H*₂CH₃), 0.89 (t, 12H, -CH₂C*H*₃). MS (ESI): m/z=1281.2 ([M+H]⁺).

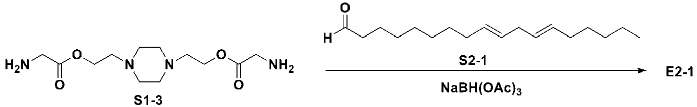

### Example 3: Cationic Lipid (E3-1)

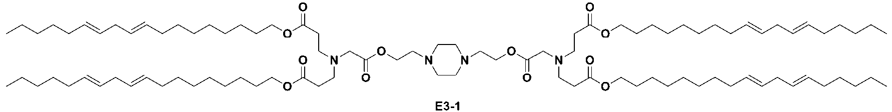

The preparation process is as follows:
Step a: (9Z,12Z)-9,12-octadecadien-1-ol (**S3-1,** 2.39 g, 9.0 mmol) and **triethylamine** (2.59 g, 26.7 mmol) were added sequentially to a dichloromethane solution (50 mL) at 0°C, and then a dichloromethane (10 mL) solution containing acryloyl chloride (**S3-2,** 1.23 g, 13.5 mmol) was added dropwise to the reaction system. The reaction solution was stirred at 20°C for 2 hours. After the reaction was completed, the solid precipitated from the reaction solution was filtered. The filtrate was collected, and washed with water and 5% hydrochloric acid in turn. The organic phase was dried with anhydrous magnesium sulfate, filtered, concentrated, and the crude product was purified by column chromatography to obtain compound (9Z,12Z)-9,12-diene octadecyl acrylate (**S3-3,** 2.17 g).
Step b: **S1-3** (0.27 g, 1.0 mmol) was dissolved in isopropanol, and a sufficient amount of anhydrous potassium carbonate was added under stirring. The reaction solution was stirred at room temperature until the reaction solution became alkaline. **S3-3** (1.93 g, 6.0 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E3-1** (1.20 g). ¹H NMR (400 MHz, CDCl₃) δ: 5.44-5.25 (m, 16H, -C*H*=C*H*-), 4.24 (t, 4H, pip-CH₂C*H*₂-), 4.17-4.08 (m, 8H, -C(=O)OC*H*₂-), 3.35 (s, 4H, -OC(=O)C*H*₂N<), 2.84-2.43 (m, 28H; 8H, pip-*H*; 4H, pip-C*H*₂CH₂-; 8H, >NC*H*₂CH₂-; 8H, -CH=CHC*H₂*CH=CH-), 2.17-2.10 (m, 8H, -CH₂C*H*₂C(=O)O-), 2.03-1.97 (m, 16H, -CH=CHC*H*₂-), 1.84-1.76 (m, 8H, -C(=O)OCH₂C*H*₂CH₂-), 1.65-1.22 (m, 64H, -CH₂C*H*₂CH₂-, -C*H*₂CH₃), 0.89 (t, 12H, -CH₂C*H*₃). MS (ESI): m/z=1569.3 ([M+H]⁺).

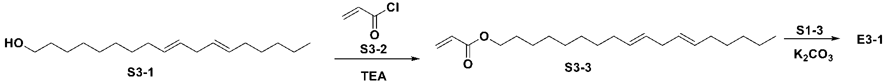

### Example 4: Cationic Lipid (E4-1)

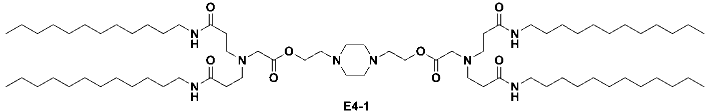

The preparation process is as follows:
**S1-3** (0.35 g, 1.2 mmol) was dissolved in isopropanol, and a sufficient amount of anhydrous potassium carbonate was added under stirring. The reaction solution was stirred at room temperature until the reaction solution became alkaline. N-dodecylacrylamide (**S4-1,** 1.72 g, 7.2 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E4-1** (1.13 g). ¹H NMR (400 MHz, CDCl₃) δ: 4.24 (t, 4H, pip-CH₂C*H*₂-), 3.28 (dd, 8H, -C(=O)NHC*H*₂-), 3.24 (s, 4H, -OC(=O)C*H*₂N<), 2.81-2.62 (m, 20H; 8H, pip-*H*; 4H, pip-C*H*₂CH₂-; 8H, >NC*H*₂CH₂-), 2.39 (t, 8H, -C*H*₂C(=O)NH-), 1.64-1.22 (m, 80H, -CH₂C*H*₂CH₂-, -C*H*₂CH₃), 0.87 (t, 12H, -CH₂C*H*₃). MS (ESI): m/z=1245.1 ([M+H]⁺).

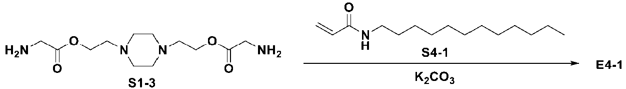

### Example 5: Cationic Lipid (E5-1)

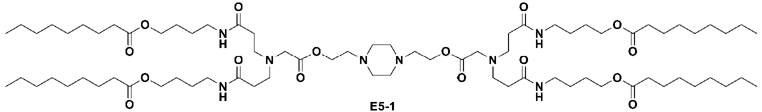

The preparation process is as follows:
Step a: under nitrogen atmosphere, **DCC** (3.63 g, 17.6 mmol) was added to a round-bottom flask containing 4-hydroxybutyl acrylamide (**S5-1,** 1.14 g, 8.0 mmol), nonanoic acid (**S5-2,** 1.52 g, 9.6 mmol), and **DMAP** (0.24 g, 2.0 mmol) dissolved in dichloromethane (50 mL), and the reaction was conducted at room temperature for 16 hours. After the reaction was completed, the precipitate was removed by filtration, the filtrate was concentrated, and the crude product was purified by column chromatography to obtain **S5-3** (1.86 g).
Step b: **S1-3** (0.29 g, 1.0 mmol) was dissolved in isopropanol, and a sufficient amount of anhydrous potassium carbonate was added under stirring. The reaction solution was stirred at room temperature until the reaction solution became alkaline. **S5-3** (1.70 g, 6.0 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E5-1** (1.08 g). ¹H NMR (400 MHz, CDCl₃) δ: 4.24 (t, 4H, pip-CH₂C*H*₂-), 4.16-4.14 (t, 8H, -C(=O)OC*H*₂-), 3.28 (dd, 8H, -C(=O)NHC*H*₂-), 3.25 (s, 4H, -OC(=O)C*H*₂N<), 2.82-2.62 (m, 20H; 8H, pip-*H*; 4H, pip-C*H*₂CH₂-; 8H, >NC*H*₂CH₂-), 2.39 (t, 8H, -C*H*₂C(=O)NH-), 2.18-2.11 (m, 8H, -CH₂C*H*₂C(=O)O-), 1.85-1.76 (m, 8H, -C(=O)OCH₂C*H*₂CH₂-), 1.64-1.23 (m, 56H, -CH₂C*H*₂CH₂-, -C*H*₂CH₃), 0.88 (t, 12H, -CH₂C*H*₃). MS (ESI): m/z=1421.0 ([M+H]⁺).

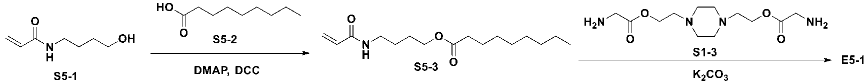

### Example 6: Cationic Lipid (E6-1)

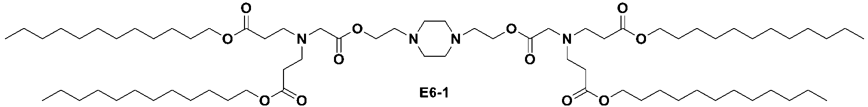

The preparation process is as follows:
**S1-3** (0.35 g, 1.2 mmol) was dissolved in isopropanol, and a sufficient amount of anhydrous potassium carbonate was added under stirring. The reaction solution was stirred at room temperature until the reaction solution became alkaline. 2-dodecyl acrylate (**S6-1,** 1.73 g, 7.2 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E6-1** (1.13 g). ¹H NMR (400 MHz, CDCl₃) δ: 4.24 (t, 4H, pip-CH₂C*H*₂-), 4.16-4.09 (m, 8H, -C(=O)OC*H*₂-), 3.36 (s, 4H, -OC(=O)C*H*₂N<), 2.85-2.43 (m, 20H; 8H, pip-*H*; 4H, pip-C*H*₂CH₂-; 8H, >NC*H*₂CH₂-), 2.18-2.11 (m, 8H, -CH₂C*H*₂C(=O)O-), 1.83-1.76 (m, 8H, -C(=O)OCH₂C*H*₂CH₂-), 1.64-1.22 (m, 72H, -CH₂C*H*₂CH₂-, -C*H*₂CH₃), 0.89 (t, 12H, -CH₂C*H*₃). MS (ESI): m/z=1249.0 ([M+H]⁺).

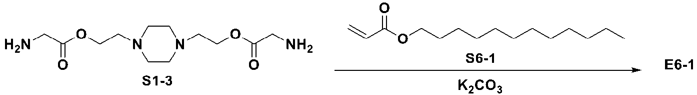

### Example 7.1: Cationic Lipid (E7-1)

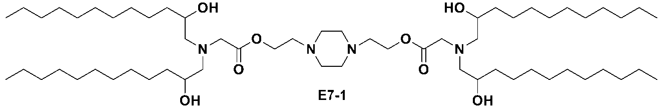

The preparation process is as follows:
**S1-3** (0.35 g, 1.2 mmol) and **DIPEA** (0.46 g, 3.6 mmol) were added sequentially to the methanol solution of 1,2-epoxydodecane (**S7-1,** 1.32 g, 7.2 mmol), and the reaction solution was placed in a reflux device (90°C) and stirred for 24 hours. After the reaction was completed, the reaction solution was concentrated, and the crude product was purified by column chromatography to obtain the cationic lipid **E7-1** (0.83 g). ¹H NMR (400 MHz, CDCl₃) δ: 4.23 (t, 4H, pip-CH₂C*H*₂-), 3.51-3.41 (m, 4H, -CH(OH)-), 3.32 (s, 4H, -OC(=O)C*H*₂N<), 2.78-2.31 (m, 20H; 4H, pip-C*H*₂CH₂-; 8H, pip-*H*; 8H, >CHC*H*₂N<), 1.44-1.23 (m, 72H, -CH₂C*H*₂CH₂-, -C*H*₂CH₃), 0.88 (t, 12H, -CH₂C*H*₃). MS (ESI): m/z=1024.9 ([M+H]⁺).

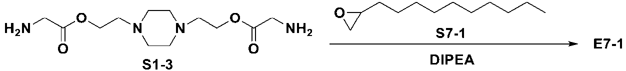

### Example 7.2: Cationic Lipid (E7-2)

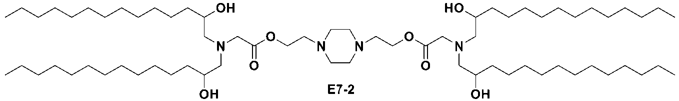

Following the same reaction steps in Example **7.1,** the starting material **S7-1** was replaced with 1,2-epoxytetradecane (**S7-2,** 1.53 g, 7.2 mmol) to obtain cationic lipid **E7-2** (0.93 g).¹H NMR (400 MHz, CDCl₃) δ: 4.22 (t, 4H, pip-CH₂C*H*₂-), 3.52-3.41 (m, 4H, -CH(OH)-), 3.33 (s, 4H, -OC(=O)C*H*₂N<), 2.78-2.30 (m, 20H; 4H, pip-C*H*₂CH₂-; 8H, pip-*H*; 8H, >CHC*H*₂N<), 1.45-1.22 (m, 88H, -CH₂C*H*₂CH₂-, -C*H*₂CH₃), 0.88 (t, 12H, -CH₂C*H*₃). MS (ESI): m/z=1137.0 ([M+H]⁺).

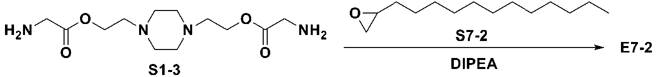

### Example 7.3: Cationic Lipid (E7-3)

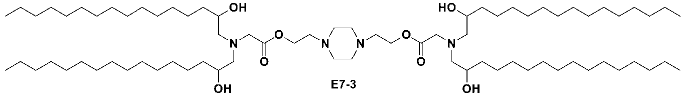

Following the same reaction steps in Example **7.1,** the starting material **S7-1** was replaced with 1,2-epoxyhexadecane (**S7-3,** 1.73 g, 7.2 mmol) to obtain cationic lipid **E7-3** (1.02 g).¹H NMR (400 MHz, CDCl₃) δ: 4.24 (t, 4H, pip-CH₂C*H*₂-), 3.51-3.42 (m, 4H, -CH(OH)-), 3.35 (s, 4H, -OC(=O)C*H*₂N<), 2.78-2.31 (m, 20H; 4H, pip-C*H*₂CH₂-; 8H, pip-*H*; 8H, >CHC*H*₂N<), 1.42-1.22 (m, 104H, -CH₂C*H*₂CH₂-, -C*H*₂CH₃), 0.87 (t, 12H, -CH₂C*H*₃). MS (ESI): m/z=1249.2 ([M+H]⁺).

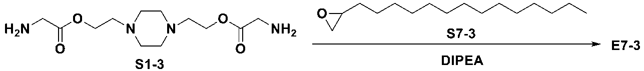

### Example 8: Cationic Lipid (E8-1)

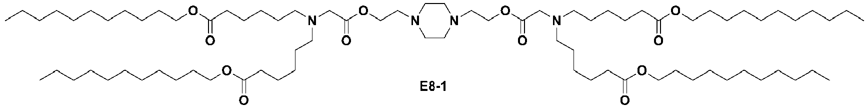

The preparation process is as follows:
Step a: under nitrogen atmosphere, **DCC** (3.63 g, 17.6 mmol) was added to a round-bottom flask containing undecanol (**S8-2,** 1.65 g, 9.6 mmol), 8-bromooctanoic acid (**S8-1,** 1.56 g, 8.0 mmol), and **DMAP** (0.24 g, 2.0 mmol) dissolved in dichloromethane (30 mL), and then reacted at room temperature for 16 hours. After the reaction was completed, the precipitate was removed by filtration, the filtrate was concentrated, and the crude product was purified by column chromatography to obtain 6-bromohexanoic undecyl ester (S**8-3,** 2.30 g).
Step b: under nitrogen atmosphere, compound **S1-3** (0.29 g, 1.0 mmol) was dissolved in acetonitrile (30 mL), **S8-3** (2.09 g, 6.0 mmol) and **DIPEA** (0.65 g, 5.0 mmol) were added successively under slow stirring, and the reaction was stirred for about 20 hours at room temperature. After the reaction was completed, the reaction solution was concentrated, then dissolved in dichloromethane, and extracted successively with 0.6 M hydrochloric acid/10% sodium chloride solution and saturated sodium bicarbonate solution. The organic phases were combined, dried with anhydrous magnesium sulfate, filtered, and the filtrate was concentrated, and then the crude product was purified by column chromatography to obtain cationic lipid **E8-1** (1.11 g). ¹H NMR (400 MHz, CDCl₃) δ: 4.24 (t, 4H, pip-CH₂C*H*₂-), 4.16-4.09 (m, 8H, -C(=O)OC*H*₂-), 3.36 (s, 4H, -OC(=O)C*H*₂N<), 2.84-2.43 (m, 20H; 8H, pip-*H*; 4H, pip-C*H*₂CH₂-; 8H, >NC*H*₂CH₂-), 2.18-2.10 (m, 8H, -CH₂C*H*₂C(=O)O-), 1.84-1.76 (m, 8H, -C(=O)OCH₂C*H*₂CH₂-), 1.64-1.22 (m, 88H, -CH₂C*H*₂CH₂-, -C*H*₂CH₃), 0.89 (t, 12H, -CH₂C*H*₃). MS (ESI): m/z=1361.1 ([M+H]⁺).

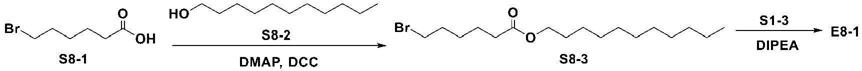

### Example 9: Cationic Lipid (E9-1)

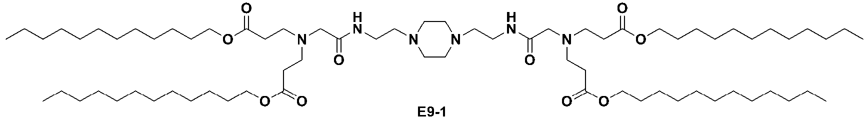

The preparation process is as follows:
Step a: 1-(3-dimethylaminopropyl)-3-ethylcarbodiimide hydrochloride **(EDCI,** 2.87 g, 15.0 mmol) and 1-hydroxybenzotriazole (**HOBt**, 2.03 g, 15.0 mmol) were added sequentially to a round-bottom flask containing **S1-2** (2.63 g, 15.0 mmol) and **DIPEA** (1.94 g, 15.0 mmol) dissolved in dichloromethane, and reacted for 10 minutes at room temperature, followed by adding 1,4-piperazine diethylamine (**S9-1,** 0.88 g, 5.0 mmol) in batches. The reaction solution was stirred at room temperature for 12 hours. After the reaction was completed, saturated NaHCO₃ (10 mL) was added to quench the reaction. The aqueous phase was extracted three times (20 mL*3) with dichloromethane. The organic phase was dried with anhydrous magnesium sulfate, filtered, the filtrate was concentrated, and then the crude product was purified by column chromatography to obtain **S9-2** (1.41 g).
Step b: Removal of the Boc protecting group. In a dry and clean round-bottom flask, a solution of trifluoroacetic acid/dichloromethane (1:2, v/v) was prepared. Under ice bath conditions, the dichloromethane solution **of S9-2** (0.97 g, 2.0 mmol) was slowly added, and the reaction was carried out at room temperature for 2 hours. After the reaction was completed, water was added to the reaction solution and stirred well, followed by extraction. The organic phase was dried with anhydrous magnesium sulfate, filtered, the filtrate was concentrated, and the crude product was recrystallized to obtain **S9-3** containing free amino groups (0.53 g, 92.5%).
Step c: **S9-3** (0.29 g, 1.0 mmol) was dissolved in isopropanol, and a sufficient amount of anhydrous potassium carbonate was added under stirring. The reaction solution was stirred at room temperature until the reaction solution became alkaline. **S6-1** (1.44 g, 6.0 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E9-1** (0.95 g). ¹H NMR (400 MHz, CDCl₃) δ: 4.15-4.08 (m, 8H, -C(=O)OC*H*₂-), 3.46-3.33 (m, 4H, pip-CH₂C*H*₂-), 3.03 (s, 4H, -NHC(=O)C*H*₂-), 2.84-2.43 (m, 20H; 8H, pip-*H*; 4H, pip-C*H*₂CH₂-; 8H, >NC*H*₂CH₂-), 2.18-2.10 (m, 8H, -CH₂C*H*₂C(=O)O-), 1.84-1.76 (m, 8H, -C(=O)OCH₂C*H*₂-), 1.62-1.22 (m, 72H, -CH₂C*H*₂CH₂-, -C*H*₂CH₃), 0.88 (t, 12H, -CH₂C*H*₃). MS (ESI): m/z=1247.1 ([M+H]⁺).

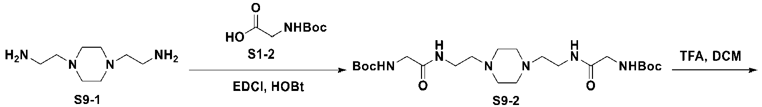

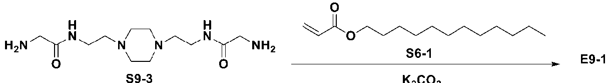

### Example 10: Cationic Lipid (E10-1)

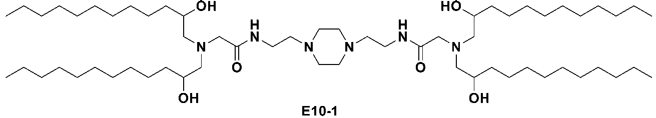

The preparation process is as follows:
**S9-3** (0.34 g, 1.2 mmol) and **DIPEA** (0.46 g, 3.6 mmol) were added sequentially to a methanol solution of **S7-1** (1.32 g, 7.2 mmol), and then the reaction solution was placed in a reflux apparatus (90°C) and stirred for 24 hours. After the reaction was completed, the reaction mixture was concentrated, and the crude product was purified by column chromatography to obtain cationic lipid **E10-1** (0.82 g). ¹H NMR (400 MHz, CDCl₃) δ: 3.51-3.33 (m, 8H; 4H, -CH(OH)-; 4H, pip-CH₂C*H*₂-), 3.04 (s, 4H, -NHC(=O)C*H*₂-), 2.78-2.31 (m, 20H; 4H, pip-C*H*₂CH₂-; 8H, pip-*H*; 8H, >CHC*H*₂N<), 1.45-1.22 (m, 72H, -CH₂C*H*₂CH₂-, -C*H*₂CH₃), 0.88 (t, 12H, -CH₂C*H*₃). MS (ESI): m/z=1022.9 ([M+H]⁺).

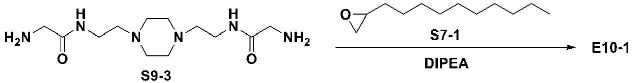

### Example 11: Cationic Lipid (E11-1)

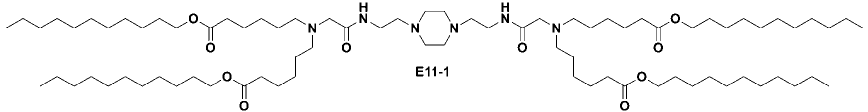

The preparation process is as follows:
Under nitrogen atmosphere, compound S9-3 (0.29 g, 1.0 mmol) was dissolved in acetonitrile (30 mL), **S8-3** (2.09 g, 6.0 mmol) and **DIPEA** (0.65 g, 5.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E11-1** (1.12 g). ¹H NMR (400 MHz, CDCl₃) δ: 4.18-4.10 (m, 8H, -C(=O)OC*H*₂-), 3.46-3.33 (m, 4H, pip-CH₂C*H*₂-), 3.04 (s, 4H, -NHC(=O)C*H*₂-), 2.78-2.31 (m, 20H; 4H, pip-C*H*₂CH₂-; 8H, pip-*H*; 8H, >NC*H*₂CH₂-), 2.18-2.10 (m, 8H, -CH₂C*H*₂C(=O)O-), 1.84-1.76 (m, 8H, -C(=O)OCH₂C*H*₂-), 1.64-1.22 (m, 88H, -CH₂C*H*₂CH₂-, -C*H*₂CH₃), 0.89 (t, 12H, -CH₂C*H*₃). MS (ESI): m/z=1359.2 ([M+H]⁺).

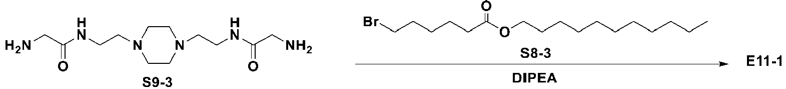

### Example 12: Cationic Lipid (E12-1)

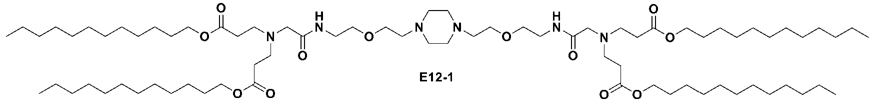

The preparation process is as follows:
Step a: *N*-Boc-2-(2-bromoethoxy)ethylamine (**S12-2,** 5.36 g, 20.0 mmol) and piperazine (**S12-1,** 0.69 g, 8.0 mmol) were dissolved in acetonitrile (100 mL), **potassium carbonate** (7.73 g, 56.0 mmol) was added to the above system, and then the reaction was heated to 60°C and stirred for 3 hours. After the reaction was completed, the reaction solution was filtered after being cooled to room temperature. The filtrate was concentrated, and the residue was purified by column chromatography to obtain **S12-3** (2.09 g).
Step b: Removal of the Boc protecting group. In a dry and clean round-bottom flask, a solution of trifluoroacetic acid/dichloromethane (1:2, v/v) was prepared. Under ice bath conditions, the dichloromethane solution of **S12-3** (1.84 g, 4.0 mmol) was slowly added dropwise, and the reaction was carried out for 2 hours at room temperature. After the reaction was completed, water was added to the reaction solution and stirred well, followed by extraction. The organic phase was dried with anhydrous magnesium sulfate, filtered, the filtrate was concentrated, and recrystallized to obtain **S12-4** containing free amino groups (0.96 g, 92.2%).
Step c: **EDCI** (1.72 g, 9.0 mmol) and **HOBt** (1.22 g, 9.0 mmol) were added to a round-bottom flask containing **S1-2** (1.57 g, 9.0 mmol) and **DIPEA** (1.61 g, 9.0 mmol) dissolved in dichloromethane, and reacted for 10 minutes at room temperature, followed by adding **S12-4** (0.78 g, 3.0 mmol) in batches. The reaction solution was stirred at room temperature for 12 hours. After the reaction was completed, saturated NaHCO₃ (10 mL) was added to quench the reaction. The aqueous phase was extracted three times (20 mL*3) with dichloromethane. The organic phase was dried with anhydrous magnesium sulfate, filtered, the filtrate was concentrated, and then the crude product was purified by column chromatography to obtain **S12-5** (0.98 g).
Step d: Removal of the Boc protecting group. In a dry and clean round-bottom flask, a solution of trifluoroacetic acid/dichloromethane (1:2, v/v) was prepared. Under ice bath conditions, the dichloromethane solution of **S12-5** (0.69 g, 1.2 mmol) was slowly added dropwise, and the reaction was carried out for 2 hours at room temperature. After the reaction was completed, water was added to the reaction solution and stirred well, followed by extraction. The organic phase was dried with anhydrous magnesium sulfate, filtered, the filtrate was concentrated, and recrystallized to obtain **S12-6** containing free amino groups (0.42 g, 93.1%).
Step e: **S12-6** (0.30 g, 0.8 mmol) was dissolved in isopropanol, and a sufficient amount of anhydrous potassium carbonate was added under stirring. The reaction solution was stirred at room temperature until the reaction solution became alkaline. **S6-1** (1.15 g, 4.8 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E12-1** (0.82 g). ¹H NMR (400 MHz, CDCl₃) δ: 4.17-4.11 (m, 8H, -C(=O)OC*H*₂-), 3.58 (t, 4H, -C(=O)NHCH₂C*H*₂O-), 3.51 (t, 4H, pip-CH₂C*H*₂-), 3.48-3.41 (m, 4H, -C(=O)NHCH₂C*H*₂O-), 3.03 (s, 4H, -NHC(=O)C*H*₂-), 2.76-2.47 (m, 12H; 8H, pip-*H*; 4H, pip-CH₂C*H*₂-), 2.45-2.43 (m, 8H, >NC*H*₂CH₂-), 2.29 (t, 8H, -C*H*₂C(=O)O-), 1.84-1.76 (m, 8H, -C(=O)OCH₂C*H*₂-), 1.68-1.23 (m, 72H, -CH₂C*H*₂CH₂-, -C*H*₂CH₃), 0.87 (t, 12H, -CH₂C*H*₃). MS (ESI): m/z=1335.1 ([M+H]⁺).

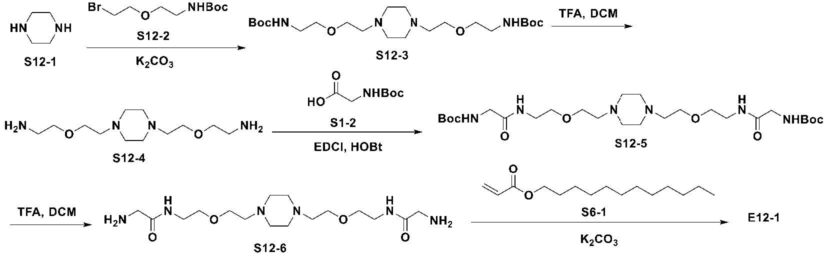

### Example 13: Cationic Lipid (E13-1)

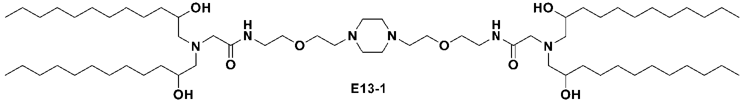

The preparation process is as follows:
**S12-6** (0.45 g, 1.2 mmol) and **DIPEA** (0.46 g, 3.6 mmol) were sequentially added to a methanol solution of **S7-1** (1.32 g, 7.2 mmol), and then the reaction solution was placed in a reflux apparatus (90°C) and stirred for 36 hours. After the reaction was completed, the reaction solution was concentrated, and the crude product was purified by column chromatography to obtain cationic lipid **E13-1** (0.91 g). ¹H NMR (400 MHz, CDCl₃) δ: 3.70-3.18 (m, 20H; 8H, -C*H*₂OC*H*₂-; 4H, -CH(OH)-; 4H, -C*H*₂NHC(=O)-; 4H, -NHC(=O)C*H*₂-), 2.77-2.31 (m, 20H; 4H, pip-C*H*₂CH₂-; 8H, pip-*H*; 8H, >CHC*H*₂N<), 1.44-1.23 (m, 72H, -CH₂C*H*₂CH₂-, -C*H*₂CH₃), 0.88 (t, 12H, -CH₂C*H*₃). MS (ESI): m/z=1112.0 ([M+H]⁺).

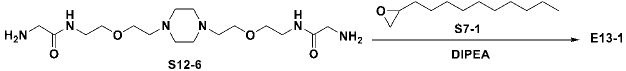

### Example 14: Cationic Lipid (E14-1)

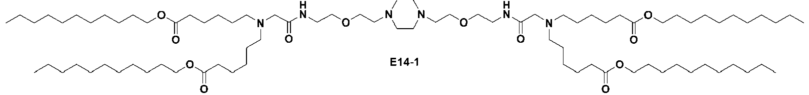

The preparation process is as follows:
Under nitrogen atmosphere, compound **S12-6** (0.37 g, 1.0 mmol) was dissolved in acetonitrile (30 mL), **S8-3** (2.09 g, 6.0 mmol) and **DIPEA** (0.65 g, 5.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E14-1** (1.18 g). ¹H NMR (400 MHz, CDCl₃) δ: 4.05 (t, 8H, -C(=O)OC*H*₂-), 3.59 (t, 4H, -C(=O)NHCH₂C*H*₂O-), 3.52 (t, 4H, pip-CH₂C*H*₂-), 3.49-3.41 (m, 4H, -C(=O)NHCH₂C*H*₂O-), 3.03 (s, 4H, -NHC(=O)C*H*₂-), 2.76-2.46 (m, 12H; 8H, pip-*H*; 4H, pip-CH₂C*H*₂-), 2.45-2.43 (m, 8H, >NC*H*₂CH₂-), 2.29 (t, 8H, -C*H*₂C(=O)O-), 1.66-1.23 (m, 96H, -CH₂C*H*₂CH₂-, -C*H*₂CH₃), 0.88 (t, 12H, -CH₂C*H*₃). MS (ESI): m/z=1448.1 ([M+H]⁺).

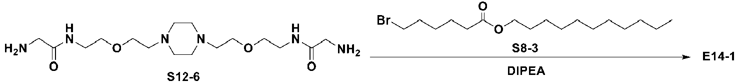

### Example 15: Cationic Lipid (E15-1)

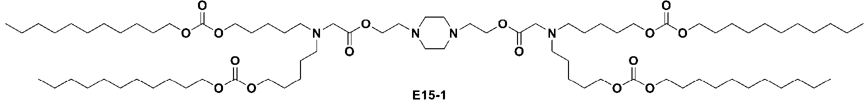

The preparation process is as follows:
Step a: 5-Bromopentanol **(S15-1,** 0.84 g, 5.0 mmol) was dissolved in dichloromethane (40 mL), then **DMAP** (1.22 g, 10 mmol) was added, followed by adding ***p*-Nitrophenyl chloroformate** (1.11 g, 5.5 mmol) in batches. The reaction was stirred at room temperature for 3 hours, and then **S8-2** (0.97 g, 5.6 mmol) was added to the reaction solution. The reaction was further stirred at room temperature overnight. After the reaction was completed, the reaction solution was diluted with dichloromethane (20 mL), washed with saturated brine (30 mL), and the organic phase was dried with anhydrous magnesium sulfate, filtered, the filtrate was concentrated, then the crude product was purified by column chromatography to obtain 5-bromopentyl undecyl carbonate (**S15-2,** 2.11 g).
Step b: under nitrogen atmosphere, compound **S1-3** (0.23 g, 0.8 mmol) was dissolved in acetonitrile (30 mL), **S15-2** (1.75 g, 4.8 mmol) and **DIPEA** (0.52 g, 4.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E15-1** (0.94 g). ¹H NMR (400 MHz, CDCl₃) δ: 4.24 (t, 4H, pip-CH₂C*H*₂-), 4.15-4.10 (m, 16H, -C*H*₂OC(=O)OC*H*₂-), 3.36 (s, 4H, -OC(=O)C*H*₂N<), 2.85-2.43 (m, 20H; 8H, pip-*H*; 4H, pip-C*H*₂CH₂-; 8H, >NC*H*₂CH₂-), 1.62-1.22 (m, 96H, -CH₂C*H*₂CH₂-, -C*H*₂CH₃), 0.88 (t, 12H, -CH₂C*H*₃). MS (ESI): m/z=1425.1 ([M+H]⁺).

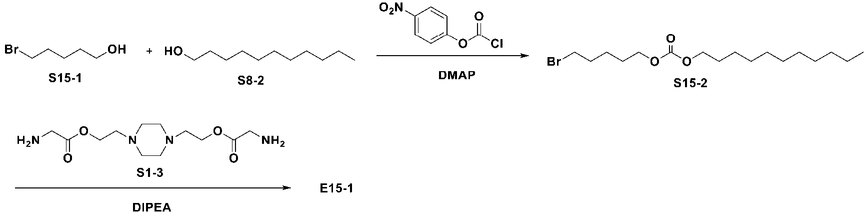

### Example 16: Cationic Lipid (E16-1)

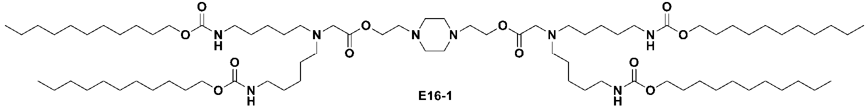

The preparation process is as follows:
Step a: **S8-2** (2.06 g, 12.0 mmol) was dissolved in dichloromethane (40 mL), triethylamine (5 mL) and *N*,*N'*-carbonyldiimidazole **(CDI,** 1.94 g, 12.0 mmol) were added sequentially, followed by reaction at 50°C for 1.5 hours. Then, 4-amino-1-butanol **(S16-1,** 1.60 g, 18.0 mmol) was added and the reaction was continued at 50°C for 16 hours. After the reaction was completed, the reaction solution was cooled to room temperature, and washed sequentially with 5% citric acid (20 mL * 2) and saturated brine (10 mL). The organic phase was dried with anhydrous magnesium sulfate, filtered, and concentrated to obtain the crude product. Dichloromethane (20 mL) was added to the crude product, stirred for 10 minutes, and filtered. The residue was washed with a small amount of dichloromethane, and concentrated to obtain compound **S16-2** (2.26 g).
Step b: **S16-2** (1.72 g, 6.0 mmol) was dissolved in dichloromethane (80 mL), and triphenylphosphine (**PPh₃**, 2.36 g, 9.0 mmol) was added. Carbon tetrabromide (**CBr₄**, 2.98 g, 9.0 mmol) was added in portions under the ice bath conditions, and the reaction was continued for 20 minutes. After the reaction was completed, 10 mL of methanol was added to quench the reaction, and the solution was directly concentrated to obtain the crude product, which was purified by column chromatography to yield the product **S16-3** (1.23 g).
Step c: under nitrogen atmosphere, compound **S1-3** (0.14 g, 0.5 mmol) was dissolved in acetonitrile (20 mL), **S16-3** (1.05 g, 3.0 mmol) and **DIPEA** (0.32 g, 2.5 mmol) were added sequentially under slow stirring, and the reaction was stirred for about 20 hours at room temperature. After the reaction was completed, the reaction solution was concentrated, then dissolved in dichloromethane, and successively extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E16-1** (0.58 g). ¹H NMR (400 MHz, CDCl₃) δ: 4.25 (t, 4H, pip-CH₂C*H*₂-), 4.11 (t, 8H, -NHC(=O)OC*H*₂-), 3.35 (s, 4H, -OC(=O)C*H*₂N<), 3.15 (t, 8H, -C*H*₂NHC(=O)O-), 2.84-2.44 (m, 20H; 8H, pip-*H*; 4H, pip-C*H*₂CH₂-; 8H, >NC*H*₂CH₂-), 1.65-1.22 (m, 96H, -CH₂C*H*₂CH₂-, -C*H*₂CH₃), 0.88 (t, 12H, -CH₂C*H*₃). MS (ESI): m/z=1421.2 ([M+H]⁺).

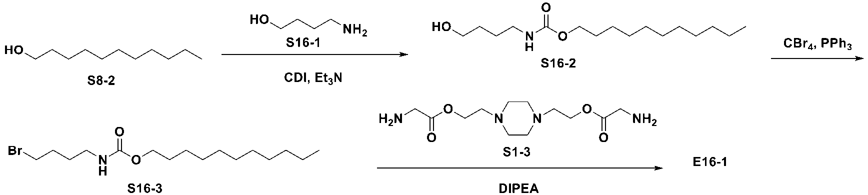

### Example 17: Cationic Lipid (E17-1)

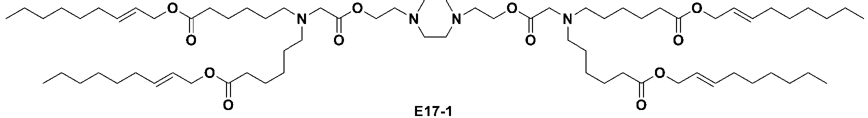

The preparation process is as follows:
Step a: under nitrogen atmosphere, **DCC** (3.63 g, 17.6 mmol) was added to a round-bottom flask containing 2-decen-1-ol **(S17-1,** 1.36 g, 9.6 mmol), **S8-1** (1.56 g, 8.0 mmol), and **DMAP** (0.24 g, 2.0 mmol) dissolved in dichloromethane (20 mL), and then reacted at room temperature for 16 hours. After the reaction was completed, the precipitate was removed by filtration, the filtrate was concentrated, and the crude product was purified by column chromatography to obtain **S17-2** (2.11 g).
Step b: under nitrogen atmosphere, compound **S1-3** (0.29 g, 1.0 mmol) was dissolved in acetonitrile (30 mL), **S17-2** (1.91 g, 6.0 mmol) and **DIPEA** (0.65 g, 5.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E17-1** (1.01 g). ¹H NMR (400 MHz, CDCl₃) δ: 5.82-5.71 (m, 4H, -C(=O)OCH₂C*H*=CH-), 5.56-5.54 (m, 4H, -C(=O)OCH₂CH=C*H*-), 4.24 (t, 4H, pip-CH₂C*H*₂-), 4.55 (t, 8H, -C(=O)OC*H*₂CH=CH-), 3.36 (s, 4H, -OC(=O)C*H*₂N<), 2.85-2.43 (m, 20H; 8H, pip-*H*; 4H, pip-C*H*₂CH₂-; 8H, >NC*H*₂CH₂-), 2.18-2.10 (m, 8H, -CH₂C*H*₂C(=O)O-), 2.06-1.99 (m, 8H, -CH=CHC*H*₂CH₂-), 1.64-1.22 (m, 56H, -CH₂C*H*₂CH₂-, -C*H*₂CH₃), 0.88 (t, 12H, -CH₂C*H*₃). MS (ESI): m/z=1241.0 ([M+H]⁺).

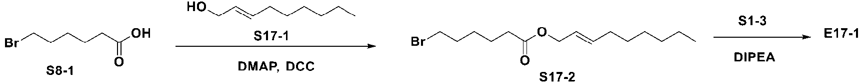

### Example 18: Cationic Lipid (E18-1)

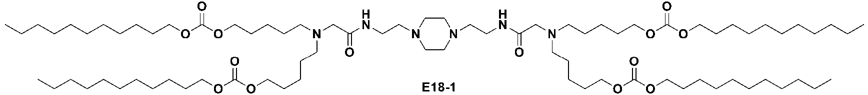

Following the same procedure in the Step b of Example **15,** the starting material **S1-3** was replaced with the starting material **S9-3** (0.29 g, 1.0 mmol) to obtain cationic lipid **E18-1** (1.18 g). ¹H NMR (400 MHz, CDCl₃) δ: 4.15-4.10 (m, 16H, -C*H*₂OC(=O)OC*H*₂-), 3.46-3.33 (m, 4H, pip-CH₂C*H*₂-), 3.04 (s, 4H, -NHC(=O)C*H*₂-), 2.78-2.32 (m, 20H; 4H, pip-C*H*₂CH₂-; 8H, pip-*H*; 8H, >NC*H*₂CH₂-), 1.60-1.22 (m, 96H, -CH₂C*H*₂CH₂-, -C*H*₂CH₃), 0.89 (t, 12H, -CH₂C*H*₃). MS (ESI): m/z=1423.2 ([M+H]⁺).

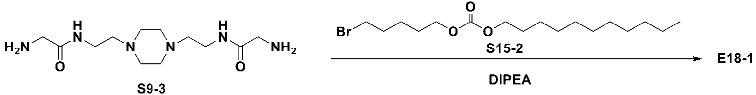

### Example 19: Cationic Lipid (E19-1)

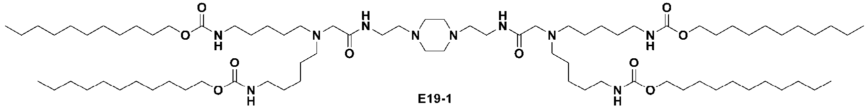

Following the same procedure in the Step c of Example **16,** the starting material **S1-3** was replaced with the starting material **S9-3** (0.29 g, 1.0 mmol) to obtain cationic lipid **E19-1** (1.17 g). ¹H NMR (400 MHz, CDCl₃) δ: 4.11 (t, 8H, -NHC(=O)OC*H*₂-), 3.46-3.33 (m, 4H, pip-CH₂C*H*₂-), 3.28 (s, 4H, -NHC(=O)C*H*₂-), 3.15 (t, 8H, -C*H*₂NHC(=O)O-), 2.84-2.42 (m, 20H; 8H, pip-H; 4H, pip-C*H*₂CH₂-; 8H, >NC*H*₂CH₂-), 1.62-1.22 (m, 96H, -CH₂C*H*₂CH₂-, -C*H*₂CH₃), 0.89 (t, 12H, -CH₂C*H*₃). MS (ESI): m/z=1419.2 ([M+H]⁺).

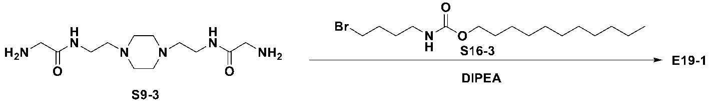

### Example 20: Cationic Lipid (E20-1)

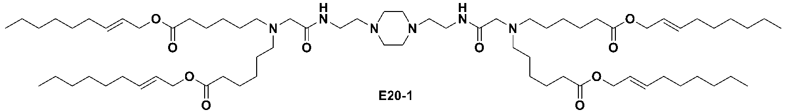

Following the same procedure in the Step b of Example **17,** the starting material **S1-3** was replaced with the starting material **S9-3** (0.37 g, 1.0 mmol) to obtain cationic lipid **E20-1** (1.03 g). ¹H NMR (400 MHz, CDCl₃) δ: 5.82-5.70 (m, 4H, -C(=O)OCH₂C*H*=CH-), 5.56-5.53 (m, 4H, -C(=O)OCH₂CH=C*H*-), 4.55 (t, 8H, -C(=O)OC*H*₂CH=CH-), 3.46-3.33 (m, 4H, pip-CH₂C*H*₂-), 3.27 (s, 4H, -NHC(=O)C*H*₂-), 2.85-2.43 (m, 20H; 8H, pip-H; 4H, pip-C*H*₂CH₂-; 8H, >NC*H*₂CH₂-), 2.18-2.10 (m, 8H, -CH₂C*H*₂C(=O)O-), 2.06-1.99 (m, 8H, -CH=CHC*H*₂CH₂-), 1.64-1.22 (m, 56H, -CH₂C*H*₂CH₂-, -C*H*₂CH₃), 0.89 (t, 12H, -CH₂C*H*₃). MS (ESI): m/z=1239.0 ([M+H]⁺).

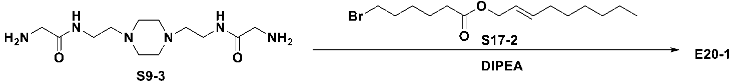

### Example 21: Cationic Lipid (E21-1)

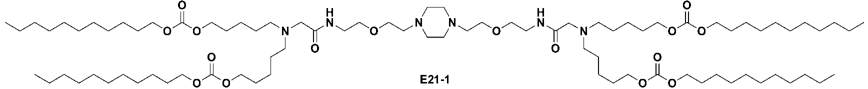

Following the same procedure in the Step b of Example **15,** the starting material **S1-3** was replaced with the starting material **S12-6** (0.37 g, 1.0 mmol) to obtain cationic lipid **E21-1** (1.26 g). ¹H NMR (400 MHz, CDCl₃) δ: 4.15-4.10 (m, 16H, -C*H*₂OC(=O)OC*H*₂-), 3.59 (t, 4H, -C(=O)NHCH₂C*H*₂O-), 3.52 (t, 4H, pip-CH₂C*H*₂-), 3.49-3.41 (m, 4H, -C(=O)NHC*H*₂CH₂O-), 3.03 (s, 4H, -NHC(=O)C*H*₂-), 2.76-2.47 (m, 12H; 8H, pip-*H*; 4H, pip-CH₂C*H*₂-), 2.46-2.43 (m, 8H, >NC*H*₂CH₂-), 1.62-1.22 (m, 96H, -CH₂C*H*₂CH₂-, -C*H*₂CH₃), 0.88 (t, 12H, -CH₂C*H*₃). MS (ESI): m/z=1511.2 ([M+H]⁺).

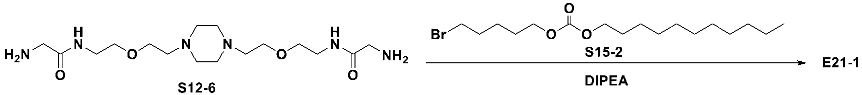

### Example 22: Cationic Lipid (E22-1)

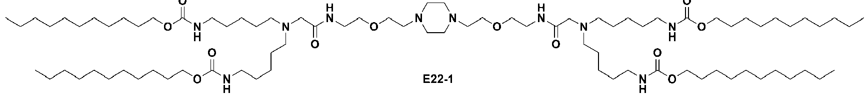

Following the same procedure in the Step c of Example **16,** the starting material **S1-3** was replaced with the starting material **S12-6** (1.37 g, 1.0 mmol) to obtain cationic lipid **E22-1** (1.24 g). ¹H NMR (400 MHz, CDCl₃) δ: 4.10 (t, 8H, -NHC(=O)OC*H*₂-), 3.59 (t, 4H, -C(=O)NHCH₂C*H*₂O-), 3.52 (t, 4H, pip-CH₂C*H*₂-), 3.49-3.41 (m, 4H, -C(=O)NHCH₂C*H*₂O-), 3.15 (t, 8H, -C*H*₂NHC(=O)O-), 3.03 (s, 4H, -NHC(=O)C*H*₂-), 2.77-2.47 (m, 12H; 8H, pip-H; 4H, pip-CH₂C*H*₂-), 2.46-2.43 (m, 8H, >NC*H*₂CH₂-), 1.62-1.22 (m, 96H, -CH₂C*H*₂CH₂-, -C*H*₂CH₃), 0.88 (t, 12H, -CH₂C*H*₃). MS (ESI): m/z=1507.3 ([M+H]⁺).

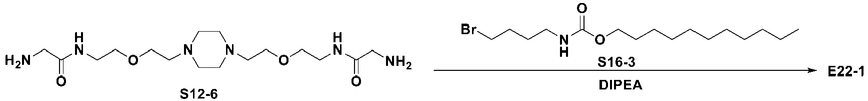

### Example 23: Cationic Lipid (E23-1)

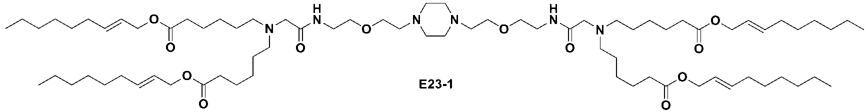

Following the same procedure in the Step b of Example **17,** the starting material **S1-3** was replaced with the starting material **S12-6** (0.37 g, 1.0 mmol) to obtain cationic lipid **E23-1** (1.10 g). ¹H NMR (400 MHz, CDCl₃) δ: 5.82-5.71 (m, 4H, -C(=O)OCH₂C*H*=CH-), 5.56-5.54 (m, 4H, -C(=O)OCH₂CH=C*H*-), 4.55 (t, 8H, -C(=O)OC*H*₂CH=CH-), 3.59 (t, 4H, -C(=O)NHCH₂C*H*₂O-), 3.52 (t, 4H, pip-CH₂C*H*₂-), 3.48-3.42 (m, 4H, -C*H*₂NHC(=O)-), 3.04 (s, 4H, -NHC(=O)C*H*₂-), 2.76-2.48 (m, 12H; 8H, pip-*H*; 4H, pip-CH₂C*H*₂-), 2.46-2.43 (m, 8H, >NC*H*₂CH₂-), 2.28 (t, 8H, -C*H*₂C(=O)O-), 2.07-1.99 (m, 8H, -CH=CHC*H*₂CH₂-), 1.62-1.22 (m, 56H, -CH₂C*H*₂CH₂-, -C*H*₂CH₃), 0.88 (t, 12H, -CH₂C*H*₃). MS (ESI): m/z=1327.0 ([M+H]⁺).

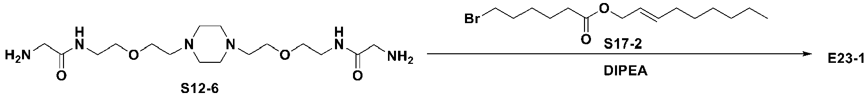

### Example 24: Cationic Lipid (E24-1)

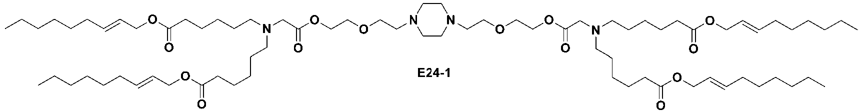

The preparation process is as follows:
Step a: 2-(2-bromoethoxy)ethanol **(S24-1,** 2.54 g, 15.0 mmol) and **S12-1** (0.52 g, 6.0 mmol) were dissolved in acetonitrile (80 mL), **potassium carbonate** (5.80 g, 42.0 mmol) was added to the above system, and then the reaction was heated to 60°C and stirred for 3 hours. After the reaction was completed, the reaction solution was filtered after cooled to room temperature, the filtrate was concentrated, and the residue was purified by column chromatography to obtain **S24-2** (0.90 g).
Step b: **S24-2** (0.79 g, 3.0 mmol), *p*-toluenesulfonic acid (0.57 g, 3.3 mmol), and anhydrous toluene (20 mL) were added to a round-bottom flask, and then the reaction solution was heated to 135°C and refluxed for 3 hours. After the reaction was completed, the reaction solution was cooled to room temperature, and **S1-2** (1.10 g, 6.3 mmol) was added in batches, and then the temperature was raised to 135°C to reflux the reaction overnight. After the reaction was completed, the reaction solution was concentrated, and the residue was dissolved in dichloromethane, sequentially washed twice with water, twice with saturated sodium carbonate solution, once with saturated saline, and finally dried with anhydrous magnesium sulfate, filtered and concentrated, and then purified by column chromatography to obtain compound **S24-3** (1.12 g).
Step c: Removal of the Boc protecting group. In a dry and clean round-bottom flask, a solution of trifluoroacetic acid/dichloromethane (1:2, v/v) was prepared. Under ice bath conditions, the dichloromethane solution of **S24-3** (0.69 g, 1.2 mmol) was slowly added, and the reaction was carried out at room temperature for 2 hours. After the reaction was completed, water was added to the reaction solution and stirred well, followed by extraction. The organic phase was dried with anhydrous magnesium sulfate, filtered, the filtrate was concentrated, and then the crude product was recrystallized to obtain **S24-4** containing free amine groups (0.42 g, 92.9%).
Step d: under nitrogen atmosphere, compound **S24-4** (0.38 g, 1.0 mmol) was dissolved in acetonitrile (30 mL), **S17-2** (1.91 g, 6.0 mmol) and **DIPEA** (0.65 g, 5.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E24-1** (1.10 g). ¹H NMR (400 MHz, CDCl₃) δ: 5.82-5.70 (m, 4H, -C(=O)OCH₂C*H*=CH-), 5.56-5.54 (m, 4H, -C(=O)OCH₂CH=C*H*-), 4.55 (t, 8H, -C(=O)OC*H*₂CH=CH-), 4.25 (t, 4H, -OCH₂C*H*₂OC(=O)-), 3.70-3.54 (m, 8H; 4H, -OC*H*₂CH₂OC(=O)-; 4H, pip-CH₂C*H*₂-), 3.44 (s, 4H, -OC(=O)C*H*₂N<), 2.98 (t, 8H, >NC*H*₂(CH₂)₄-), 2.84-2.34 (t, 12H; 4H, pip-C*H*₂CH₂-; 8H, pip-H), 2.29 (t, 8H, -C*H*₂C(=O)O-), 2.06-1.99 (m, 8H, -CH=CHC*H*₂CH₂-), 1.65-1.22 (m, 56H, -CH₂C*H*₂CH₂-, -C*H*₂CH₃), 0.89 (t, 12H, -CH₂C*H*₃). MS (ESI): m/z=1329.0 ([M+H]⁺).

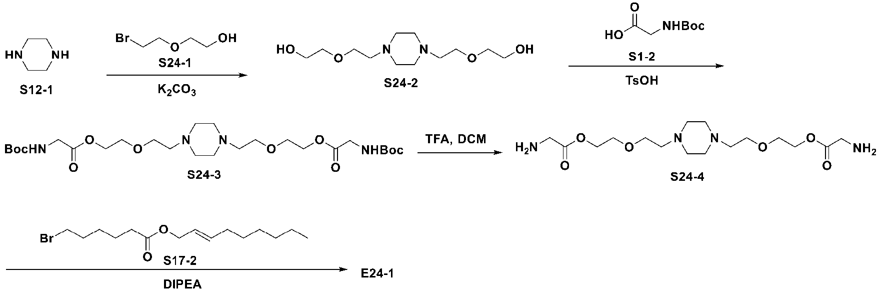

### Example 25: Cationic Lipid (E25-1)

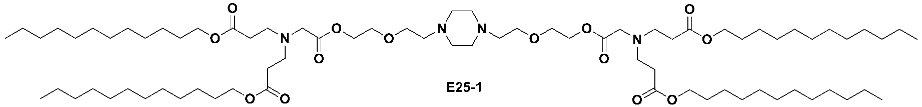

The preparation process is as follows:
**S24-4** (0.38 g, 1.0 mmol) was dissolved in isopropanol, and a sufficient amount of anhydrous potassium carbonate was added under stirring, and then the reaction solution was stirred at room temperature until the reaction solution became alkaline. **S6-1** (1.44 g, 6.0 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E25-1** (1.04 g). ¹H NMR (400 MHz, CDCl₃) δ: 4.24 (t, 4H, -OCH₂C*H*₂OC(=O)-), 4.05 (t, 8H, -C(=O)OC*H*₂), 3.70-3.54 (m, 8H; 4H, -OC*H*₂CH₂OC(=O)-; 4H, pip-CH₂C*H*₂-), 3.44 (s, 4H, -OC(=O)C*H*₂N<), 2.98 (t, 8H, >NC*H*₂CH₂C(=O)O-), 2.84-2.34 (t, 20H; 8H, -C*H*₂C(=O)O-; 4H, pip-C*H*₂CH₂-; 8H, pip-*H*), 1.63-1.26 (m, 80H, -CH₂C*H*₂CH₂-, -C*H*₂CH₃), 0.88 (t, 12H, -CH₂C*H*₃). MS (ESI): m/z=1337.9 ([M+H]⁺).

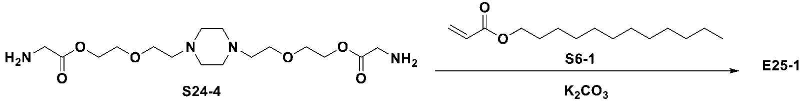

### Example 26: Cationic Lipid (E26-1)

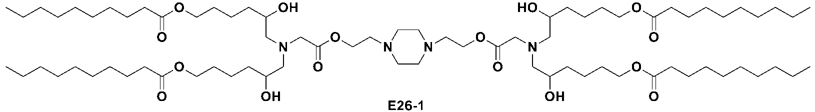

The preparation process is as follows:
Steps a: under nitrogen atmosphere, **DCC** (3.63 g, 17.6 mmol) was added to a round-bottom flask containing 5-hexen-1-ol **(S26-2,** 1.38 g, 8.0 mmol), decanoic acid **(S26-1,** 0.96 g, 9.6 mmol), and **DMAP** (0.24 g, 2.0 mmol) dissolved in dichloromethane (30 mL), and then reacted at room temperature for 16 hours. After the reaction was completed, the precipitate was removed by filtration, the filtrate was concentrated, and the crude product was purified by column chromatography to obtain **S26-3** (1.65 g).
Step b: compound **S26-3** (1.27 g, 5.0 mmol) was dissolved in dichloromethane (20 mL), and meta-chloroperoxybenzoic acid **(m-CPBA,** 1.29 g, 7.5 mmol) was added under ice bath conditions. After stirring for 15 minutes, the ice bath was removed and the reaction was stirred overnight. After the reaction was completed, excess saturated sodium bisulfite solution was added to consume the unreacted **m-CPBA,** and the dichloromethane was removed by concentrated. 20 mL of ethyl acetate was added, followed by washing three times, each with 20 mL of saturated sodium bicarbonate solution, and washing once with 20 mL of saturated sodium chloride solution. The organic phase was dried with anhydrous magnesium sulfate, filtered, the filtrate was concentrated, and then the crude product was purified by column chromatography to obtain **S26-4** (1.00 g).
Step c: **S1-3** (0.29 g, 1.0 mmol) and **DIPEA** (0.39 g, 3.0 mmol) were sequentially added to a methanol solution of **S26-4** (1.62 g, 6.0 mmol), and the reaction solution was placed in a reflux apparatus (90°C) and stirred continuously for 24 hours. After the reaction was completed, the reaction solution was concentrated, and the crude product was purified by column chromatography to obtain the cationic lipid **E26-1** (0.92 g).¹H NMR (400 MHz, CDCl₃) δ: 4.24 (t, 4H, pip-CH₂C*H*₂-), 4.17-4.14 (t, 8H, -C*H*₂OC(=O)-), 3.51-3.42 (m, 4H, -CH(OH)-), 3.33 (s, 4H, -OC(=O)C*H*₂N<), 2.78-2.31 (m, 20H; 4H, pip-C*H*₂CH₂-; 8H, pip-*H*; 8H, >CHC*H*₂N<), 2.18-2.10 (m, 8H, -CH₂C*H*₂C(=O)O-), 1.84-1.76 (m, 8H, -C(=O)OCH₂C*H*₂-), 1.64-1.22 (m, 72H, -CH₂C*H*₂CH₂-, -C*H*₂CH₃), 0.87 (t, 12H, -CH₂C*H*₃). MS (ESI): m/z=1369.1 ([M+H]⁺).

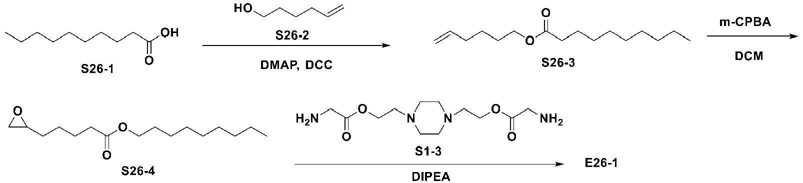

### Example 27: Cationic Lipid (E27-1)

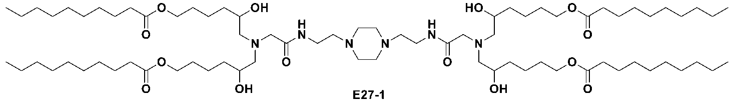

The preparation process is as follows:
**S9-3** (0.34 g, 1.2 mmol) and **DIPEA** (0.46 g, 3.6 mmol) were sequentially added to a methanol solution of **S26-4** (1.94 g, 7.2 mmol), and the reaction solution was placed in a reflux apparatus (90°C) and stirred continuously for 24 hours. After the reaction was completed, the reaction solution was concentrated, and the crude product was purified by column chromatography to obtain the cationic lipid **E27-1** (1.09 g). ¹H NMR (400 MHz, CDCl₃) δ: 4.17-4.14 (m, 8H, -C*H*₂OC(=O)-), 3.51-3.33 (m, 8H; 4H, -CH(OH)-; 4H, pip-CH₂C*H*₂-), 3.04 (s, 4H, -NHC(=O)C*H*₂-), 2.79-2.31 (m, 20H; 4H, pip-C*H*₂CH₂-; 8H, pip-*H*; 8H, >CHC*H*₂N<), 2.18-2.10 (m, 8H, -CH₂C*H*₂C(=O)O-), 1.84-1.76 (m, 8H, -C(=O)OCH₂C*H*₂-), 1.64-1.22 (m, 72H, -CH₂C*H*₂CH₂-, -C*H*₂CH₃), 0.88 (t, 12H, -CH₂C*H*₃). MS (ESI): m/z=1367.1 ([M+H]⁺).

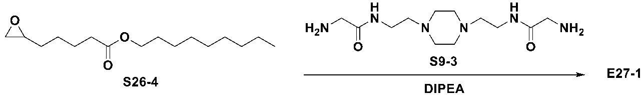

### Example 28: Cationic Lipid (E28-1)

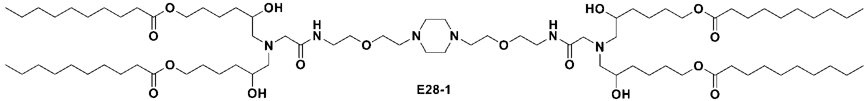

Following the same procedure in Example **27,** the starting material **S9-3** was replaced with the starting material **S12-6** (1.51 g, 7.2 mmol) to obtain cationic lipid **E28-1** (1.19 g).¹H NMR (400 MHz, CDCl₃) δ: 4.07-4.00 (m, 8H, -C*H*₂OC(=O)-), 3.72-3.39 (m, 16H; 8H, -C*H*₂OC*H*₂-; 4H, -CH(OH)-; 4H, -C*H*₂NHC(=O)-), 3.31-3.19 (m, 4H, -NHC(=O)C*H*₂-), 2.82-2.33 (m, 20H; 4H, pip-C*H*₂CH₂-; 8H, >CHC*H*₂N<; 8H, pip-*H*), 2.27 (t, 8H, -C*H*₂C(=O)O-), 1.67-1.19 (m, 80H, -CH₂C*H*₂CH₂-, -C*H*₂CH₃), 0.88 (t, 12H, -CH₂C*H*₃). MS (ESI): m/z=1455.8 ([M+H]⁺).

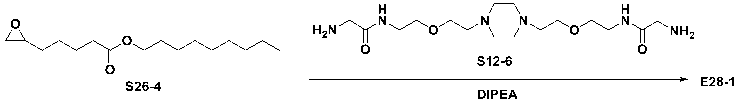

### Example 29: Cationic Lipid (E29-1)

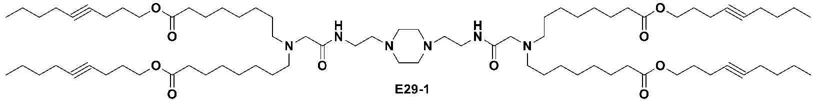

Following the same procedure in the Step c of Example 1, the starting material **S1-3** was replaced with the starting material **S9-3** (0.29 g, 1.0 mmol) to obtain cationic lipid **E29-1** (1.09 g). ¹H NMR (400 MHz, CDCl₃) δ: 4.17-4.11 (m, 8H, -C(=O)OC*H*₂-), 3.46-3.33 (m, 4H, pip-CH₂C*H*₂-), 3.03 (s, 4H, -NHC(=O)C*H*₂-), 2.84-2.42 (m, 20H; 8H, pip-H; 4H, pip-C*H*₂CH₂-; 8H, >NC*H*₂CH₂-), 2.33-2.20 (m, 16H, -C*H*₂D≡CC*H*₂(CH₂)₂CH₃), 2.18-2.11 (m, 8H, -CH₂C*H*₂C(=O)O-), 1.84-1.76 (m, 8H, -C(=O)OCH₂C*H*₂CH₂-), 1.64-1.22 (m, 56H, -CH₂C*H*₂CH₂-, -C*H*₂CH₃), 0.89 (t, 12H, -CH₂C*H*₃). MS (ESI): m/z=1343.1 ([M+H]⁺).

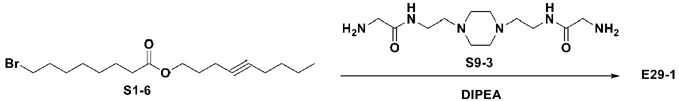

### Example 30: Cationic Lipid (E30-1)

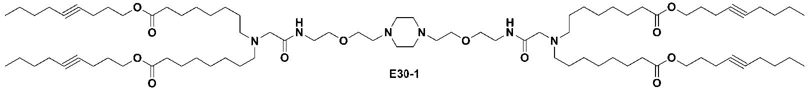

Following the same procedure in the Step c of Example 1, the starting material **S1-3** was replaced with the starting material **S12-6** (0.37 g, 1.0 mmol) to obtain cationic lipid **E30-1** (1.17 g). ¹H NMR (400 MHz, CDCl₃) δ: 4.18-4.10 (m, 8H, -C(=O)OC*H*₂-), 3.59 (t, 4H, -C(=O)NHCH₂C*H*₂O-), 3.52 (t, 4H, pip-CH₂C*H*₂-), 3.48-3.42 (m, 4H, -C*H*₂NHC(=O)-), 3.04 (s, 4H, -NHC(=O)C*H*₂N<), 2.84-2.42 (m, 20H; 8H, pip-*H*; 4H, pip-C*H*₂CH₂-; 8H, >NC*H*₂CH₂-), 2.33-2.20 (m, 16H, -C*H*₂C≡CCH₂(CH₂)₂CH₃), 2.18-2.11 (m, 8H, -CH₂C*H*₂C(=O)O-), 1.84-1.76 (m, 8H, -C(=O)OCH₂C*H*₂CH₂-), 1.64-1.21 (m, 56H, -CH₂C*H*₂CH₂-, -C*H*₂CH₃), 0.89 (t, 12H, -CH₂C*H*₃). MS (ESI): m/z=1431.1 ([M+H]⁺).

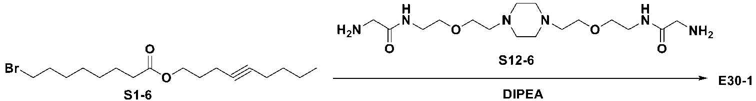

### Example 31: Cationic Lipid (E31-1)

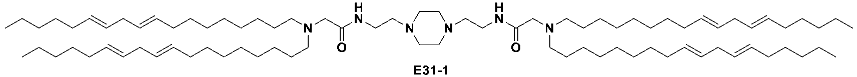

Following the same procedure in Example **2,** the starting material **S1-3** was replaced with the starting material **S9-3** (0.29 g, 1.0 mmol) to obtain cationic lipid **E31-1** (1.19 g). ¹H NMR (400 MHz, CDCl₃) δ: 5.45-5.26 (m, 16H, -C*H*=C*H*-), 3.46-3.33 (m, 4H, pip-CH₂C*H*₂-), 3.03 (s, 4H, -NHC(=O)C*H*₂-), 2.82-2.43 (m, 28H; 8H, pip-*H*; 4H, pip-C*H*₂CH₂-; 8H, >NC*H*₂CH₂-; 8H, -CH=CHC*H*₂CH=CH-), 2.03-1.97 (m, 16H, -CH=CHC*H*₂-), 1.64-1.22 (m, 72H, -CH₂C*H*₂CH₂-, -C*H*₂CH₃), 0.89 (t, 12H, -CH₂C*H*₃). MS (ESI): m/z=1279.2 ([M+H]⁺).

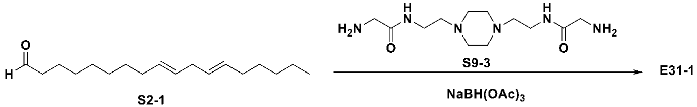

### Example 32: Cationic Lipid (E32-1)

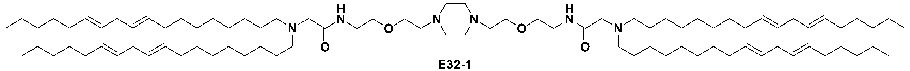

Following the same procedure in Example 2, the starting material **S1-3** was replaced with the starting material **S12-6** (0.37 g, 1.0 mmol) to obtain cationic lipid **E32-1** (1.26 g). ¹H NMR (400 MHz, CDCl₃) δ: 5.45-5.24 (m, 16H, *-CH=CH-),* 3.59 (t, 4H, -C(=O)NHCH₂C*H*₂O-), 3.52 (t, 4H, pip-CH₂C*H*₂-), 3.48-3.42 (m, 4H, -C*H*₂NHC(=O)-), 3.04 (s, 4H, -NHC(=O)C*H*₂-), 2.81-2.43 (m, 28H; 8H, pip-*H*; 4H, pip-C*H*₂CH₂-; 8H, >NC*H*₂CH₂-; 8H, -CH=CHC*H*₂CH=CH-), 2.05-1.97 (m, 16H, -CH=CHC*H*₂-), 1.64-1.22 (m, 72H, -CH₂C*H*₂CH₂-, -C*H*₂CH₃), 0.89 (t, 12H, -CH₂C*H*₃). MS (ESI): m/z=1367.3 ([M+H]⁺).

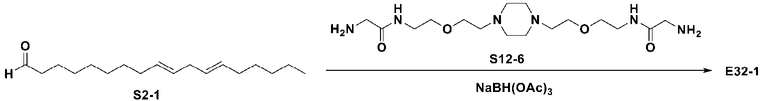

### Example 33: Cationic Lipid (E33-1)

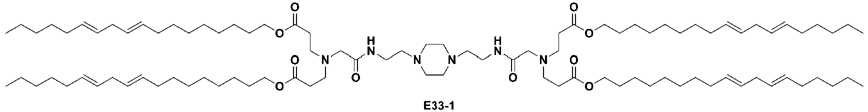

Following the same procedure in the Step b of Example **3,** the starting material **S1-3** was replaced with the starting material **S9-3** (0.29 g, 1.0 mmol) to obtain cationic lipid **E33-1** (1.21 g). ¹H NMR (400 MHz, CDCl₃) δ: 5.45-5.24 (m, 16H, *-CH=CH-),* 4.16-4.09 (m, 8H, -C(=O)OC*H*₂-), 3.46-3.34 (m, 4H, pip-CH₂C*H*₂-), 3.03 (s, 4H, -NHC(=O)C*H*₂-), 2.83-2.43 (m, 28H; 8H, pip-*H*; 4H, pip-C*H*₂CH₂-; 8H, >NC*H*₂CH₂-; 8H, -CH=CHC*H*₂CH=CH-), 2.23 (t, 8H, -CH₂C*H*₂C(=O)O-), 2.03-1.97 (m, 16H, -CH=CHC*H*₂-), 1.84-1.76 (m, 8H, -C(=O)OCH₂C*H*₂CH₂-), 1.64-1.22 (m, 64H, -CH₂C*H*₂CH₂-, -C*H*₂CH₃), 0.89 (t, 12H, -CH₂C*H*₃). MS (ESI): m/z=1567.3 ([M+H]⁺).

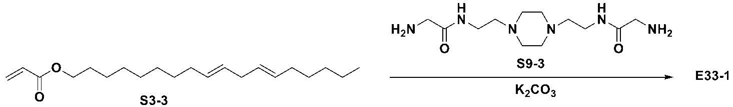

### Example 34: Cationic Lipid (E34-1)

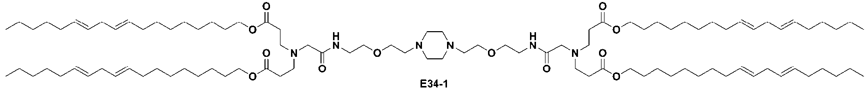

Following the same procedure in the Step b of Example 3, the starting material **S1-3** was replaced with the starting material **S12-6** (0.37 g, 1.0 mmol) to obtain cationic lipid **E34-1** (1.28 g). ¹H NMR (400 MHz, CDCl₃) δ: 5.44-5.24 (m, 16H, *-CH=CH-),* 4.16-4.10 (m, 8H, -C(=O)OC*H*₂-), 3.59 (t, 4H, -C(=O)NHCH₂C*H*₂O-), 3.52 (t, 4H, pip-CH₂C*H*₂-), 3.48-3.42 (m, 4H, -C*H*₂NHC(=O)-), 3.04 (s, 4H, -NHC(=O)C*H*₂-), 2.81-2.43 (m, 28H; 8H, pip-*H*; 4H, pip-C*H*₂CH₂-; 8H, >NC*H*₂CH₂-; 8H, -CH=CHC*H*₂CH=CH-), 2.23 (t, 8H, -CH₂C*H*₂C(=O)O-), 2.03-1.97 (m, 16H, -CH=CHC*H*₂-), 1.82-1.76 (m, 8H, -C(=O)OCH₂C*H*₂CH₂-), 1.64-1.20 (m, 64H, -CH₂C*H*₂CH₂-, -C*H*₂CH₃), 0.87 (t, 12H, -CH₂C*H*₃). MS (ESI): m/z=1655.4 ([M+H]⁺).

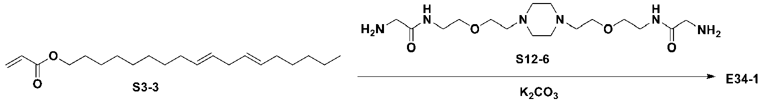

### Example 35: Cationic Lipid (E35-1)

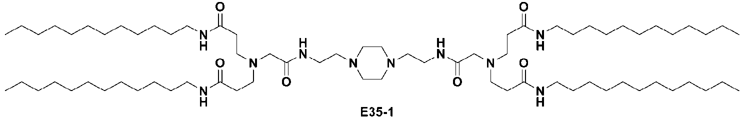

Following the same procedure in Example **4,** the starting material **S1-3** was replaced with the starting material **S9-3** (0.29 g, 1.0 mmol) to obtain cationic lipid **E35-1** (0.94 g). ¹H NMR (400 MHz, CDCl₃) δ: 3.46-3.33 (m, 4H, pip-CH₂C*H*₂-), 3.28 (dd, 8H, -C(=O)NHC*H*₂(CH₂)₁₀-), 3.03 (s, 4H, -NHC(=O)C*H*₂N<), 2.82-2.62 (m, 20H; 8H, pip-H; 4H, pip-C*H*₂CH₂-; 8H, >NC*H*₂CH₂-), 2.37 (t, 8H, >NCH₂C*H*₂-), 1.65-1.22 (m, 80H, -CH₂C*H*₂CH₂-, -C*H*₂CH₃), 0.89 (t, 12H, -CH₂C*H*₃). MS (ESI): m/z=1243.1 ([M+H]⁺).

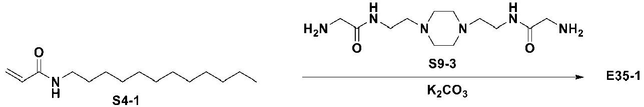

### Example 36: Cationic Lipid (E36-1)

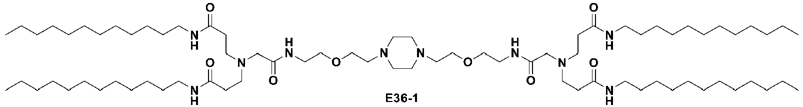

Following the same procedure in Example **4,** the starting material **S1-3** was replaced with the starting material **S12-6** (0.37 g, 1.0 mmol) to obtain cationic lipid **E36-1** (1.00 g). ¹H NMR (400 MHz, CDCl₃) δ: 3.59 (t, 4H, -C(=O)NHCH₂C*H*₂O-), 3.52 (t, 4H, pip-CH₂C*H*₂-), 3.48-3.42 (m, 4H, -C*H*₂NHC(=O)-), 3.28 (dd, 8H, -C(=O)NHCH₂(CH₂)₁₀-), 3.04 (s, 4H, -NHC(=O)C*H*₂N<), 2.81-2.62 (m, 20H; 8H, pip-H; 4H, pip-C*H*₂CH₂-; 8H, >NC*H*₂CH₂-), 2.39 (t, 8H, >NCH₂C*H*₂-), 1.64-1.22 (m, 80H, -CH₂C*H*₂CH₂-, -C*H*₂CH₃), 0.86 (t, 12H, -CH₂C*H*₃). MS (ESI): m/z=1331.2 ([M+H]⁺).

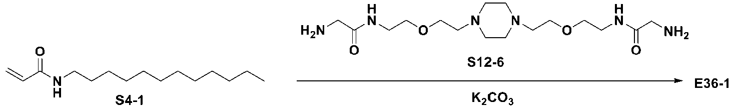

### Example 37: Cationic Lipid (E37-1)

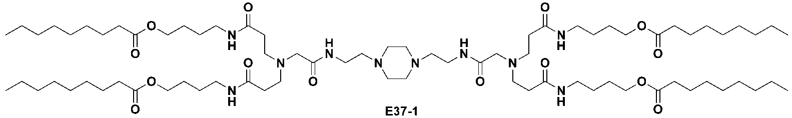

Following the same procedure in Example **5,** the starting material **S1-3** was replaced with the starting material **S9-3** (0.29 g, 1.0 mmol) to obtain cationic lipid **E37-1** (1.09 g). ¹H NMR (400 MHz, CDCl₃) δ: 4.17-4.11 (m, 8H, -C(=O)OC*H*₂-), 3.46-3.33 (m, 4H, pip-CH₂C*H*₂-), 3.28 (dd, 8H, -C(=O)NHC*H*₂(CH₂)₃-), 3.03 (s, 4H, -NHC(=O)C*H*₂N<), 2.82-2.62 (m, 20H; 8H, pip-*H*; 4H, pip-C*H*₂CH₂-; 8H, >NC*H*₂CH₂-), 2.39 (t, 8H, -C*H*₂C(=O)NH-), 2.18-2.11 (m, 8H, -CH₂C*H*₂C(=O)O-), 1.84-1.76 (m, 8H, -C(=O)OCH₂C*H*₂CH₂-), 1.64-1.22 (m, 56H, -CH₂C*H*₂CH₂-, -C*H*₂CH₃), 0.89 (t, 12H, -CH₂C*H*₃). MS (ESI): m/z=1419.1 ([M+H]⁺).

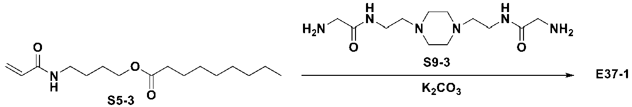

### Example 38: Cationic Lipid (E38-1)

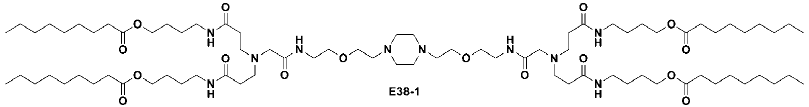

Following the same procedure in the Step b of Example **5,** the starting material **S1-3** was replaced with the starting material **S12-6** (0.37 g, 1.0 mmol) to obtain cationic lipid **E38-1** (1.16 g). ¹H NMR (400 MHz, CDCl₃) δ: 4.18-4.12 (m, 8H, -C(=O)OC*H*₂-), 3.59 (t, 4H, -C(=O)NHCH₂C*H*₂O-), 3.52 (t, 4H, pip-CH₂C*H*₂-), 3.48-3.42 (m, 4H, -C*H*₂NHC(=O)-), 3.28 (dd, 8H, -C(=O)NHC*H*₂(CH₂)₃-), 3.03 (s, 4H, -NHC(=O)C*H*₂N<), 2.81-2.62 (m, 20H; 8H, pip-*H*; 4H, pip-C*H*₂CH₂-; 8H, >NC*H*₂CH₂-), 2.39 (t, 8H, >NCH₂C*H*₂-), 2.18-2.11 (m, 8H, -CH₂C*H*₂C(=O)O-), 1.84-1.76 (m, 8H, -C(=O)OCH₂C*H*₂CH₂-), 1.642-1.20 (m, 56H, -CH₂C*H*₂CH₂-, -C*H*₂CH₃), 0.89 (t, 12H, -CH₂C*H*₃). MS (ESI): m/z=1507.1 ([M+H]⁺).

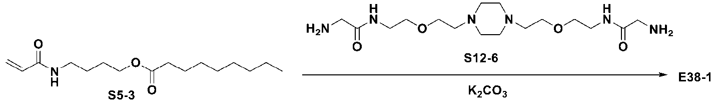

### Example 39: Cationic Lipid (E39-1)

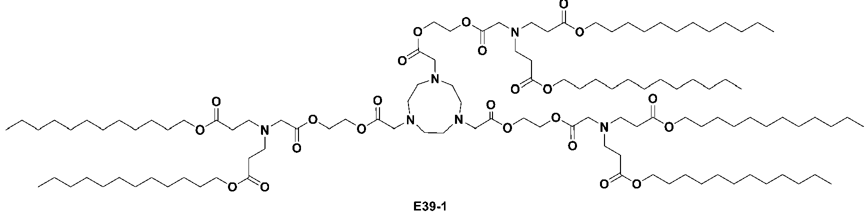

The preparation process is as follows:
Step a: under nitrogen atmosphere, **DCC** (6.80 g, 33.0 mmol) was added to a round-bottom flask containing **S39-1** (1.52 g, 5.0 mmol, **S39-1** was obtained by the reaction of 1,4,7-triazacyclononane 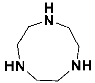 with bromoacetic acid 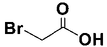 ), t-butyldimethyl (hydroxyethoxy) silane **(S39-2,** 3.08 g, 17.5 mmol) and **DMAP** (0.49 g, 4.0 mmol) dissolved in dichloromethane (100 mL), and then reacted at room temperature for 16 hours. After the reaction was completed, the precipitate was removed by filtration, the filtrate was concentrated, and the residue was dissolved in THF (10 mL), and then **TBAF** (10 mL, 1 M) was added under nitrogen atmosphere, reacted for overnight to remove TBS protection. After the reaction was completed, the reaction solution was concentrated and extracted. The organic phases were combined, dried with anhydrous magnesium sulfate, filtered, the filtrate was concentrated, and then the crude product was purified by column chromatography to obtain **S39-3** (1.62 g).
Step b: under nitrogen atmosphere, **DCC** (4.08 g, 19.8 mmol) was added to a round-bottom flask containing **S39-3** (1.31 g, 8.0 mmol), **S1-2** (1.84 g, 10.5 mmol), and **DMAP** (0.29 g, 2.4 mmol) dissolved in dichloromethane (50 mL), and then reacted at room temperature for 16 hours. After the reaction was completed, the precipitate was removed by filtration, the filtrate was concentrated, the residue was dissolved in dichloromethane, and slowly added dropwise to a pre-prepared solution of trifluoroacetic acid/dichloromethane (1:2, v/v) solution under ice bath conditions, and then the solution was reacted at room temperature for 2 hours. After the reaction was completed, water was added to the reaction solution and stirred well, followed by extraction. The organic phase was dried with anhydrous magnesium sulfate, filtered, the filtrate was concentrated, and then the crude product was recrystallized to obtain **S39-4** (1.33 g).
Step c: **S39-4** (0.61 g, 1.0 mmol) was dissolved in isopropanol, and a sufficient amount of anhydrous potassium carbonate was added under stirring, and then the reaction solution was stirred at room temperature until the reaction solution became alkaline. **S6-1** (2.16 g, 9.0 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E39-1** (1.55 g). ¹H NMR (400 MHz, CDCl₃) δ: 4.27-4.09 (m, 24H; 12H, -C(=O)OC*H*₂C*H*₂OC(=O)-; 12H, -C(=O)OC*H*₂(CH₂)₁₀-), 3.36 (s, 6H, -OC(=O)C*H*₂N<), 3.20 (s, 6H, Ring-C*H*₂-), 2.84-2.43 (m, 24H; 12H, Ring-*H*; 12H, >NC*H*₂CH₂-), 2.18-2.10 (m, 12H, -CH₂C*H*₂C(=O)O-), 1.84-1.76 (m, 12H, -C(=O)OCH₂C*H*₂CH₂-), 1.64-1.22 (m, 108H, -CH₂C*H*₂CH₂-, -C*H*₂CH₃), 0.89 (t, 18H, -CH₂C*H*₃). MS (ESI): m/z=2047.5 ([M+H]⁺).

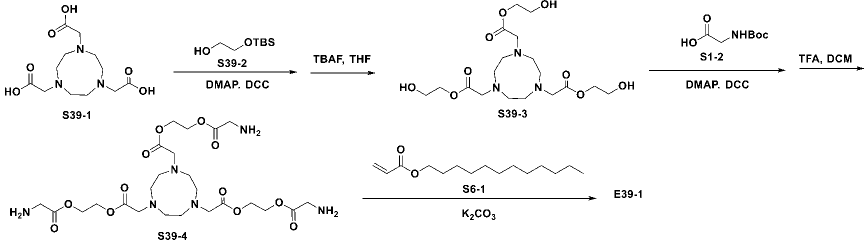

### Example 40: Cationic Lipid (E40-1)

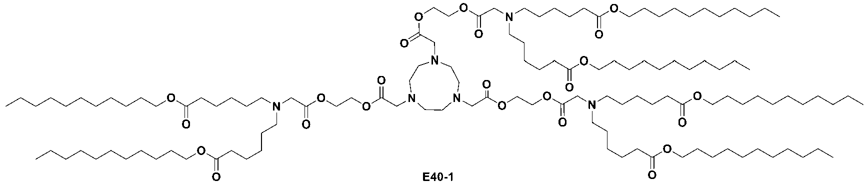

The preparation process is as follows:
Under nitrogen atmosphere, compound **S39-4** (0.61 g, 1.0 mmol) was dissolved in acetonitrile (30 mL), **S8-3** (3.14 g, 9.0 mmol) and **DIPEA** (1.03 g, 8.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E40-1** (1.11 g). ¹H NMR (400 MHz, CDCl₃) δ: 4.27-4.09 (m, 24H; 12H, -C(=O)OC*H*₂C*H*₂OC(=O)-; 12H, -C(=O)OC*H*₂(CH₂)₁₀-), 3.36 (s, 6H, -OC(=O)C*H*₂N<), 3.20 (s, 6H, Ring-C*H*₂-), 2.84-2.43 (m, 24H; 12H, Ring-*H*; 12H, >NC*H*₂CH₂-), 2.18-2.10 (m, 12H, -CH₂C*H*₂C(=O)O-), 1.84-1.76 (m, 12H, -C(=O)OCH₂C*H*₂CH₂-), 1.64-1.22 (m, 132H, -CH₂C*H*₂CH₂-, -C*H*₂CH₃), 0.89 (t, 18H, -CH₂C*H*₃). MS (ESI): m/z=2215.7 ([M+H]⁺).

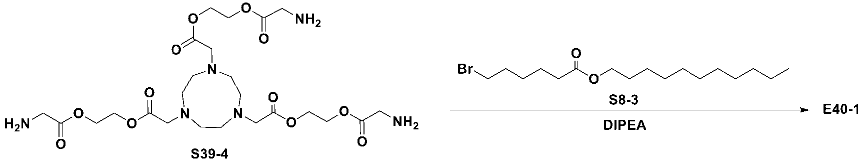

### Example 41: Cationic Lipid (E41-1)

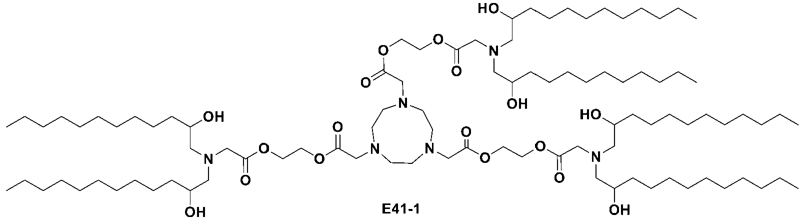

The preparation process is as follows:
**S39-4** (0.73 g, 1.2 mmol) and **DIPEA** (0.70 g, 5.4 mmol) were sequentially added to a methanol solution **of S7-1** (1.98 g, 10.8 mmol), and the reaction solution was placed in a reflux apparatus (90°C) and stirred continuously for 24 hours. After the reaction was completed, the reaction solution was concentrated, and the crude product was purified by column chromatography to obtain the cationic lipid **E41-1** (1.38 g). ¹H NMR (400 MHz, CDCl₃) δ: 4.27-4.09 (m, 12H, -C(=O)OC*H*₂C*H*₂OC(=O)-), 3.51-3.41 (m, 6H, -C*H*(OH)-), 3.34 (s, 6H, -OC(=O)C*H*₂N<), 3.20 (s, 6H, Ring-C*H*₂-), 2.84-2.43 (m, 24H; 12H, Ring-*H*; 12H, >CHC*H*₂N<), 1.44-1.23 (m, 108H, -CH₂C*H*₂CH₂-, -C*H*₂CH₃), 0.89 (t, 18H, -CH₂C*H*₃). MS (ESI): m/z=1711.4 ([M+H]⁺).

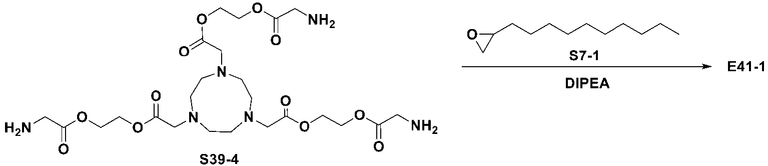

### Example 42: Cationic Lipid (E42-1)

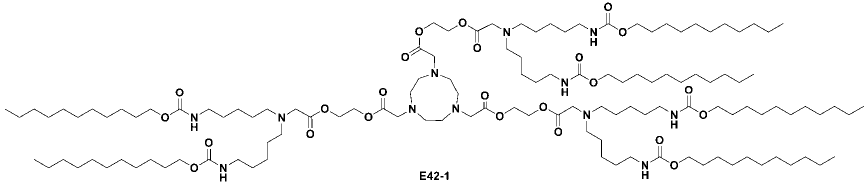

The preparation process is as follows:
Under nitrogen atmosphere, compound **S39-4** (0.30 g, 0.5 mmol) was dissolved in acetonitrile (20 mL), **S16-3** (1.58 g, 4.5 mmol) and **DIPEA** (0.52 g, 4.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E42-1** (0.94 g). ¹H NMR (400 MHz, CDCl₃) δ: 4.27-4.09 (m, 24H; 12H, -C(=O)OC*H*₂C*H*₂OC(=O)-; 12H, -NHC(=O)OC*H*₂-), 3.36 (s, 6H, -OC(=O)C*H*₂N<), 3.20 (s, 6H, Ring-C*H*₂-), 3.15 (t, 12H, -C*H*₂NHC(=O)O-), 2.84-2.43 (m, 24H; 12H, Ring-H; 12H, >NC*H*₂CH₂-), 1.62-1.22 (m, 144H, -CH₂C*H*₂CH₂-, -C*H*₂CH₃), 0.88 (t, 18H, -CH₂C*H*₃). MS (ESI): m/z=2305.8 ([M+H]⁺).

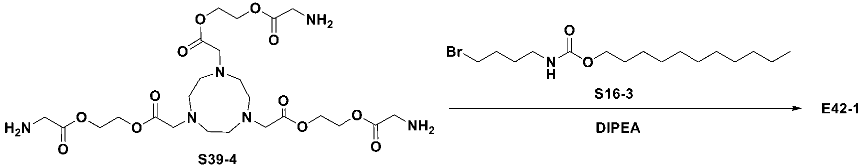

### Example 43: Cationic Lipid (E43-1)

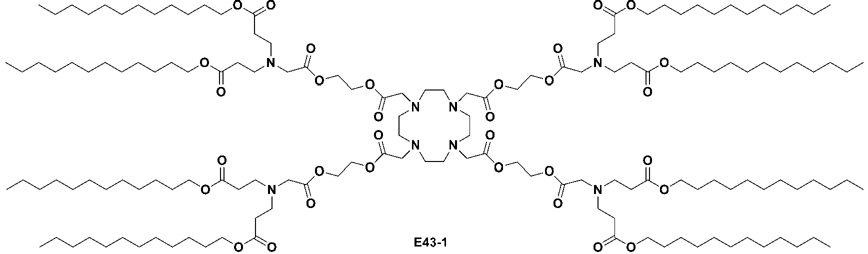

The preparation process is as follows:
Step a: under nitrogen atmosphere, **DCC** (9.06 g, 44.0 mmol) was added to a round-bottom flask containing **S43-1** (2.02 g, 5.0 mmol), **S39-2** (4.4 g, 25.0 mmol), and **DMAP** (0.61 g, 5.0 mmol) dissolved in dichloromethane (150 mL), and then reacted at room temperature for 16 hours. After the reaction was completed, the precipitate was removed by filtration, the filtrate was concentrated, and the residue was dissolved in THF (15 mL), and then **TBAF** (15 mL, 1 M) was added under nitrogen atmosphere, reacted for overnight to remove TBS protection. After the reaction was completed, the reaction solution was concentrated, and extracted. The organic phases were combined, dried with anhydrous magnesium sulfate, filtered, the filtrate was concentrated, and then the crude product was purified by column chromatography to obtain **S43-2** (2.13 g).
Step b: under nitrogen atmosphere, **DCC** (5.44 g, 26.4 mmol) was added to a round-bottom flask containing **S43-2** (1.74 g, 8.0 mmol), **S1-2** (2.63 g, 15.0 mmol), and **DMAP** (0.37 g, 3.0 mmol) dissolved in dichloromethane (50 mL), and then reacted at room temperature for 16 hours. After the reaction was completed, the precipitate was removed by filtration, the filtrate was concentrated, the residue was dissolved in dichloromethane, and slowly added dropwise to a pre-prepared solution of trifluoroacetic acid/dichloromethane (1:2, v/v) solution under ice bath conditions, and then the solution was reacted at room temperature for 2 hours. After the reaction was completed, water was added to the reaction solution and stirred well, followed by extraction. The organic phase was dried with anhydrous magnesium sulfate, filtered, the filtrate was concentrated, and then the crude product was recrystallized to obtain **S43-3** (1.76 g).
Step c: **S43-3** (0.81 g, 1.0 mmol) was dissolved in isopropanol, and a sufficient amount of anhydrous potassium carbonate was added under stirring, and then the reaction solution was stirred at room temperature until the reaction solution became alkaline. **S6-1** (2.88 g, 12.0 mmol) was added to the reaction solution, which was then placed in a reflux apparatus (90°C) and stirred for 36 hours. After the reaction was completed, the reaction solution was concentrated, and the crude product was purified by column chromatography to obtain cationic lipid **E43-1** (1.99 g). ¹H NMR (400 MHz, CDCl₃) δ: 4.27-4.07 (m, 32H; 16H, -C(=O)OC*H*₂C*H*₂OC(=O)-; 16H, -C(=O)OC*H*₂(CH₂)₁₀-), 3.36 (s, 8H, -OC(=O)C*H*₂N<), 3.21 (s, 8H, Ring-C*H*₂-), 2.84-2.43 (m, 32H; 16H, Ring-H; 16H, >NC*H*₂CH₂-), 2.19-2.10 (m, 16H, -CH₂C*H*₂C(=O)O-), 1.84-1.76 (m, 16H, -C(=O)OCH₂C*H*₂CH₂-), 1.65-1.22 (m, 144H, -CH₂C*H*₂CH₂-, -C*H*₂CH₃), 0.86 (t, 24H, -CH₂C*H*₃). MS (ESI): m/z=2730.1 ([M+H]⁺).

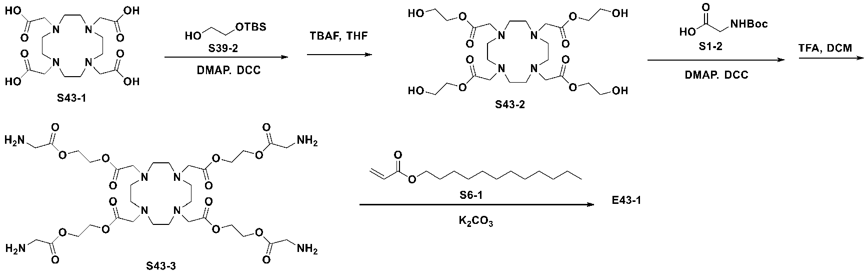

### Example 44: Cationic Lipid (E44-1)

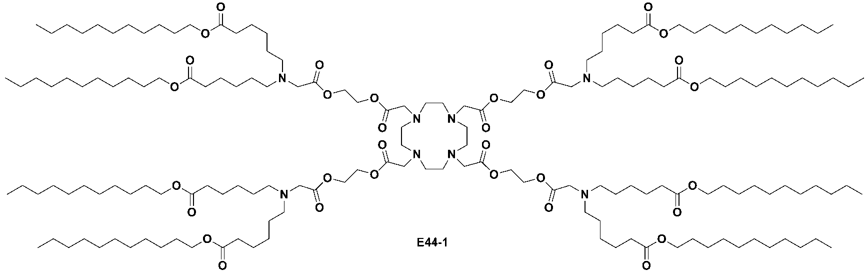

The preparation process is as follows:
Under nitrogen atmosphere, compound **S43-3** (0.81 g, 1.0 mmol) was dissolved in acetonitrile (60 mL), **S8-3** (4.19 g, 12.0 mmol) and **DIPEA** (1.29 g, 10.0 mmol) were added sequentially under slow stirring, and the reaction was stirred for about 20 hours at room temperature. After the reaction was completed, the reaction solution was concentrated, then dissolved in dichloromethane, and sequentially extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E44-1** (2.39 g). ¹H NMR (400 MHz, CDCl₃) δ: 4.25-4.09 (m, 32H; 16H, -C(=O)OC*H*₂C*H*₂OC(=O)-; 16H, -C(=O)OC*H*₂(CH₂)₁₀-), 3.36 (s, 8H, -OC(=O)C*H*₂N<), 3.19 (s, 8H, Ring-C*H*₂-), 2.85-2.43 (m, 32H; 16H, Ring-*H*; 16H, >NC*H*₂CH₂-), 2.18-2.10 (m, 16H, -CH₂C*H*₂C(=O)O-), 1.84-1.76 (m, 16H, -C(=O)OCH₂C*H*₂CH₂-), 1.64-1.56 (m, 16H, -OC(=O)CH₂C*H*₂-), 1.52-1.21 (m, 160H, -CH₂C*H*₂CH₂-, -C*H*₂CH₃), 0.88 (t, 24H, -CH₂C*H*₃). MS (ESI): m/z=2954.3 ([M+H]⁺).

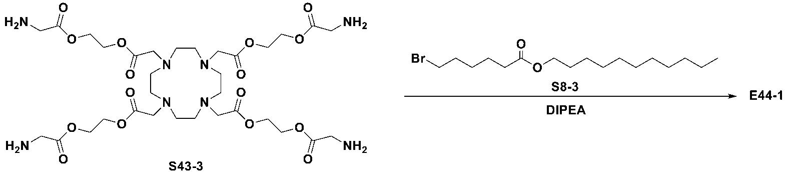

### Example 45: Cationic Lipid (E45-1)

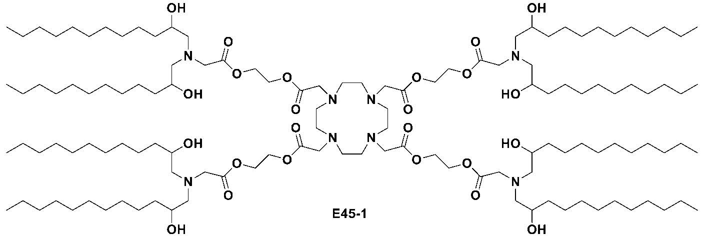

The preparation process is as follows:
**S43-3** (0.97 g, 1.2 mmol) and **DIPEA** (0.93 g, 7.2 mmol) were sequentially added to a methanol solution **of S7-2** (2.65 g, 14.4 mmol), and the reaction solution was placed in a reflux apparatus (90°C) and stirred continuously for 24 hours. After the reaction was completed, the reaction solution was concentrated, and the crude product was purified by column chromatography to obtain the cationic lipid **E45-1** (1.87 g). ¹H NMR (400 MHz, CDCl₃) δ: 4.25-4.09 (m, 16H, -C(=O)OC*H*₂C*H*₂OC(=O)-), 3.52-3.41 (m, 8H, -C*H*(OH)-), 3.33 (s, 8H, -OC(=O)C*H*₂N<), 3.20 (s, 8H, Ring-C*H*₂-), 2.84-2.43 (m, 32H; 16H, Ring-*H*; 16H, >CHC*H*₂N<), 1.46-1.23 (m, 144H, -CH₂C*H*₂CH₂-, -C*H*₂CH₃), 0.87 (t, 24H, -CH₂C*H*₃). MS (ESI): m/z=2281.8 ([M+H]⁺).

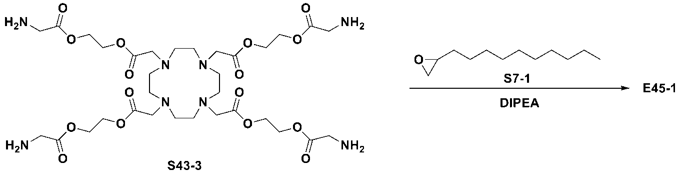

### Example 46: Cationic Lipid (E46-1)

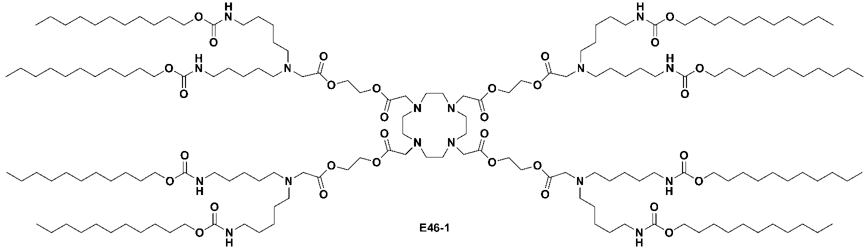

The preparation process is as follows:
Under nitrogen atmosphere, compound **S43-3** (0.41 g, 0.5 mmol) was dissolved in acetonitrile (30 mL), **S16-3** (2.10 g, 6.0 mmol) and **DIPEA** (0.65 g, 5.0 mmol) were added successively under slow stirring, and the reaction was stirred for about 20 hours at room temperature. After the reaction was completed, the reaction solution was concentrated, then dissolved in dichloromethane, and successively extracted with 0.6 M hydrochloric acid/10% sodium chloride solution and saturated sodium bicarbonate solution. The organic phases were combined, dried with anhydrous magnesium sulfate, filtered, the filtrate was concentrated, and the crude product was purified by column chromatography to obtain the cationic lipid **E46-1** (1.24 g). ¹H NMR (400 MHz, CDCl₃) δ: 4.26-4.09 (m, 32H; 16H, -C(=O)OC*H*₂C*H*₂OC(=O)-; 16H, -NHC(=O)OC*H*₂-), 3.34 (s, 8H, -OC(=O)C*H*₂N<), 3.21 (s, 8H, Ring-C*H*₂-), 3.15 (t, 16H, -C*H*₂NHC(=O)O-), 2.83-2.42 (m, 32H; 16H, Ring-*H*; 16H, >NC*H*₂CH₂-), 1.61-1.22 (m, 192H, -CH₂C*H*₂CH₂-, -C*H*₂CH₃), 0.88 (t, 24H, -CH₂C*H*₃). MS (ESI): m/z=3074.4 ([M+H]⁺).

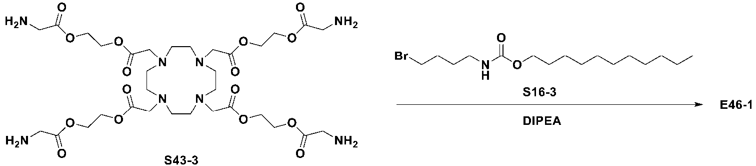

### Example 47: Preparation of LNP-mRNA Pharmaceutical Compositions and Characterization of Physicochemical Properties Thereof

### Example 47.1: Preparation of LNP-mRNA Pharmaceutical Compositions

In the present embodiment, LNP-mRNA pharmaceutical compositions (LNP-mRNAs) containing Fluc-mRNA were prepared; wherein, the phospholipid contained in each group was DSPC, the steroid lipid contained in each group was cholesterol, and the PEGylated lipid contained in each group was PEG2k-DMG; the difference among the groups lied in the cationic lipid and the molar ratio of lipids.

The preparation method of LNP-mRNA is as follows:
Step a: A certain amount of cationic lipid, DSPC, cholesterol, and PEGylated lipid stock solutions were pipetted and dissolved in ethanol to obtain the ethanol phase solution; wherein, the control groups **L-CT1, L-CT2, L-CT3,** and **L-CT4** used cationic lipids from prior art, while the experimental groups L-1 to L-50 used the azacycle-containing cationic lipids in Examples 1 to 46 of the present application; wherein, the molar ratios of the lipids used were 35:16:46.5:2.5 for **L-CT1, L-CT2, L-CT3,** and **L-1 to L-48,** and 50:10:38:1.5 for **L-CT4, L-49,** and **L-50.**
   Wherein, the cationic lipid used in L-CT1 was C-1, prepared according to the method disclosed in patent literature CN115010681A; the cationic lipid used in L-CT2 was C-2, prepared according to the method disclosed in patent literature US20220040308A1; the cationic lipid used in L-CT3 was 244cis, prepared according to the method disclosed in "Kim, Minjeong, et al. Novel piperazine-based ionizable LNPs allow the repeated dose of mRNA to fibrotic lungs with improved potency and safety. Bioengineering & Translational Medicine 8.6 (2023): e10556". The structures of C-1, C-2, and 244cis are as follows: C-1: 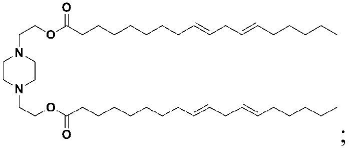 C-2: 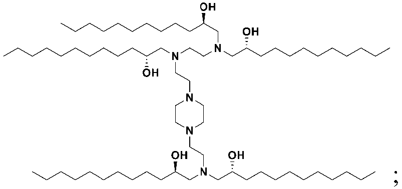 244cis: 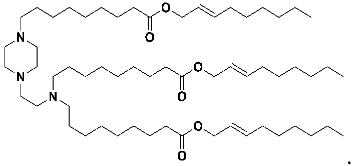
Step b: Fluc-mRNA was added to 10~50 mM citrate buffer (pH=4) to obtain the aqueous solution.
Step c: the ethanol phase solution and the aqueous phase solution were mixed (1:3, v/v) to prepare LNP-mRNA, which was washed by multiple DPBS ultrafiltration to remove ethanol and free molecules. Finally, it was filtered through a 0.2 µm sterile filter and stored for subsequent use.

### Example 47.2: Characterization of Physicochemical Properties of LNP-mRNA Pharmaceutical Compositions

Determination of encapsulation rate: The encapsulation efficiency of LNP-mRNA compositions was determined using the Quant-it Ribogreen RNA Quantification Kit. The results showed that the lipid compositions of the present application (L-1~L-50) exhibited high nucleic acid encapsulation efficiencies for nucleic acid drugs, all in the range of 80%-96%, and most in the range of 90%-96%. The results showed that mRNA could be effectively encapsulated in the experimental groups with azacycle-containing cationic lipids, wherein the encapsulation efficiencies were comparable to or better than those of C-1, C-2, and 244cis; there were also differences in the encapsulation efficiency among the azacycle-containing cationic lipids with different structures.

Determination of particle size: In the present embodiment, the particle size of LNP-mRNA was determined by dynamic light scattering (DLS). The measured LNP-mRNAs exhibited high dimensional uniformity, with PDI values less than 0.3. The particle sizes of the LNP-mRNAs prepared from the lipid compositions of the present application were in the range of 90-120 nm, which meets the requirements of particle size for gene vectors.

**Table 1: Summary of Formulations of Lipid Compositions and Particle Sizes and Encapsulation Efficiency of the Prepared LNP-mRNAs**

| **LNP-mRNA** | **L-CT1** | **L-CT2** | **L-CT3** | **L-1** | **L-2** | **L-3** |
|---|---|---|---|---|---|---|
| **Cationic Lipid, CL** | C-1 | C-2 | 244cis | E1-1 | E2-1 | E3-1 |
| **Encapsulation Efficiency /%** | 87.58 | 91.61 | 90.11 | 94.62 | 92.25 | 94.33 |
| **Particle Size /nm** | 93.70 | 93.05 | 92.33 | 93.25 | 94.51 | 95.32 |

| **LNP-mRNA** | **L-4** | **L-5** | **L-6** | **L-7** | **L-8** | **L-9** |
|---|---|---|---|---|---|---|
| **Cationic Lipid, CL** | E4-1 | E5-1 | E6-1 | E7-1 | E7-2 | E7-3 |
| **Encapsulation Efficiency /%** | 92.18 | 93.63 | 92.55 | 93.52 | 94.19 | 93.36 |
| **Particle Size /nm** | 95.26 | 95.38 | 94.37 | 92.12 | 97.47 | 96.51 |

| **LNP-mRNA** | **L-10** | **L-11** | **L-12** | **L-13** | **L-14** | **L-15** |
|---|---|---|---|---|---|---|
| Cationic **Lipid, CL** | E8-1 | E9-1 | E10-1 | E11-1 | E12-1 | E13-1 |
| **Encapsulation Efficiency** /% | 92.29 | 93.17 | 90.92 | 94.14 | 92.22 | 93.65 |
| **Particle Size /nm** | 96.48 | 93.56 | 93.42 | 93.25 | 94.69 | 95.32 |

| **LNP-mRNA** | **L-16** | **L-17** | **L-18** | **L-19** | **L-20** | **L-21** |
|---|---|---|---|---|---|---|
| **Cationic Lipid, CL** | E14-1 | E15-1 | E16-1 | E17-1 | E18-1 | E19-1 |
| **Encapsulation Efficiency /%** | 93.88 | 90.76 | 94.47 | 93.27 | 92.19 | 93.72 |
| **Particle Size /nm** | 98.41 | 95.26 | 93.72 | 94.51 | 95.86 | 92.36 |

| **LNP-mRNA** | **L-22** | **L-23** | **L-24** | **L-25** | **L-26** | **L-27** |
|---|---|---|---|---|---|---|
| **Cationic Lipid, CL** | E20-1 | E21-1 | E22-1 | E23-1 | E24-1 | E25-1 |
| **Encapsulation Efficiency /%** | 92.17 | 91.66 | 93.39 | 92.56 | 94.69 | 95.88 |
| **Particle Size /nm** | 93.59 | 92.36 | 93.68 | 94.43 | 93.56 | 92.55 |

| **LNP-mRNA** | **L-28** | **L-29** | **L-30** | **L-31** | **L-32** | **L-33** |
|---|---|---|---|---|---|---|
| **Cationic Lipid, CL** | E26-1 | E27-1 | E28-1 | E29-1 | E30-1 | E31-1 |
| **Encapsulation Efficiency /%** | 92.82 | 92.22 | 95.42 | 92.19 | 94.12 | 91.85 |
| **Particle Size /nm** | 94.51 | 94.11 | 93.44 | 94.82 | 95.42 | 94.13 |

| **LNP-mRNA** | **L-34** | **L-35** | **L-36** | **L-37** | **L-38** | **L-39** |
|---|---|---|---|---|---|---|
| **Cationic Lipid, CL** | E32-1 | E33-1 | E34-1 | E35-1 | E36-1 | E37-1 |
| **Encapsulation Efficiency /%** | 92.05 | 93.64 | 93.82 | 90.72 | 91.03 | 93.48 |
| **Particle Size /nm** | 94.10 | 95.46 | 94.18 | 94.55 | 94.68 | 94.69 |

| **LNP-mRNA** | **L-40** | **L-41** | **L-42** | **L-43** | **L-44** | **L-45** |
|---|---|---|---|---|---|---|
| **Cationic Lipid, CL** | E38-1 | E39-1 | E40-1 | E41-1 | E42-1 | E43-1 |
| **Encapsulation Efficiency /%** | 90.83 | 93.95 | 93.66 | 92.93 | 93.59 | 93.75 |
| **Particle Size /nm** | 94.73 | 93.94 | 94.94 | 96.82 | 94.83 | 96.13 |

| **LNP-mRNA** | **L-46** | **L-47** | **L-48** | **L-49** | **L-50** | **L-CT4** |
|---|---|---|---|---|---|---|
| **Cationic Lipid, CL** | E44-1 | E45-1 | E46-1 | E13-1 | E25-1 | C-1 |
| **Encapsulation Efficiency /%** | 94.01 | 93.62 | 94.03 | 92.74 | 93.12 | 90.78 |
| **Particle Size /nm** | 94.51 | 95.37 | 93.48 | 95.83 | 98.01 | 94.88 |

### Example 48: Biological Activity Tests of LNP-mRNA Pharmaceutical Compositions

### (1) Serum Stability Evaluation

LNP-mRNA pharmaceutical compositions were added to the medium containing 10% fetal bovine serum (FBS) with agitation at 37°C. Samples was taken at regular intervals to determine the particle size change of LNP-mRNA, and the serum stability of the LNP-mRNA pharmaceutical composition formulation was analyzed by testing its particle size change. The experimental results showed that within 7 days, the particle size change of control groups and experimental groups were less than 10%, and the particle size change of all experimental groups were less than 5%, indicating that the formulation of LNP-mRNA pharmaceutical composition prepared from the cationic lipids of the present application exhibited good serum stability.

### (2) Assays for Cytotoxicity (Biocompatibility)

DMEM high glucose complete culture medium containing 10% FBS was prepared. The samples (L-1 to L-50 and L-CT1 to L-CT4) were respectively formulated into working solutions of 0.1, 0.15, 0.2, 0.25, 0.3 µg/100 µL using the complete culture medium, and were stored for subsequent use. 293T cells in the logarithmic growth phase was inoculated onto a 96-well plate at a density of 7×10³ cells/well, with 100 µL/well. Both the control and experimental groups were set with six replicate wells. After incubation in a constant temperature incubator with 5% CO₂ at 37°C for 24 hours, the original culture medium was retained, the control group was supplemented with 100 µL/well of complete culture medium, and the sample group was supplemented with 100 µL/well of the working solution. After further incubation for 24 hours, 100 µL/well of culture medium containing 10% CCK-8 was added, and the solution was cultured in a constant temperature incubator with 5% CO₂ at 37°C for 2 hours. The absorbance at 450 nm was measured using a microplate reader. The relative cell viability was calculated using the following formula:
Relative Viability% = (Sample Group Absorbance - Background Absorbance) / (Control Group Absorbance - Background Absorbance) × 100%; wherein, the background absorbance refers to the absorbance of the sample containing only CCK-8 reagent and culture medium.

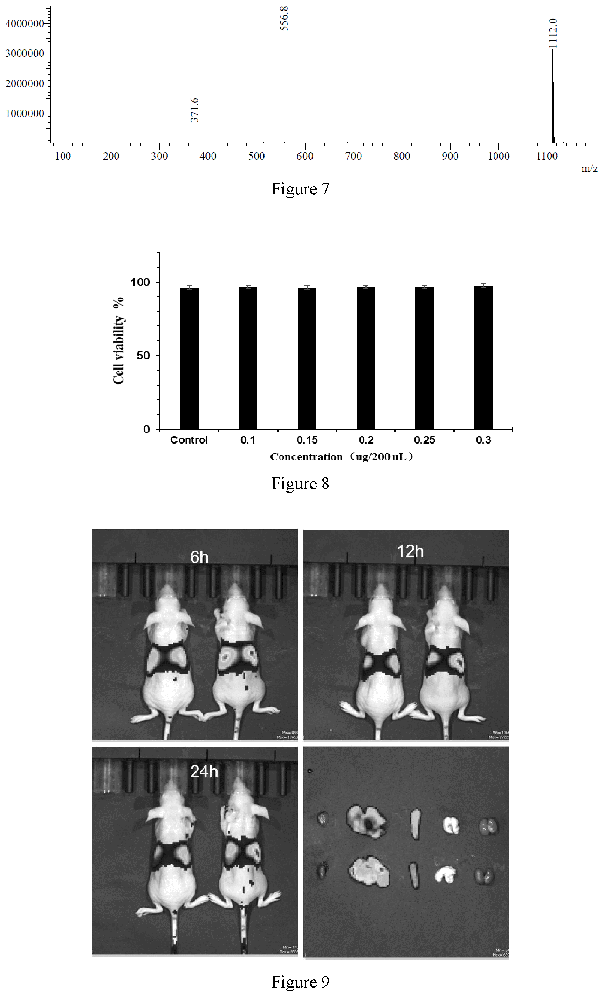

The experimental results showed that the LNP-mRNA pharmaceutical composition prepared with the cationic lipid of the present application has no obvious cytotoxicity at five concentration gradients, with cell viability above 95%. Specifically, for example, the results of the experimental group L-15 are shown in Figure 8.

### (3) Evaluation of In Vitro Transfection Efficiency

In order to investigate the mRNA transfection efficiency at the cellular level of each group of LNP-mRNA pharmaceutical compositions prepared in Example **47.1** of the present application, luciferase bioluminescence was used for testing. The LNP-mRNA pharmaceutical composition was dissolved in medium to prepare the required dose. 293T cells were used as the cell model, and cell suspensions of 100 µL/well were inoculated in a black-edged, clear-bottomed 96-well plate at a density of 6000 cells/well. After inoculation was completed, the cells were cultured in a cell culture incubator for 24 hours, and then the cells were dosed with 0.2 µg mRNA per well, while the blank control group was added with the corresponding dose of free Fluc-mRNA. After 24 hours of transfection, the old medium was replaced with fresh medium containing D-fluorescein sodium substrate (1.5 mg/mL). After 5 min of incubation, bioluminescence was detected using a microplate reader. The stronger the fluorescence, the more Fluc-mRNA is transported into the cytoplasm and translated the corresponding fluorescent protein. The results are shown in Table 2; wherein, the relative value of fluorescence intensity is the ratio of the fluorescence intensity of each group to the fluorescence intensity of the control group. The results show that the LNP-mRNA pharmaceutical compositions prepared in the present application exhibited excellent in vitro transfection effects, indicating that the LNPs in the experimental groups are effective nucleic acid delivery carriers. This is probably attributed to the fact that the cationic lipids of the present application contain multiple ionizable tertiary amine structures, which endow the LNPs with superior transfection efficiency compared to those prepared from the azacycle-containing cationic lipids in the prior art (i.e., L-CT1, L-CT2, and L-CT3 groups). The relative fluorescence values of the experimental groups L-1, L-3, L-15, L-16, L-19, L-26, L-27, L-28, L-30, L-32, and L-36 are higher. Meanwhile, there were differences in fluorescence intensity among the various experimental groups, which is probably due to the following variations in the cationic lipids: the type and/or number of degradable linking groups, and/or the type of hydrophobic hydrocarbon tail chain, and/or the degree of saturation of the hydrophobic hydrocarbon tail chain, and/or the carbon chain length between the linking group and the tertiary amine, and/or the number of ionizable tertiary amines. Specifically, when comparing E8-1 (L-10), E9-1 (L-11), and E35-1 (L-37), the transfection efficiency of E8-1 and E9-1, which contain hydrophobic hydrocarbon tail chains with linking groups, is higher. The activity of E8-1, with an ester bond as the linking group, was higher than that of E9-1, with an amide bond as the linking group. This is probably because the degradable ester bond can prevent the endosomal accumulation of LNP-mRNA, promote the endosomal escape of LNP-mRNA, and allow mRNA to be released into the cytoplasm to exert therapeutic effects. When comparing E1-1 (L-1), E17-1 (L-19), and E6-1 (L-6), the transfection efficiency of E1-1 and E17-1, which contain unsaturated hydrophobic hydrocarbon tail chains, is higher. This is probably because the unsaturated hydrocarbon chains can increase membrane fluidity and enhance cellular uptake. Similarly, when comparing E2-1 (L-2), E3-1 (L-3), and E31-1 (L-33), all of which contain unsaturated hydrocarbon chains, the transfection efficiency decreases in the order of E3-1 > E2-1 > E31-1, further indicating that introducing an ester bond in the hydrophobic hydrocarbon tail chain can result in better delivery effects and faster in vivo liver and plasma elimination. In the present application, the azacyclic cores of the cationic lipids used in groups L-41 to L-48 contain more tertiary amines than the piperazine ring; this attributes to the effective delivery and transfection of nucleic acids into cells but does not lead to the optimal transfection efficiency, regardless of the presence of more tertiary amines. This is probably because the molar ratios used in the present application are more suitable for cationic lipids containing a piperazine ring, while the formulation using azacycle-containing cationic lipids with a higher number of tertiary amines needs to be further optimized.

Furthermore, certain differences can also be observed among the LNP-mRNAs prepared with different molar ratios. When cationic lipid C-1 was used to prepare the LNP-mRNA with a molar ratio of 35:16:46.5:2.5 (L-CT1), the transfection efficiency was only 75% that of the LNP-mRNA with a molar ratio of 50:10:38:1.5 (L-CT4). This is probably because C-1 only has the piperazine ring that provides effective ionizable tertiary amines, and it only contains two linear hydrophobic hydrocarbon tail chains. Such a structure is not conducive to forming a conical geometry, thereby resulting in lower transfection efficiency. Comparing L-15 with L-49, or L-27 with L-50, the transfection effects of the LNP-mRNA prepared with cationic lipid E13-1 or E25-1 are comparable between two different molar ratios, or slightly better for the molar ratio of 35:16:46.5:2.5. This indicates that the cationic lipids of the present application can also show excellent transfection effects with a lower molar ratio, which can greatly reduce the amount of cationic lipid used.

**Table 2: Results of Cell Transfection Tests**

| **Entry** | **Relative Fluorescence Value** | **Entry** | **Relative Fluorescence Value** | **Entry** | **Relative Fluorescence Value** |
|---|---|---|---|---|---|
| **Blank** | 1 | **L-16** | 9.9 | **L-35** | 8.0 |
| **L-CT1** | 5.1 | **L-17** | 7.6 | **L-36** | 9.8 |
| **L-CT2** | 4.7 | **L-18** | 8.1 | **L-37** | 7.5 |
| **L-CT3** | 6.9 | **L-19** | 9.4 | **L-38** | 7.4 |
| **L-1** | 9.6 | **L-20** | 7.5 | **L-39** | 7.9 |
| **L-2** | 8.0 | **L-21** | 8.6 | **L-40** | 8.1 |
| **L-3** | 9.3 | **L-22** | 8.4 | **L-41** | 8.4 |
| **L-4** | 6.3 | **L-23** | 7.5 | **L-42** | 8.2 |
| **L-5** | 7.8 | **L-24** | 8.7 | **L-43** | 8.4 |
| **L-6** | 8.5 | **L-25** | 7.9 | **L-44** | 8.7 |
| **L-7** | 8.2 | **L-26** | 9.5 | **L-45** | 8.9 |
| **L-8** | 8.3 | **L-27** | 10.1 | **L-46** | 8.8 |
| **L-9** | 8.0 | **L-28** | 9.8 | **L-47** | 8.6 |
| **L-10** | 9.1 | **L-29** | 7.6 | **L-48** | 8.0 |
| **L-11** | 8.4 | **L-30** | 8.3 | **L-49** | 9.2 |
| **L-12** | 8.0 | **L-31** | 7.5 | **L-50** | 9.3 |
| **L-13** | 8.3 | **L-32** | 9.6 | **L-CT4** | 6.8 |
| **L-14** | 8.5 | **L-33** | 8.2 | | |
| **L-15** | 10.2 | **L-34** | 8.1 | | |

### (4) Evaluation of In Vivo Transfection Efficiency

The lipid nanoparticle **L-15** was delivered to 6-8-week-old female BALB/c mice at a dose of 10 µg per mouse by tail vein injection, and in vivo fluorescence imaging of the mice was performed at 6, 12, and 24 hours post-administration. After the last imaging was completed, the mice were euthanized, and imaging was conducted on the major organs including heart, liver, spleen, lungs, and kidneys (from left to right in the figure). 10 to 15 minutes prior to imaging, 0.2 mL of D-luciferin sodium salt (15 mg/mL) was injected intraperitoneally. The experimental results (Figure 9) indicate that the lipid-nucleic acid pharmaceutical composition prepared from the cationic lipid of the present application can achieve efficient in vivo delivery of nucleic acid drugs, and the LNP-mRNA pharmaceutical composition delivered into the body was primarily distributed in the liver and spleen.

Those described above are only embodiments of the present invention, not limiting the patent scope of the present invention. Any transformation of equivalent structure or equivalent route based on the specification content of the present invention, directly or indirectly applied in other related arts, should be included in the protected patent scope of the present invention in the same way.

For those skilled in the art, without departing from the purpose and the scope of the present invention, and without unnecessary experimentation, the present invention can be implemented in a wider range with equivalent parameters, concentrations, and conditions. While the present invention has given particular examples, it should be understood that the present invention can be further modified. In conclusion, in accordance with the principles of the present invention, the present application is intended to cover any alterations, uses, or improvements of the present invention, including changes departing from the scope disclosed in this application but made using conventional techniques known in the art.

## Claims

1. A cationic lipid, wherein the structure is represented by the general formula (1-A): 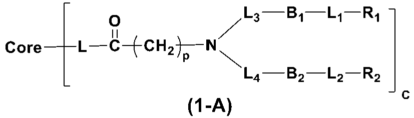
or a salt, tautomer, stereoisomer, deuterated derivative, or solvate thereof;
wherein, Core is an azacyclic core;
L is a divalent linking group L_{d}; the L_{d} is selected from the group consisting of -(CH₂)ₜₘ-, -Z-, -(CH₂)ₜₘZ-, -Z(CH₂)ₜₘ-, -Z(CH₂)ₜₘZ-, -(CH₂)ₜₘZ(CH₂)ₜₘ-, -Z(CH₂)ₜₘZ(CH₂)ₜₘ-, -(CH₂)ₜₘZ(CH₂)ₜₘZ-, and -(CH₂)ₜₘZ(CH₂)ₜₘZ(CH₂)ₜₘ-; wherein, tm is independently an integer from 1 to 12 at each occurrence; Z is independently selected from the group consisting of -C(=O)-, -OC(=O)-, -C(=O)O-, -OC(=O)O-, -O-, -S-, -NH-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S- at each occurrence, wherein R_{c} is independently H or a C₁₋₁₂ alkyl group at each occurrence;
p is an integer from 1 to 4;
B₁ and B₂ are each independently a linking bond or a C₁₋₂₀ alkylene group at each occurrence;
L₁, L₂, L₃, and L₄ are each independently a linking bond or a divalent linking group -(CH₂)ₜₙLₐ(CH₂)ₜₙ- at each occurrence; the Lₐ is selected from the group consisting of -OC(=O)-, -C(=O)O-, -OC(=O)O-, -CH(OH)-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-; wherein, s is 1, 2, 3, or 4; tn is independently an integer from 0 to 12 at each occurrence; L₁ and L₃ are not linking bonds simultaneously; L₂ and L₄ are not linking bonds simultaneously;
R₁ and R₂ are each independently a C₅₋₃₀ hydrocarbon group or a C₅₋₃₀ hydrocarbon derivative group at each occurrence;
c is 2, 3, or 4.

2. The cationic lipid according to claim **1,** wherein the structure of the cationic lipid is represented by the general formula (1-A-1): 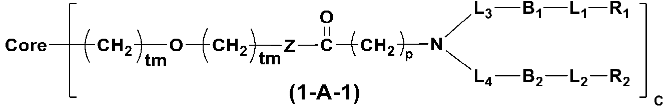 wherein, tm is independently an integer from 1 to 5 at each occurrence; Z is -O- or -NH-.

3. The cationic lipid according to claim **1,** wherein the Core is 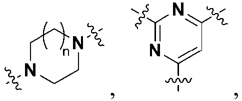 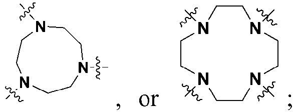 wherein, n is 1 or 2; Core is preferably 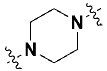 or 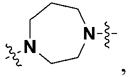 most preferably

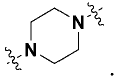

4. The cationic lipid according to claim **1,** wherein the L is selected from the group consisting of -(CH₂)ₜₘ-, -(CH₂)ₜₘO-, -(CH₂)ₜₘC(=O)NH-, -(CH₂)ₜₘOC(=O)NH-, -(CH₂)ₜₘNHC(=O)NH-, -(CH₂)ₜₘO(CH₂)ₜₘ-, -(CH₂)ₜₘC(=O)(CH₂)ₜₘ-, -(CH₂)ₜₘC(=O)O(CH₂)ₜₘ-, -C(=O)(CH₂)ₜₘC(=O)(CH₂)ₜₘ-, -(CH₂)ₜₘC(=O)O(CH₂)ₜₘO-, -(CH₂)ₜₘOC(=O)(CH₂)ₜₘ-, -(CH₂)ₜₘC(=O)NH(CH₂)ₜₘ-, -(CH₂)ₜₘNHC(=O)(CH₂)ₜₘ-, -(CH₂)ₜₘOC(=O)O(CH₂)ₜₘ-, -(CH₂)ₜₘNHC(=O)O(CH₂)ₜₘ-, -(CH₂)ₜₘOC(=O)NH(CH₂)ₜₘ-, -(CH₂)ₜₘC(=O)O(CH₂)ₜₘO(CH₂)ₜₘ-, -(CH₂)ₜₘNH-, -(CH₂)ₜₘO(CH₂)ₜₘNH-, -(CH₂)ₘOC(=O)NH(CH₂)ₜₘ-, -(CH₂)ₜₘNHC(=O)NH(CH₂)ₜₘ-, and -(CH₂)ₜₘO(CH₂)ₜₘO-, wherein the left end of L is connected to the azacycle; preferably, L is selected from the group consisting of -(CH₂)ₜₘO(CH₂)ₜₘ-, -(CH₂)ₜₘC(=O)O(CH₂)ₜₘ-, -(CH₂)ₜₘO-, -(CH₂)ₜₘC(=O)O(CH₂)ₜₘO-, -(CH₂)ₜₘNH-, -(CH₂)ₜₘC(=O)O(CH₂)ₜₘO(CH₂)ₜₘ-, -(CH₂)ₜₘO(CH₂)ₜₘNH-, -(CH₂)ₜₘOC(=O)(CH₂)ₜₘ-, and -(CH₂)ₜₘO(CH₂)ₜₘO-; preferably, L is selected from the group consisting of -CH₂C(=O)O(CH₂)₄-, -CH₂C(=O)O(CH₂)₃-, -CH₂C(=O)O(CH₂)₂O(CH₂)₂-, -(CH₂)₂O-, -(CH₂)₂NH-, -(CH₂)₂O(CH₂)₂NH-, -CH₂C(=O)O(CH₂)₂O-, and -(CH₂)₂O(CH₂)₂O-, wherein the left end of L is connected to the azacycle.

5. The cationic lipid according to claim **1,** wherein the B₁ and B₂ are selected from any of the following cases:
Case (1): B₁ and B₂ are each independently a C₁₋₂₀ alkylene group, more preferably a C₂₋₁₀ alkylene group;
Case (2): one of B₁ and B₂ is a linking bond, and the other is a C₁₋₂₀ alkylene group;
Case (3): both B₁ and B₂ are linking bonds;
B₁ and B₂ are more preferably selected from the aforementioned Case (1) or Case (3);
wherein, the aforementioned C₂₋₁₀ alkylene group is preferably selected from the group consisting of an ethylene group, a propylene group, a butylene group, a pentylene group, a hexylene group, a heptylene group, and an octylene group.

6. The cationic lipid according to claim **1,** wherein L₁ and L₂ are each independently a linking bond or Lₐ; the Lₐ is selected from the group consisting of -OC(=O)-, -C(=O)O-, -OC(=O)O-, -CH(OH)-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-;
more preferably, L₁ and L₂ are selected from any of the following cases:
Case (1): L₁ and L₂ are each independently Lₐ;
Case (2): one of L₁ and L₂ is a linking bond, and the other is Lₐ;
Case (3): both L₁ and L₂ are linking bonds;
more preferably, L₁ and L₂ are each independently selected from the group consisting of a linking bond, -C(=O)-, -O-, -NH-, -OC(=O)-, -C(=O)O-, -OC(=O)O-, -CH(OH)-, -OC(=O)NH-, -NHC(=O)O-, -NHC(=O)-, and -C(=O)NH-.

7. The cationic lipid according to claim **1,** wherein the L₃ and L₄ are each independently selected from the group consisting of a linking bond, -Lₐ-, -(CH₂)ₜₙLₐ-, -Lₐ(CH₂)ₜₙ-, and -(CH₂)tnLₐ(CH₂)ₜₙ-; wherein, tn is an integer from 1 to 12; preferably, L₃ and L₄ are each independently selected from the group consisting of -(CH₂)ₜₙC(=O)-, -(CH₂)ₜₙC(=O)O-, -(CH₂)ₘOC(=O)-, -(CH₂)ₘOC(=O)O-, -(CH₂)ₜₙOC(=O)NH-, and -(CH₂)ₜₙNHC(=O)O-, wherein the right end is connected to B₁ or B₂; preferably, L₃ and L₄ are each independently selected from the group consisting of a linking bond, -CH₂CH(OH)-, -(CH₂)₂C(=O)NH-, -C(=O)-, and -C(=O)O-; more preferably, L₃ and L₄ are the same andeach a linking bond, -CH₂CH(OH)-, or -(CH₂)₂C(=O)NH-.

8. The cationic lipid according to claim **1,** wherein the R₁ and R₂ are each independently a C₅₋₃₀ linear hydrocarbon group, a C₅₋₃₀ branched hydrocarbon group, or a C₅₋₃₀ hydrocarbon derivative group; the C₅₋₃₀ hydrocarbon derivative group is 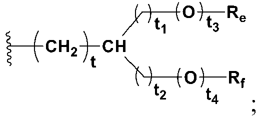 the linear hydrocarbon group and branched hydrocarbon group are each independently substituted or unsubstituted, wherein the substitution is preferably a C₁₋₆ alkyl group, halogen, or a hydroxyl group;
the linear hydrocarbon group is selected from the group consisting of a linear alkyl group, a linear alkenyl group, and a linear alkynyl group, more preferably a C₅₋₂₅ linear hydrocarbon group;
the branched hydrocarbon group is selected from the group consisting of a branched alkyl group, a branched alkenyl group, and a branched alkynyl group, each independently represented by 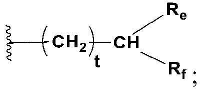 wherein, t is an integer from 0 to 12, t₁ and t₂ are each independently an integer from 0 to 5, t₃ and t₄ are each independently 0 or 1, and t₁, t₂, t₃, and t₄ are not simultaneously zero; Rₑ and R_{f} are each independently selected from the group consisting of a C₁₋₁₅ alkyl group, a C₂₋₁₅ alkenyl group, and a C₂₋₁₅ alkynyl group; more preferably, Rₑ and R_{f} are each independently selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a vinyl group, a propenyl group, a butenyl group, a pentenyl group, a hexenyl group, a heptenyl group, an octenyl group, a nonenyl group, a decenyl group, an ethynyl group, a propynyl group, a butynyl group, a pentynyl group, a hexynyl group, a heptynyl group, an octynyl group, a nonynyl group, and a decynyl group; more preferably, Rₑ and R_{f} are each independently selected from the group consisting of a methyl group, an ethyl group, a propyl group, a butyl group, a pentyl group, a hexyl group, a heptyl group, and an octyl group.

9. The cationic lipid according to claim **8,** wherein the R₁ and R₂ are selected from any of the following cases:
Case (1): R₁ and R₂ are each independently a C₅₋₃₀ linear hydrocarbon group;
Case (2): one of R₁ and R₂ is a C₅₋₃₀ linear hydrocarbon group, and the other is a C₅₋₃₀ branched hydrocarbon group represented by 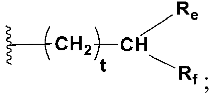
Case (3): R₁ and R₂ are each independently a C₅₋₃₀ branched hydrocarbon group represented by 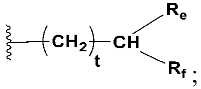
Case (4): R₁ and R₂ are each independently represented by 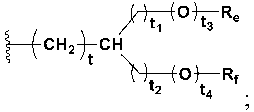
Case (5): one of R₁ and R₂ is a C₅₋₃₀ linear hydrocarbon group or a C₅₋₃₀ branched hydrocarbon group represented by 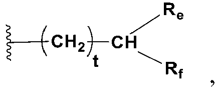 and the other is 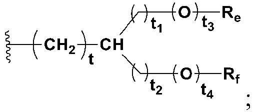
the R₁ and R₂ selected from the above cases are more preferably, each independently selected from the group consisting of the following structures:

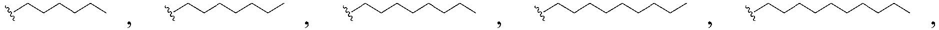

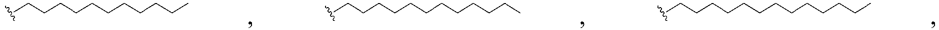

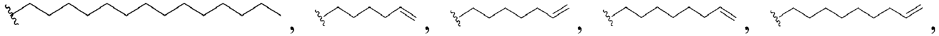

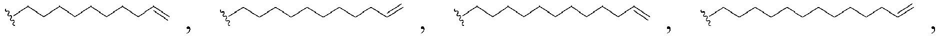

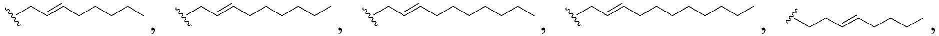

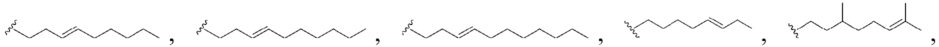

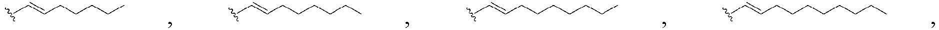

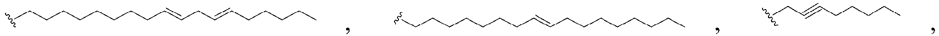

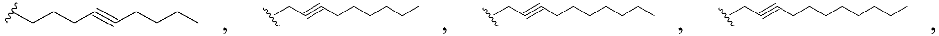

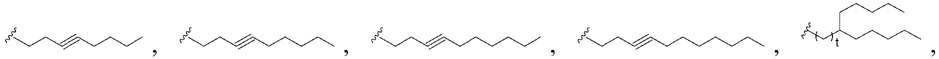

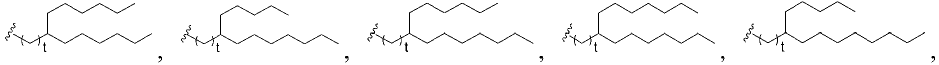

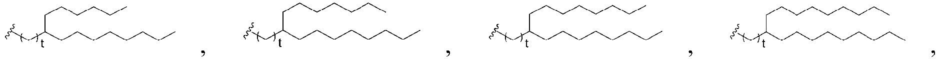

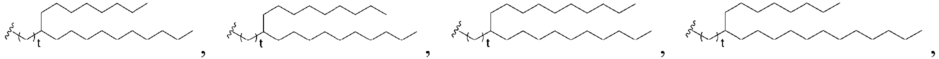

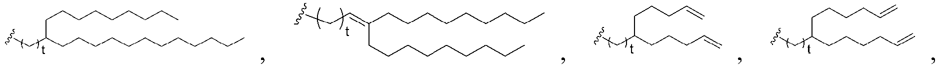

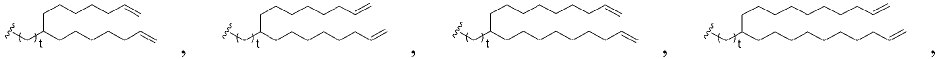

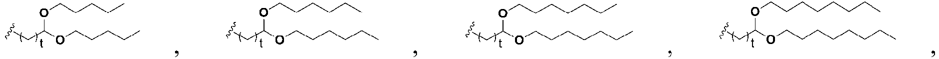

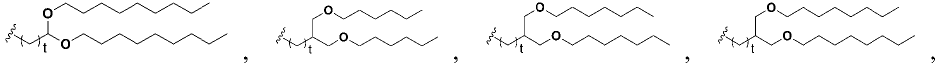

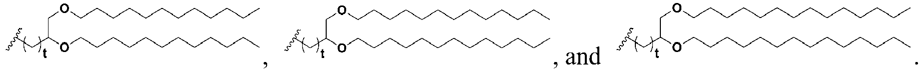

10. The cationic lipid according to any one of claims **1** to **9,** wherein the fragments -L₃-B₁-L₁-R₁ and -L₄-B₂-L₂-R₂ are each independently selected from the group consisting of the following structures:

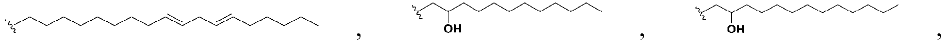

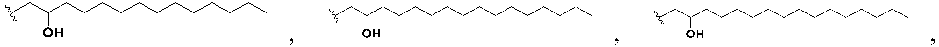

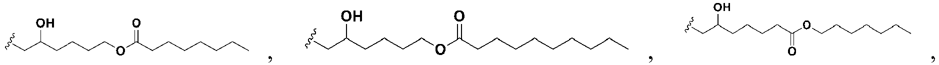

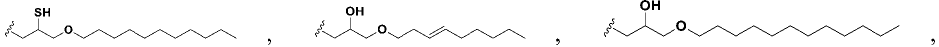

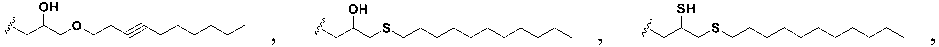

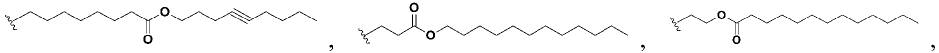

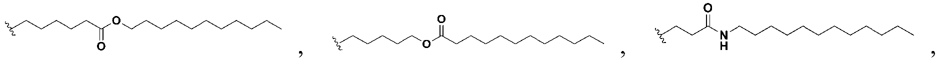

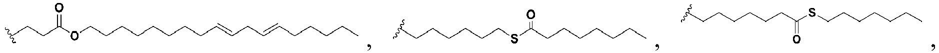

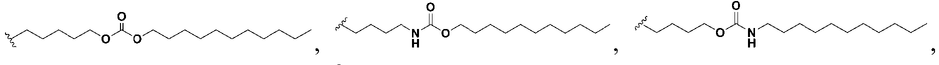

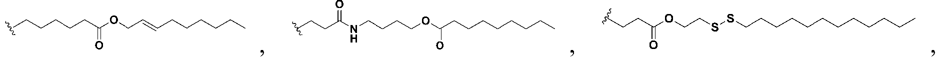

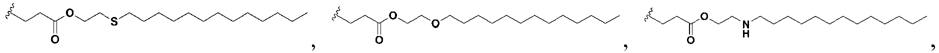

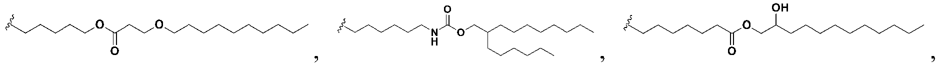

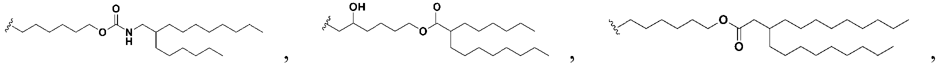

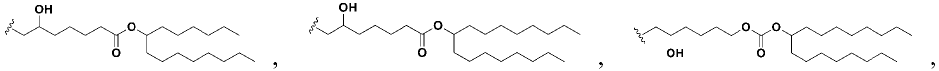

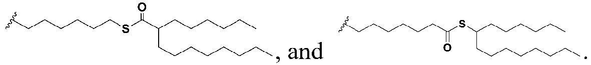

11. The cationic lipid according to claim **1,** wherein the structure of cationic lipid satisfies any one of the following general formulas: 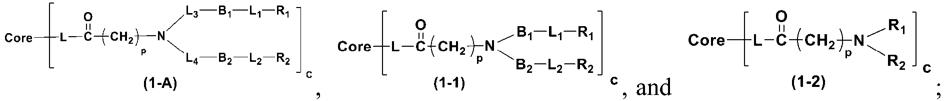 wherein, L₁, L₂, L₃, L₄, B₁, and B₂ are not linking bonds;
the structure of cationic lipid preferably satisfies any one of the following general formulas: 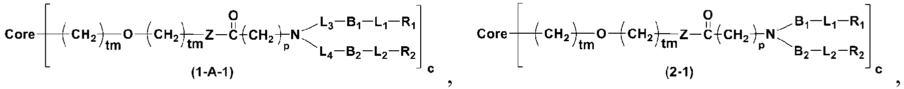 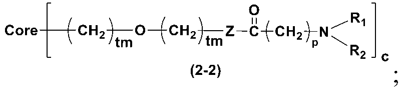
more preferably, the structure of cationic lipid satisfies any one of the following general formulas: 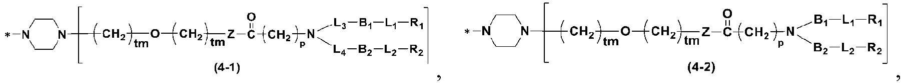 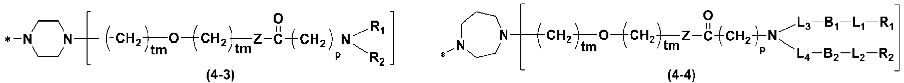 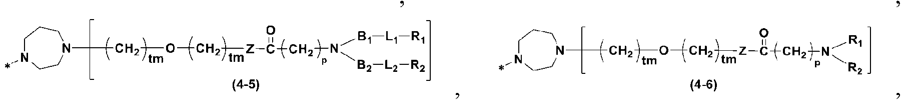 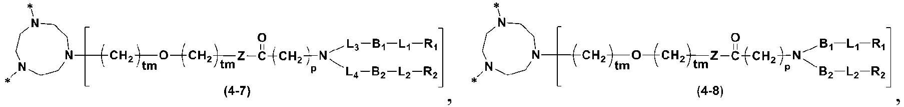 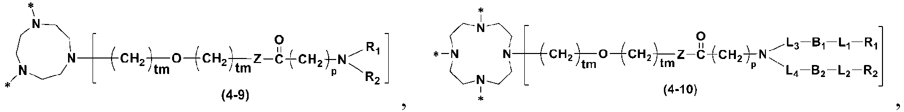 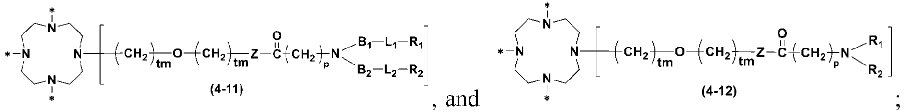 wherein, * refers to the same structure as in [ ];
in all of the aforementioned general formulas, L₁ and L₂ are, at each occurrence, preferably each independently selected from the group consisting of -C(=O)-, -O-, -NH-, -C(=O)O-, -OC(=O)-, -OC(=O)O-, -C(=O)NH-, -NHC(=O)-, -OC(=O)NH-, and -NHC(=O)O-; more preferably, L₁ and L₂ are each independently selected from the group consisting of -C(=O)-, -C(=O)O-, -OC(=O)-, and -OC(=O)O-;
preferably, the structure of a cationic lipid conforms to any one of formulas (4-1) to (4-3); preferably, in formula (4-1), L₃ and L₄ are each independently -CH₂CH(OH)-, L₁ and L₂ are selected from the group consisting of -C(=O)O-, -OC(=O)-, and -OC(=O)O-; R₁ and R₂ of formula (4-3) are each independently a C₅₋₂₅ alkenyl group or a C₅₋₂₅ alkynyl group.

12. The cationic lipid according to claim **1,** wherein the cationic lipid is preferably selected from the group consisting of the following structures: 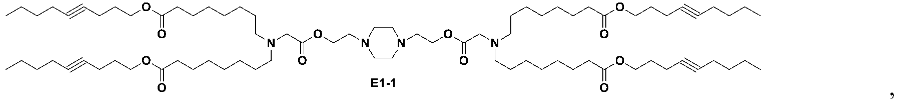 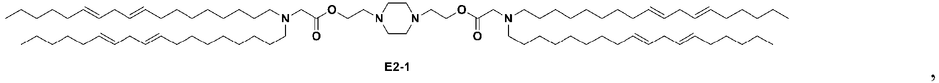 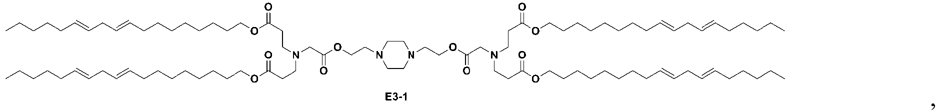 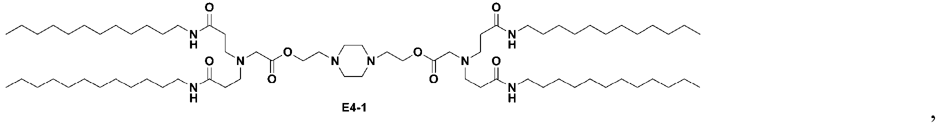 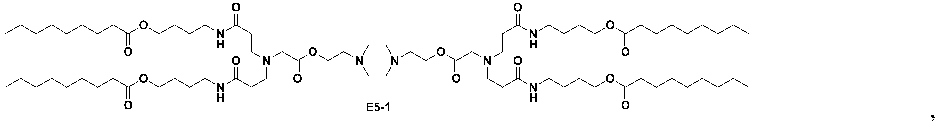 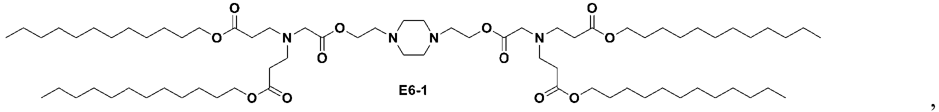 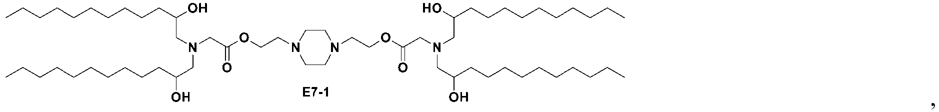 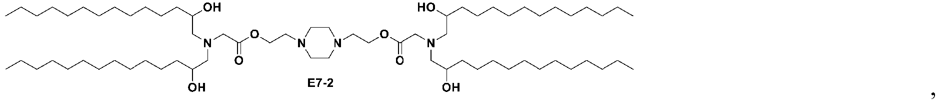 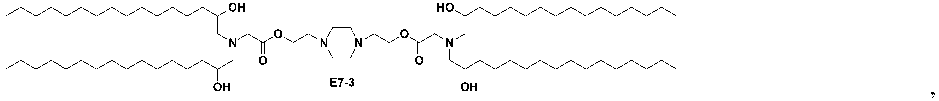 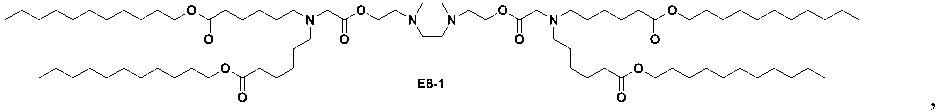 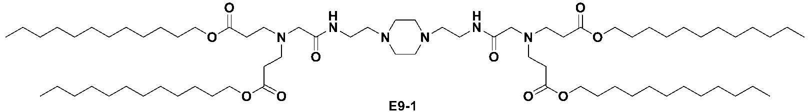 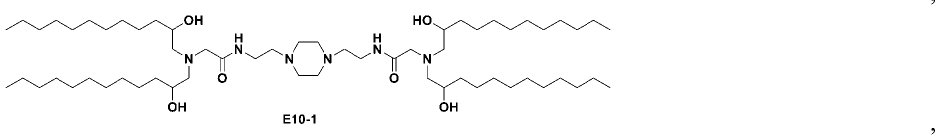 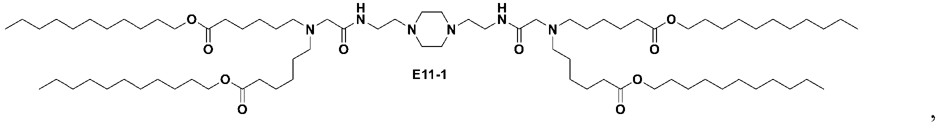 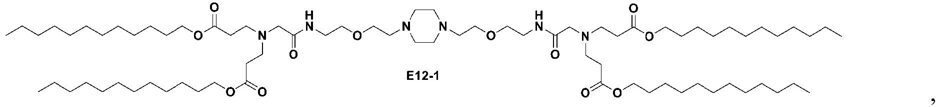 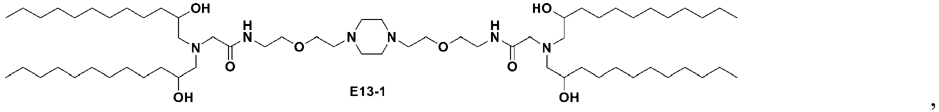 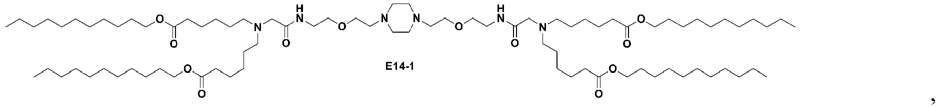 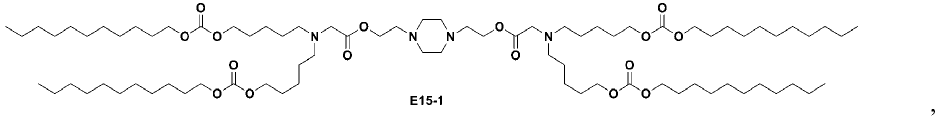 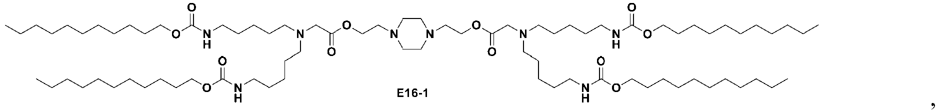 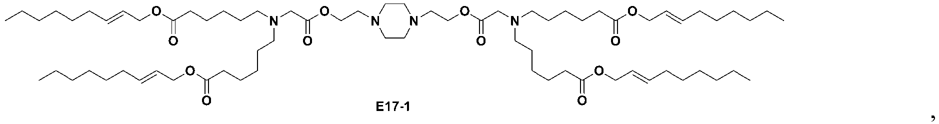 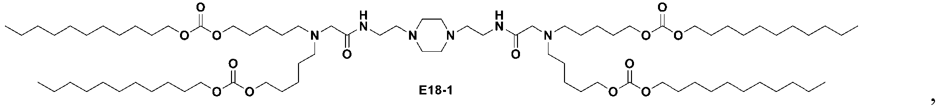 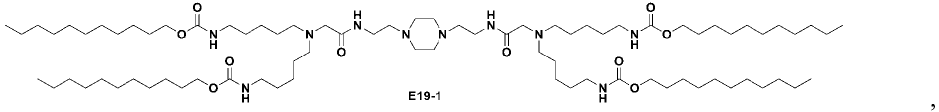 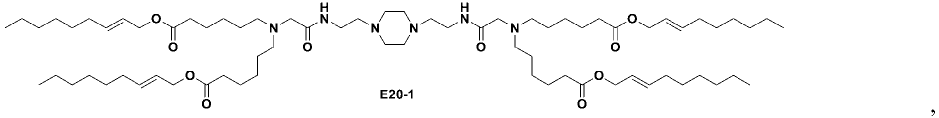 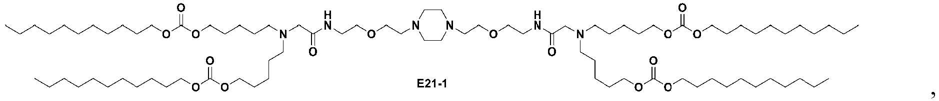 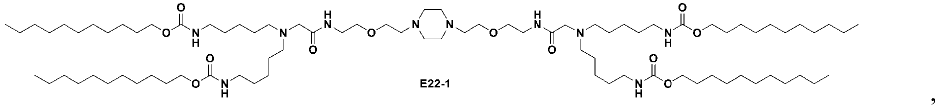 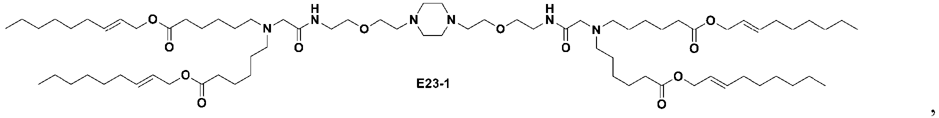 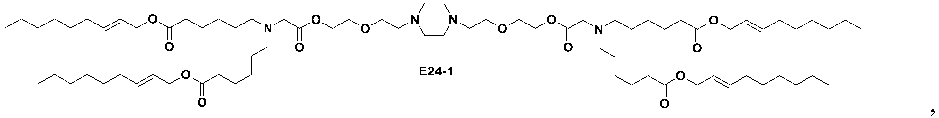 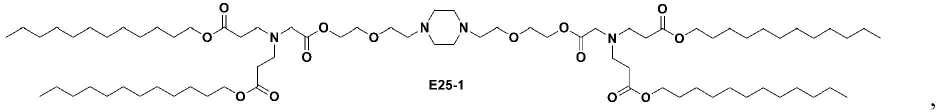 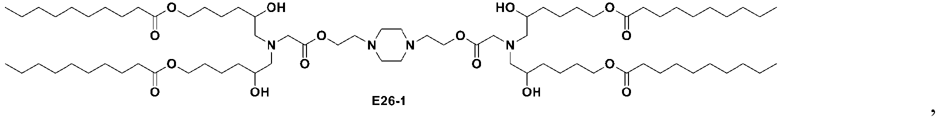 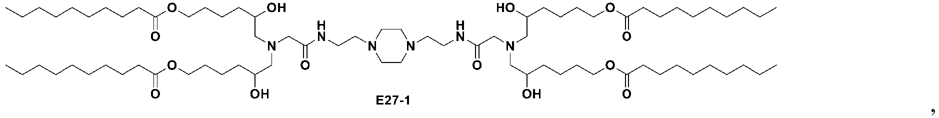 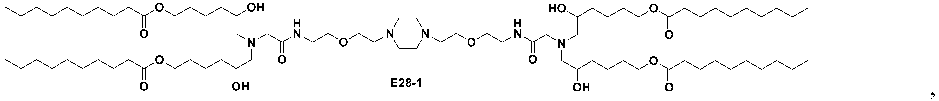 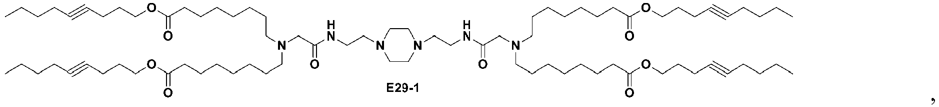 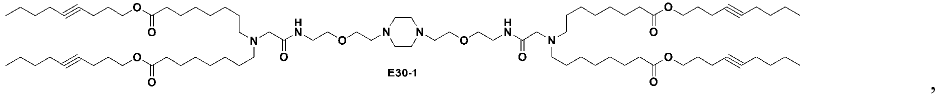 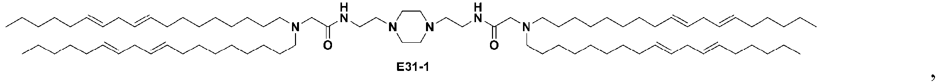 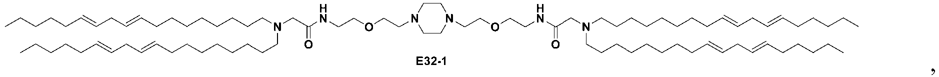 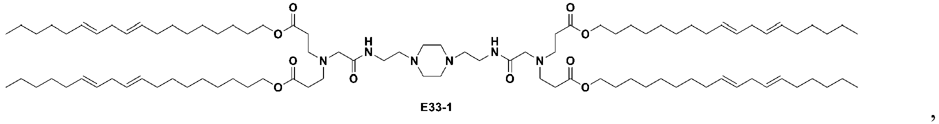 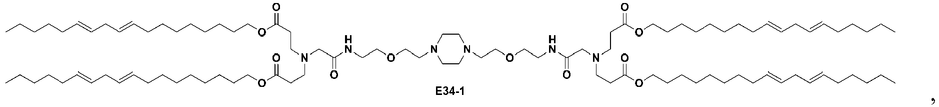 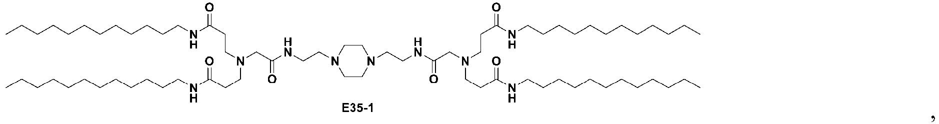 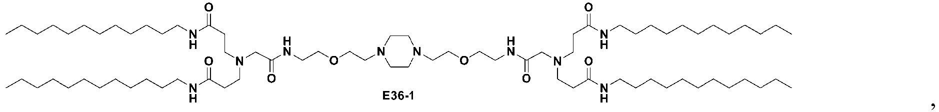 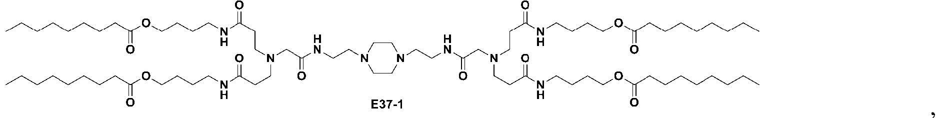 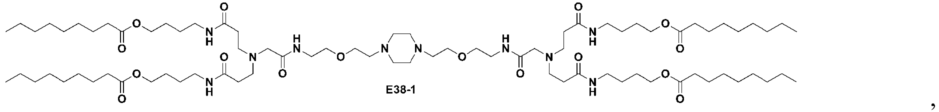 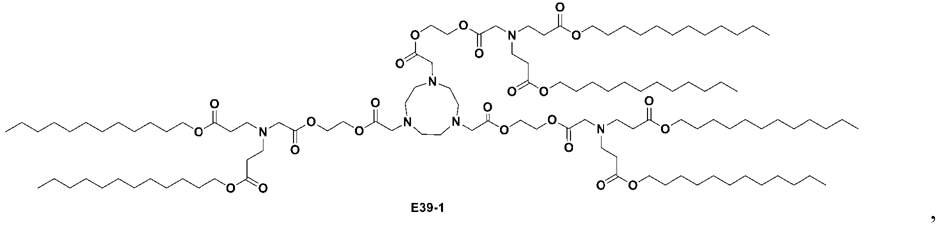 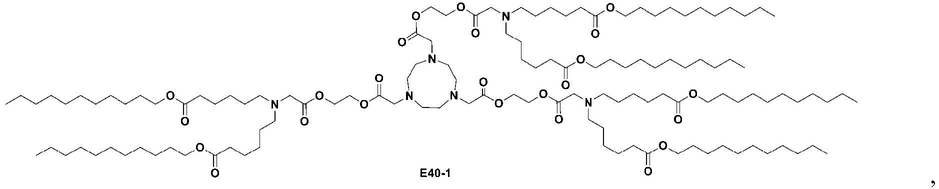 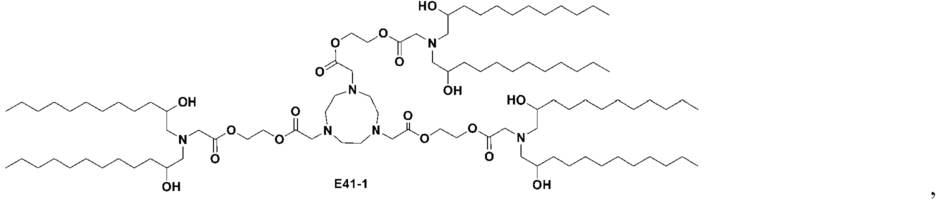 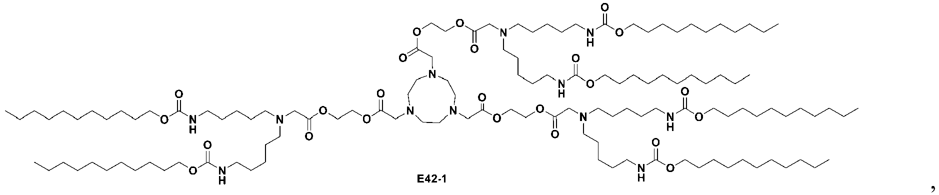 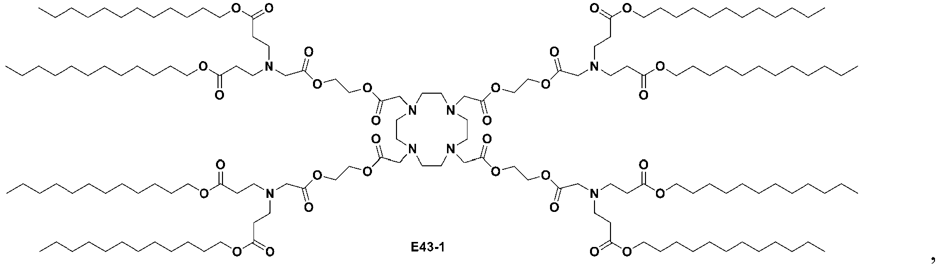 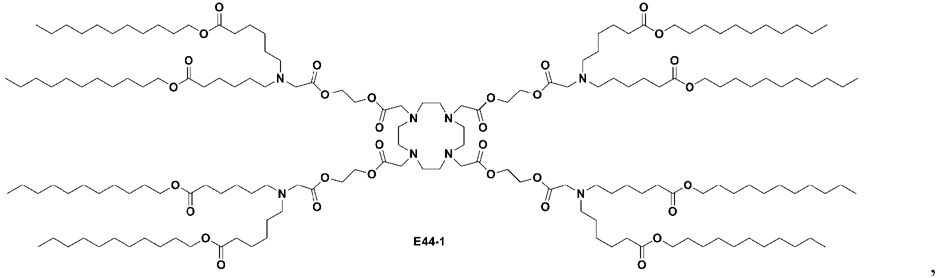 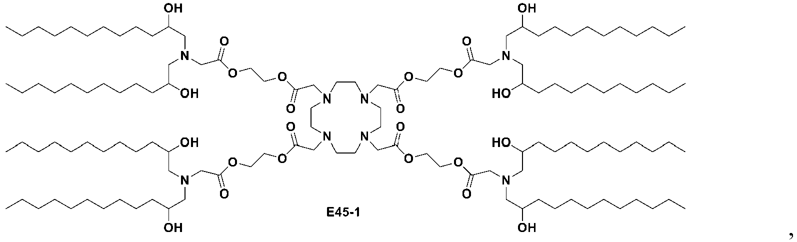 and

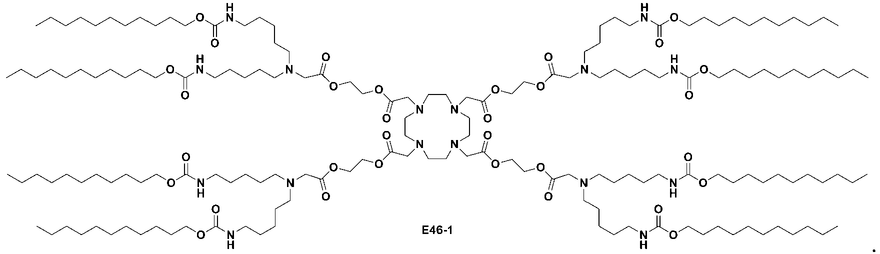

13. A lipid composition comprising a cationic lipid of any one of claims **1** to **12.**

14. The lipid composition according to claim **13,** wherein the lipid composition comprises one or more types of lipids selected from the group consisting of phospholipid, steroid lipid, and PEGylated lipid; the lipid composition is selected from any of the following cases:
Case (1): the lipid composition further comprises a phospholipid;
Case (2): the lipid composition further comprises a steroid lipid;
Case (3): the lipid composition further comprises a PEGylated lipid;
Case (4): the lipid composition further comprises a phospholipid and a steroid lipid;
Case (5): the lipid composition further comprises a phospholipid and a PEGylated lipid;
Case (6): the lipid composition further comprises a steroid lipid and a PEGylated lipid;
Case (7): the lipid composition further comprises a phospholipid, a steroid lipid, and a PEGylated lipid;
Case (8): the lipid composition further comprises a phospholipid, a steroid lipid, a PEGylated lipid, and another cationic lipid;
Case (9): the lipid composition further comprises a phospholipid, a steroid lipid, a PEGylated lipid, and an anionic lipid;
more preferably, the lipid composition further comprises three types of lipids, simultaneously, which are a neutral lipid, a steroid lipid, and a PEGylated lipid.

15. The lipid composition according to claim **14,** wherein the phospholipid in the lipid composition is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphatidylcholine, 1,2-diundecanoyl-sn-glycero-3-phosphatidylcholine, 1-plamitoyl-2-oleoyl-sn-glycero-3-phosphocholine, 1,2-di-O-octadecenyl-sn-glycero-3-phosphatidylcholine, 1-oleoyl-2-cholesterylhemisuccinyl-sn-glycero-3-phosphocholine, 1-*O*-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoyl phosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof;
or the steroid lipid in the lipid composition is selected from the group consisting of cholesterol, fecal sterol, sitosterol, ergosterol, campesterol, stigmasterol, rapeseed sterol, lycopene, ursolic acid, α-tocopherol, and combinations thereof;
or the PEGylated lipid in the lipid composition is selected from the group consisting of 1,2-dimyristoyl-sn-glycerol methoxypolyethylene glycol, 1,2-distearoyl-sn-glycero-3-phosphoethanolamine-*N*-[amino(polyethylene glycol)], PEG-cholesterol, PEG-diacylglycamide, and PEG-dialkyloxypropyl, specifically including PEG500-dipalmitoylphosphatidylcholine, PEG2000-dipalmitoylphosphatidylcholine, PEG500-stearylphosphatidylethanolamine, PEG2000-distearylphosphatidylethanolamine, PEG500-1,2-oleoylphosphatidylethanolamine, PEG2000-1,2-oleoylphosphatidylethanolamine, PEG2000-2,3-dimyristoylglycerol, and combinations thereof;
or the PEGylated lipid in the lipid composition is selected from the group consisting of the following structures and combinations thereof: 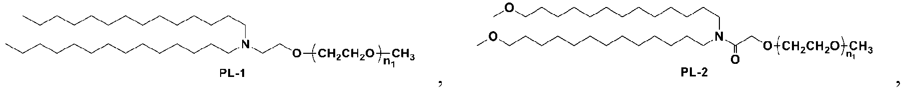 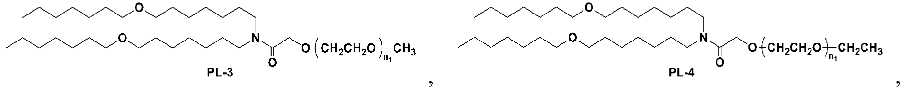 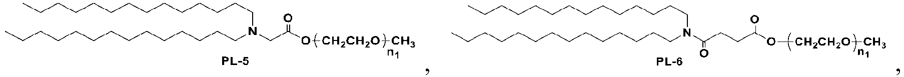 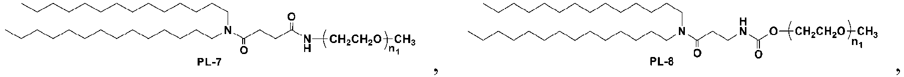 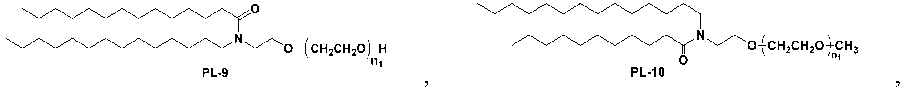 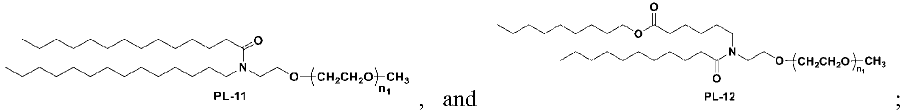 wherein, n₁ is an integer of 25 to 300, more preferably selected from the group consisting of 44, 45, 46, 47, and 48;
or the other cationic lipid in the lipid composition is preferably selected from the group consisting of 1,2-dioleoyl-3-trimethylammonium-propane, 1,2-bisoctadecoxy-3-methylammonium propane chloride, 1-[2-(oleoyloxy)ethyl]-2-oleo-3-(2-hydroxyethyl)imidazoline chloride, 1,2-diolecanol-3-dimethylamino-propane, 2,3-bis(tetradecanoyloxy)propyltrimonium chloride, didecyldimonium chloride, bisdecyldimonium bromide, *N*,*N*-diole-*N*,*N*-dimethylammonium chloride, *N*-(4-carboxybenzyl)-*N*,*N*-dimethyl-2,3-bis(oleoyloxy)propyl-1-ammonium, 3,6-bis{4-[bis(2-hydroxydodecyl)amino]butyl}piperazine-2,5-dione, 1'-((2-(4-(2-(2-(2-(bis(2-hydroxydodecyl)amino)ethyl)(2-hydroxydodecyl)amino)ethyl)pipera zine-1-yl)ethyl)azanediyl)bis(dodecane-2-ol), 4-(*N*,*N*-dimethylamino)butyric acid(dilinole)methyl ester, 1,2-dimyristoyl-sn-glycero-3-ethylphosphocholine, ((4-hydroxybutyl) azadiyl)bis(hexane-6,1-diyl)bis(2-hexyldecanoate), 8-[(2-hydroxyethyl)(6-oxo-6-(undecoxy)hexyl)amino]caprylic acid (hepta-9-yl) ester, ((2-(2-hydroxyethoxy)ethyl)azadialkyl)bis(hexane-6,1-diyl)bis(2-hexyldecanoate), and combinations thereof;
or the anionic lipid in the lipid composition is preferably selected from the group consisting of 1,2-dioleoyl-sn-glycerol-3-phosphate sodium salt, 1,2-dimyristoyl-sn-glycerol-3-phosphate sodium salt, bis(monoolein) phosphatidylmonomethylethanolamine, cardiolipin, and combinations thereof.

16. The lipid composition according to any one of claims **14** to **15,** wherein the lipid composition comprises 20-80% cationic lipids, 5-16% phospholipids, 25-55% steroid lipids and 0.5-10% PEGylated lipids; the percentages are molar percentages of each lipid with respect to the total lipids in the solvent-comprising solution.

17. The lipid composition according to claim **16,** wherein the molar percentage of cationic lipid in the total lipids in the solvent-comprising solution is 30-65%, more preferably 35%, 40%, 45%, 46%, 47%, 48%, 49%, 50%, or 55%;
or the molar percentage of phospholipid in the total lipids in the solvent-comprising solution is 7.5-16%, more preferably 8%, 9%, 10%, 11%, 12%, or 16%;
or the molar percentage of steroid lipid in the total lipids in the solvent-comprising solution is 35-50%, more preferably 40%, 41%, 42%, 43%, 44%, 45%, 46%, 47%, 48%, 49%, or 50%;
or the molar percentage of PEGylated lipid in the total lipids in the solvent-comprising solution is 0.5-5%, preferably 1-3%, more preferably 1.5%, 1.6%, 1.7%, 1.8%, 1.9%, 2.0%, or 2.5%.

18. A lipid pharmaceutical composition comprising a lipid composition of any one of claims **13** to **17** and a drug, wherein the drug is selected from the group consisting of a nucleic acid drug, a gene vaccine, an antitumor drug, a small molecule drug, a peptide drug, and a protein drug.

19. The lipid pharmaceutical composition according to claim **18,** wherein the nucleic acid drug is selected from the group consisting of RNA, DNA, antisense nucleic acid, plasmid, interfering nucleic acid, aptamer, antagomir, and ribozyme; the RNA is selected from the group consisting of mRNA, saRNA, circRNA, miRNA, and siRNA; the nucleic acid drug is preferably selected from the group consisting of DNA, mRNA, miRNA, and siRNA.

20. A lipid pharmaceutical composition according to any one of claims **18** to **19,** wherein the lipid pharmaceutical composition is used as a drug, selected from any of the following drugs: an antineoplastic agent, an antiviral agent, an antifungal agent, and a vaccine.

21. A formulation of lipid pharmaceutical composition comprising any lipid pharmaceutical composition of claim **20** and a pharmaceutically acceptable diluent or excipient; the diluent or excipient is preferably deionized water, ultrapure water, phosphate buffer or normal saline, more preferably phosphate buffer or normal saline, and most preferably normal saline.

22. A liposome or lipid nanoparticle comprising a lipid composition of any one of claims **13** to **17.**

23. The liposome or lipid nanoparticle according to claim **22,** wherein the lipid nanoparticle is an LNP-pharmaceutical composition, an LPP-pharmaceutical composition, or a PNP-pharmaceutical composition, preferably an LNP-pharmaceutical composition, more preferably an LNP-nucleic acid pharmaceutical composition, more preferably an LNP-mRNA pharmaceutical composition.
